# EUROPÄISCHE PATENTSCHRIFT

(11) **EP 0 507 171 B1**
(45) Veröffentlichungstag und Bekanntmachung des Hinweises auf die Patenterteilung: **01.10.1997**
(21) Anmeldenummer: 92104971.4
(22) Anmeldetag: 23.03.1992
(51) Int. Cl.: C07D 249/12, C07D 403/12, C07D 409/12, C07D 401/12, C07D 401/14, C07D 417/12, C07D 413/12, A01N 47/38

(54) **Sulfonylaminocarbonyltriazolinone mit über Sauerstoff gebundenen Substituenten**
Sulfonylaminocarbonyltriazolinones with oxygen-bound substituents
Sulfonylaminocarbonyltriazolinones avec des substituants liés par l'oxygène

(30) Priorität: 04.04.1991 DE 4110795
(43) Veröffentlichungstag der Anmeldung: 07.10.1992
(73) Patentinhaber: BAYER AG, 51368 Leverkusen (DE)
(72) Erfinder: Müller, Klaus-Helmut, Dr., W-4000 Düsseldorf 13 (DE); König, Klaus, Dr., W-5068 Odenthal (DE); Kluth, Joachim, Dr., W-4018 Langenfeld (DE); Lürssen, Klaus,Dr., W-5060 Bergisch Gladbach 2 (DE); Santel, Hans-Joachim, Dr., W-5090 Leverkusen 1 (DE); Schmidt, Robert R., Dr., W-5060 Bergisch Gladbach 2 (DE)

(56) Entgegenhaltungen:
- EP-A- 0 341 489
- EP-A- 0 422 469
- DE-A- 1 940 367
- DE-A- 2 259 974
- Archiv der Pharmazie, Bd.307, 1974, Nr.11

## Beschreibung

Die Erfindung betrifft neue Sulfonylaminocarbonyltriazolinone mit über Sauerstoff gebundenen Substituenten, mehrere Verfahren und neue Zwischenprodukte zu ihrer Herstellung sowie ihre Verwendung als Herbizide.

Es ist bekannt, daß bestimmte substituierte Sulfonylaminocarbonyltriazolinone, wie z.B. 2-(2-Chlor-phenylsulfonylaminocarbonyl)-4,5-dimethyl-2,4-dihydro-3H-1,2,4-triazol-3-on, herbizide Eigenschaften aufweisen (vgl. EP-A 341 489). Die Wirkung dieser Verbindungen ist jedoch nicht in allen Belangen zufriedenstellend.

Weitere Sulfonylaminocarbonyltriazolinone, wie z.B. 2-(2-Methoxycarbonyl-phenylsulfonylaminocarbonyl)-4-methyl-5-methoxy-2,4-dihydro-3H-1,2,4-triazol-3-on, sind Gegenstand einer vorgängigen, jedoch nicht vorveröffentlichten Patentanmeldung (vgl. DE-P 3934081 vom 12.10.1989).

Es wurden nun die neuen Sulfonylaminocarbonyltriazolinone mit über Sauerstoff gebundenen Substituenten der allgemeinen Formel (I) gefunden, 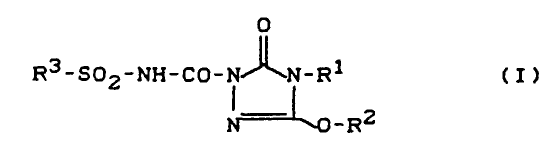 in welcher
- R¹: für Wasserstoff, Amino, für gegebenenfalls durch Fluor, Chlor, Brom, Cyano, C₁-C₄-Alkoxy, C₁-C₄-Alkylcarbonyl oder C₁-C₄-Alkoxy-carbonyl substituiertes C₁-C₆-Alkyl, für jeweils gegebenenfalls durch Fluor, Chlor und/oder Brom substituiertes C₃-C₆-Alkenyl oder C₃-C₆-Alkinyl, für gegebenenfalls durch Fluor, Chlor, Brom und/oder C₁-C₄-Alkyl substituiertes C₃-C₆-Cycloalkyl, für gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl-C₁-C₃-alkyl, für gegebenenfalls durch Fluor, Chlor, Brom, Cyano, Nitro, C₁-C₄-Alkyl, Trifluormethyl, C₁-C₄-Alkoxy, Fluor- und/oder Chlor-substituiertes C₁-C₃-Alkoxy, C₁-C₄-Alkylthio, Fluor- und/oder Chlor-substituiertes C₁-C₃-Alkylthio, C₁-C₄-Alkylsulfinyl, C₁-C₄-Alkylsulfonyl und/oder C₁-C₄-Alkoxy-carbonyl substituiertes Phenyl, für gegebenenfalls durch Fluor, Cyano, C₁-C₄-Alkoxy oder C₁-C₄-Alkoxy-carbonyl substituiertes C₁-C₄-Alkylamino, C₃-C₆-Cycloalkylamino oder für Di-(C₁-C₄-alkyl)-amino steht,
- R²: für gegebenenfalls durch Fluor, Chlor, Brom, Cyano, C₃-C₆-Cycloalkyl, C₁-C₄-Alkoxy oder C₁-C₄-Alkoxy-carbonyl substituiertes C₁-C₆-Alkyl, für jeweils gegebenenfalls durch Fluor, Chlor und/oder Brom substituiertes C₃-C₆-Alkenyl oder C₃-C₆-Alkinyl, für gegebenenfalls durch Fluor, Chlor, Brom und/oder C₁-C₄-Alkyl substituiertes C₃-C₆-Cycloalkyl, für Cyclohexenyl, für gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl-C₁-C₃-alkyl, oder für gegebenenfalls durch Fluor, Chlor, Brom, Cyano, Nitro, C₁-C₄-Alkyl, Trifluormethyl, C₁-C₄-Alkoxy, Fluor- und/oder Chlor-substituiertes C₁-C₃-Alkoxy, C₁-C₄-Alkylthio, Fluor-und/oder Chlor-substituiertes C₁-C₃-Alkylthio, C₁-C₄-Alkyl-sulfinyl, C₁-C₄-Alkylsulfonyl und/oder C₁-C₄-Alkoxy-carbonyl substituiertes Phenyl steht und
- R³: für die Gruppierung 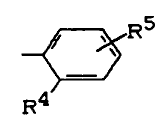 steht, worin
R⁴ und R⁵ gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxycarbonyl, C₁-C₄-Alkylamino-carbonyl, Di-(C₁-C₄-alkyl)amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist), für C₂-C₆-Alkenyl (welches gegebenenfalls durch Fluor, Chlor, Brom, Cyano, C₁-C₄-Alkoxy-carbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxy-carbonyl, C₁-C₄-Alkylthio, C₁-C₄-Alkylsulfinyl oder C₁-C₄-Alkylsulfonyl substituiert ist), für C₃-C₆-Alkenyloxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxycarbonyl substituiert ist), C₃-C₆-Alkinyloxy, C₃-C₆-Alkinylthio oder für den Rest -S(O)ₚ-R⁶ stehen, wobei
p für die Zahlen 1 oder 2 steht und
R⁶ für C₁-C₄-Alkyl (welches gegebenenfalls durch Fluor, Chlor, Brom, Cyano oder C₁-C₄-Alkoxy-carbonyl substituiert ist), C₃-C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
R⁷ für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkyl-carbonyl, C₁-C₄-Alkoxy-carbonyl, C₁-C₄-Alkylamino-carbonyl oder Di-(C₁-C₄-alkyl)-amino-carbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-Cycloalkyl-C₁-C₂-alkyl, Phenyl-C₁-C₂-alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio, Trifluormethylthio oder C₁-C₄-Alkoxy-carbonyl substituiert ist) steht,
- R⁴ und/oder R⁵: weiterhin für Phenyl oder Phenoxy, für C₁-C₄-Alkylcarbonylamino, C₁-C₄-Alkoxy-carbonylamino, C₁-C₄-Alkylamino-carbonyl-amino, Di-(C₁-C₄-alkyl)-amino-carbonylamino, oder für den Rest -CO-R⁸ stehen, wobei
R⁸ für C₁-C₆-Alkyl, C₁-C₆-Alkoxy, C₃-C₆-Cycloalkoxy, C₃-C₆-Alkenyloxy, C₁-C₄ Alkylthio, C₁-C₄-Alkylamino, C₁-C₄-Alkoxyamino, C₁-C₄-Alkoxy-C₁-C₄-alkyl-amino oder Di-(C₁-C₄-alkyl)-amino steht (welche gegebenenfalls durch Fluor und/oder Chlor substituiert sind),
- R⁴ und/oder R⁵: weiterhin für Trimethylsilyl, Thiazolinyl, C₁-C₄-Alkylsulfonyloxy, Di-(C₁-C₄-alkyl)-aminosulfonylamino oder für den Rest -CH=N-R⁹ stehen, wobei
R⁹ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/oder Chlor substituiertes C₁-C₆-Alkoxy, C₃-C₆-Alkenoxy, C₃-C₆-Alkinoxy oder Benzyloxy, für Amino, C₁-C₄-Alkylamino, Di-(C₁-C₄-alkyl)-amino, Phenylamino, C₁-C₄-Alkyl-carbonyl-amino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht,
weiterhin
- R³: für den Rest 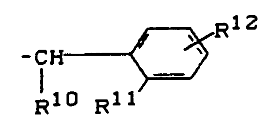 steht, worin
R¹⁰ für Wasserstoff oder C₁-C₄-Alkyl steht,
R¹¹ und R¹² gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), Carboxy, C₁-C₄-Alkoxy-carbonyl, Dimethylaminocarbonyl, C₁-C₄-Alkylsulfonyl oder Di-(C₁-C₄-alkyl)-aminosulfonyl stehen;
weiterhin
- R³: für den Rest 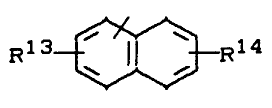 steht, worin
R¹³ und R¹⁴ gleich oder verschieden sind und für Wasserstoff, Fluor, Chlor, Brom, Nitro, Cyano, C₁-C₄-Alkyl (welches gegebenenfalls durch Fluor und/oder Chlor substituiert ist) oder C₁-C₄-Alkoxy (welches gegebenenfalls durch Fluor und/oder Chlor substituiert ist), stehen;
weiterhin
- R³: für den Rest 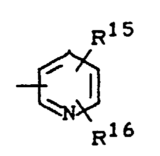 steht, worin
R¹⁵ und R¹⁶ gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), für C₁-C₄-Alkylthio, C₁-C₄-Alkylsulfinyl oder C₁-C₄-Alkylsulfonyl (welche gegebenenfalls durch Fluor und/ oder Chlor substituiert sind), für Aminosulfonyl, Mono-(C₁-C₄-alkyl)-aminosulfonyl, für Di-(C₁-C₄-alkyl)-aminosulfonyl oder C₁-C₄-Alkoxy-carbonyl oder Dimethylaminocarbonyl stehen;
weiterhin
- R³: für den Rest 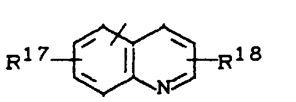 steht, worin
R¹⁷ und R¹⁸ gleich oder verschieden sind und für Wasserstoff, Fluor, Chlor, Brom, C₁-C₄-Alkyl (welches gegebenenfalls durch Fluor und/oder Brom substituiert ist), C₁-C₄-Alkoxy (welches gegebenenfalls durch Fluor und/oder Chlor substituiert ist), für C₁-C₄-Alkylthio, C₁-C₄-Alkylsulfinyl oder C₁-C₄-Alkylsulfonyl (welche gegebenenfalls durch Fluor und/oder Chlor substituiert sind), oder für Di-(C₁-C₄-Alkyl)-aminosulfonyl stehen;
weiterhin
- R³: für den Rest 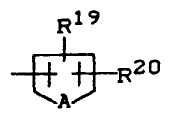 steht, worin
R¹⁹ und R²⁰ gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), C₁-C₄-Alkylthio-C₁-C₄-Alkylsulfinyl oder C₁-C₄-Alkylsulfonyl (welches gegebenenfalls durch Fluor und/oder Chlor substituiert ist), Di-(C₁-C₄-alkyl)-amino-sulfonyl, C₁-C₄-Alkoxy-carbonyl oder Dimethylaminocarbonyl stehen, und
A für Sauerstoff, Schwefel oder die Gruppierung N-Z¹ steht, wobei
Z¹ für Wasserstoff, C₁-C₄-Alkyl (welches gegebenenfalls durch Fluor, Chlor, Brom oder Cyano substituiert ist), C₃-C₆-Cycloalkyl, Benzyl, Phenyl (welches gegebenenfalls durch Fluor, Chlor, Brom oder Nitro substituiert ist), C₁-C₄-Alkylcarbonyl, C₁-C₄-Alkoxy-carbonyl oder Di-(C₁-C₄-alkyl)-aminocarbonyl steht;
weiterhin
- R³: für den Rest 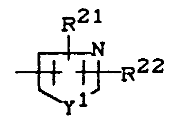 steht, worin
R²¹ und R²² gleich oder verschieden sind und für Wasserstoff, C₁-C₄-Alkyl, Halogen, C₁-C₄-Alkoxycarbonyl, C₁-C₄-Alkoxy oder C₁-C₄-Halogenalkoxy stehen,
Y¹ für Schwefel oder die Gruppierung N-R²³ steht, wobei
R²³ für Wasserstoff oder C₁-C₄-Alkyl steht;
weiterhin
- R³: für den Rest 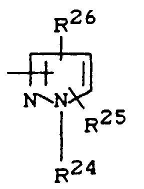 steht, worin
R²⁴ für Wasserstoff, C₁-C₄-Alkyl, Benzyl, Pyridyl, Chinolinyl oder Phenyl steht,
R²⁵ für Wasserstoff, Halogen, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Dioxolanyl oder C₁-C₄-Alkoxy-carbonyl steht und
R²⁶ für Wasserstoff, Halogen oder C₁-C₄-Alkyl steht;
weiterhin
- R³: für eine der nachstehend aufgeführten Gruppierungen steht, 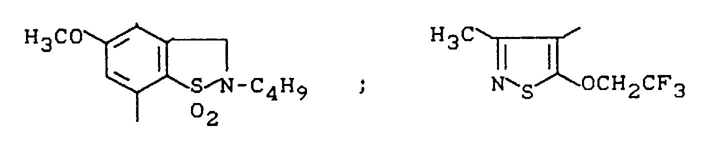 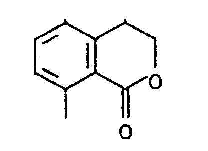
sowie Salze von Verbindungen der Formel (I),
wobei jedoch die Verbindungen
2-(2-Methoxycarbonyl-phenylsulfonylaminocarbonyl)-4-methyl-5-methoxy-2,4-dihydro-3H-1,2,4-triazol-3-on,
2-(2-Trifluormethoxy-phenylsulfonylaminocarbonyl)-4-cyclopropyl-5-methoxy-2,4-dihydro-3H-1,2,4-triazol-3-on und
2-(2-Difluormethoxy-phenylsulfonylaminocarbonyl)-4-cyclobutyl-5-ethoxy-2,4-dihydro-3H-1,2,4-triazol-3-on ausgeschlossen sind (vgl. DE-A-3934081/EP-A-0422469).

Man erhält die neuen Sulfonylaminocarbonyltriazolinone mit über Sauerstoff gebundenen Substituenten der allgemeinen Formel (I), wenn man
(a) Triazolinone der allgemeinen Formel (II) 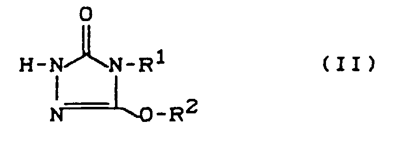 in welcher
   - R¹ und R²: die oben angegebenen Bedeutungen haben,
   mit Sulfonylisocyanaten der allgemeinen Formel (III)

   R³-SO₂-N=C=O (III)

   in welcher
   - R³: die oben angegebene Bedeutung hat,
   gegebenenfalls in Gegenwart eines Verdünnungsmittels umsetzt, oder wenn man
(b) Triazolinon-Derivate der allgemeinen Formel (IV) 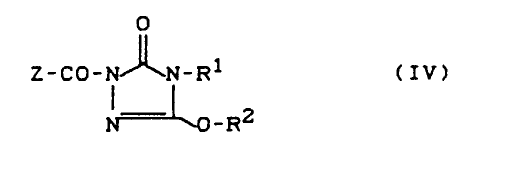 in welcher
   - R¹ und R²: die oben angegebenen Bedeutungen haben und
   - Z: für Chlor, C₁-C₄-Alkoxy, Benzyloxy oder Phenoxy steht,
   mit Sulfonsäureamiden der allgemeinen Formel (V)

   R³-SO₂-NH₂ (V)

   in welcher
   - R³: die oben angegebene Bedeutung hat,
   gegebenenfalls in Gegenwart eines Säureakzeptors und gegebenenfalls in Gegenwart eines Verdünnungsmittels umsetzt, oder wenn man
(c) Triazolinone der allgemeinen Formel (II) 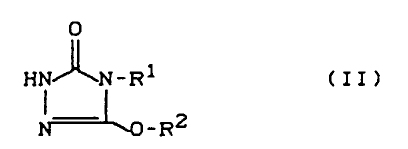 in welcher
   - R¹ und R²: die oben angegebenen Bedeutungen haben,
   mit Sulfonsäureamid-Derivaten der allgemeinen Formel (VI)

   R³-SO₂-NH-CO-Z (VI)

   in welcher
   - R³: die oben angegebene Bedeutung hat und
   - Z: für Chlor, C₁-C₄-Alkoxy, Benzyloxy oder Phenoxy steht,
   gegebenenfalls in Gegenwart eines Säureakzeptors und gegebenenfalls in Gegenwart eines Verdünnungsmittels umsetzt und gegebenenfalls aus den nach Verfahren (a), (b) oder (c) hergestellten Verbindungen der Formel (I) nach üblichen Methoden Salze erzeugt.

Die neuen Sulfonylaminocarbonyltriazolinone mit über Sauerstoff gebundenen Substituenten der allgemeinen Formel (1) und ihre Salze zeichnen sich durch starke herbizide Wirksamkeit aus.

Überraschenderweise zeigen die neuen Verbindungen der Formel (I) erheblich bessere herbizide Wirkung als die strukturell ähnliche bekannte Verbindung 2-(2-Chlorphenylsulfonylaminocarbonyl)-4,5-dimethyl-2,4-dihydro-3H-1,2,4-triazol-3-on.

Gegenstand der Erfindung sind vorzugsweise Verbindungen der Formel (I), in welcher
- R¹: für Wasserstoff, Amino, für gegebenenfalls durch Fluor, Cyano, Methoxy oder Ethoxy substituiertes C₁-C₄-Alkyl, für Allyl, für C₃-C₆-Cycloalkyl, für Benzyl, für Phenyl, für C₁-C₃-Alkylamino, C₃-C₆-Cycloalkylamino oder für Di-(C₁-C₃-alkyl)-amino steht,
- R²: für Wasserstoff, für gegebenenfalls durch Fluor und/oder Chlor, Methoxy oder Ethoxy substituiertes C₁-C₄-Alkyl, für gegebenenfalls durch Fluor und/ oder Chlor substituiertes C₃-C₄-Alkenyl, für C₃-C₆-Cycloalkyl oder für gegebenenfalls durch Fluor, Chlor und/oder Methyl substituiertes Benzyl steht, und
- R³: für die Gruppierung 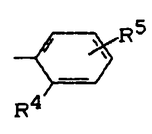 steht, worin
R⁴ für Fluor, Chlor, Brom, Methyl, Trifluormethyl, Methoxy, Difluormethoxy, Trifluormethoxy, 2-Chlor-ethoxy, 2-Methoxy-ethoxy, C₁-C₃-Alkylthio, C₁-C₃-Alkylsulfinyl, C₁-C₃-Alkylsulfonyl, Dimethylaminosulfonyl, Diethylaminosulfonyl, N-Methoxy-N-methylaminosulfonyl, Methoxyaminosulfonyl, Phenyl, Phenoxy oder C₁-C₃-Alkoxy-carbonyl steht und
R⁵ für Wasserstoff, Fluor, Chlor oder Brom steht;
weiterhin
- R³: für den Rest 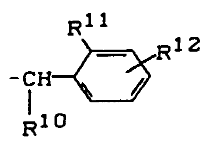 steht, worin
R¹⁰ für Wasserstoff steht,
R¹¹ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
R¹² für Wasserstoff steht;
weiterhin
- R³: für den Rest 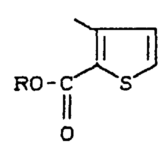 steht,
worin R für C₁-C₄-Alkyl steht, oder
für den Rest 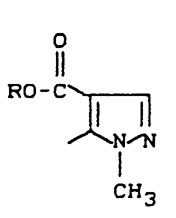 steht, worin R für C₁-C₄-Alkyl steht,
sowie Salze von Verbindungen der Formel (I), wobei jedoch die Verbindungen
2-(2-Methoxycarbonyl-phenylsulfonylaminocarbonyl)-4-methyl-5-methoxy-2,4-dihydro-3H-1,2,4-triazol-3-on,
2-(2-Trifluormethoxy-phenylsulfonylaminocarbonyl)-4-cyclopropyl-5-methoxy-2,4-dihydro-3H-1,2,4-triazol-3-on und
2-(2-Difluormethoxy-phenylsulfonylaminocarbonyl)-4-cyclobutyl-5-ethoxy-2,4-dihydro-3H-1,2,4-triazol-3-on ausgeschlossen sind.

Gegenstand der Erfindung sind weiter vorzugsweise Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, C₅- oder C₆-Cycloalkyl-ammonium- und Di-(C₁-C₂-alkyl)-benzyl-ammonium-Salze von Verbindungen der Formel (I).

Die oben aufgeführten allgemeinen oder in Vorzugsbereichen aufgefürhrten Restedefinitionen können untereinander, also auch zwischen den jeweiligen Vorzugsbereichen beliebig kombiniert werden.

Beispiele für die erfindungsgemäßen Verbindungen sind in der nachstehenden Tabelle 1 aufgeführt - vgl. auch die Herstellungsbeispiele.

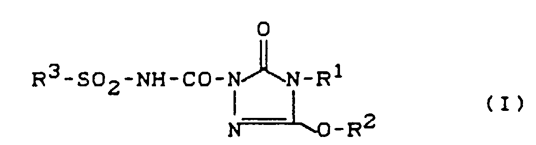

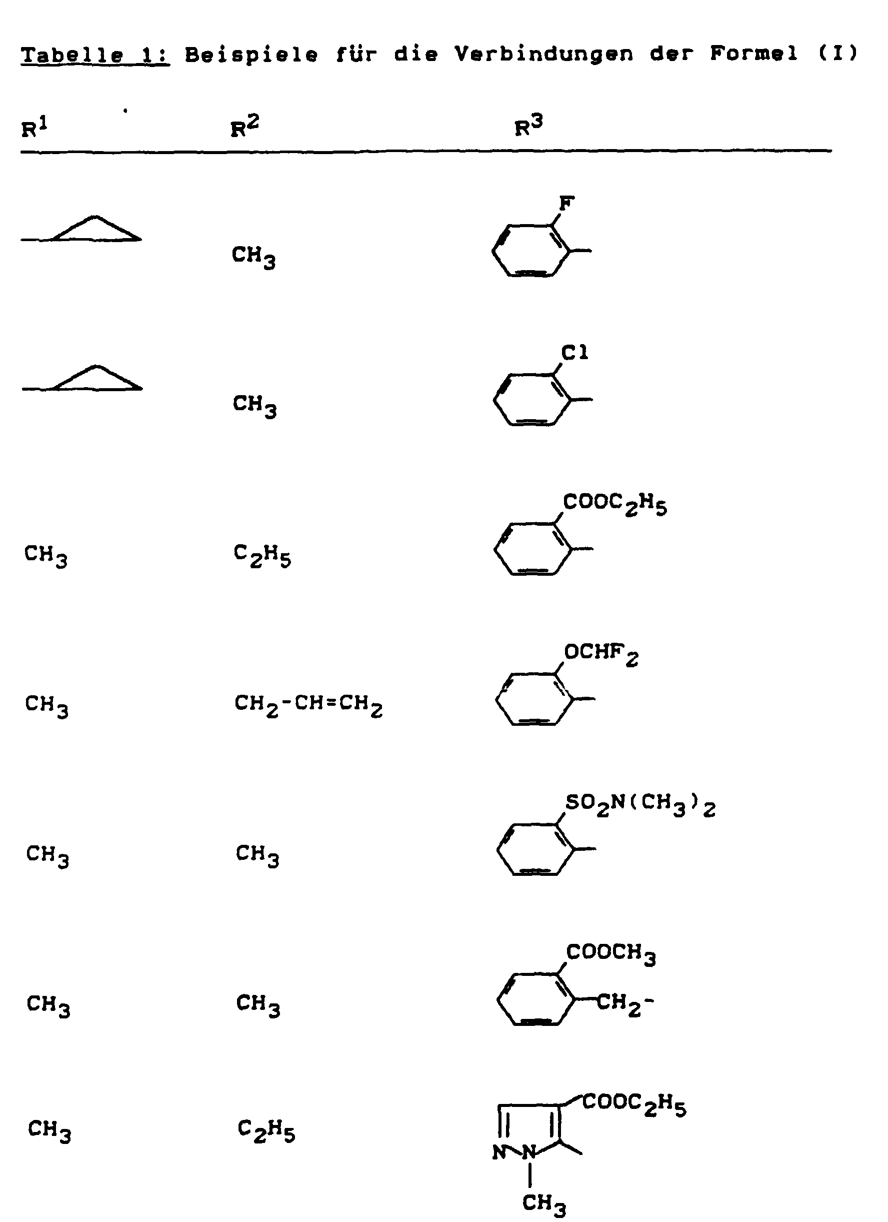

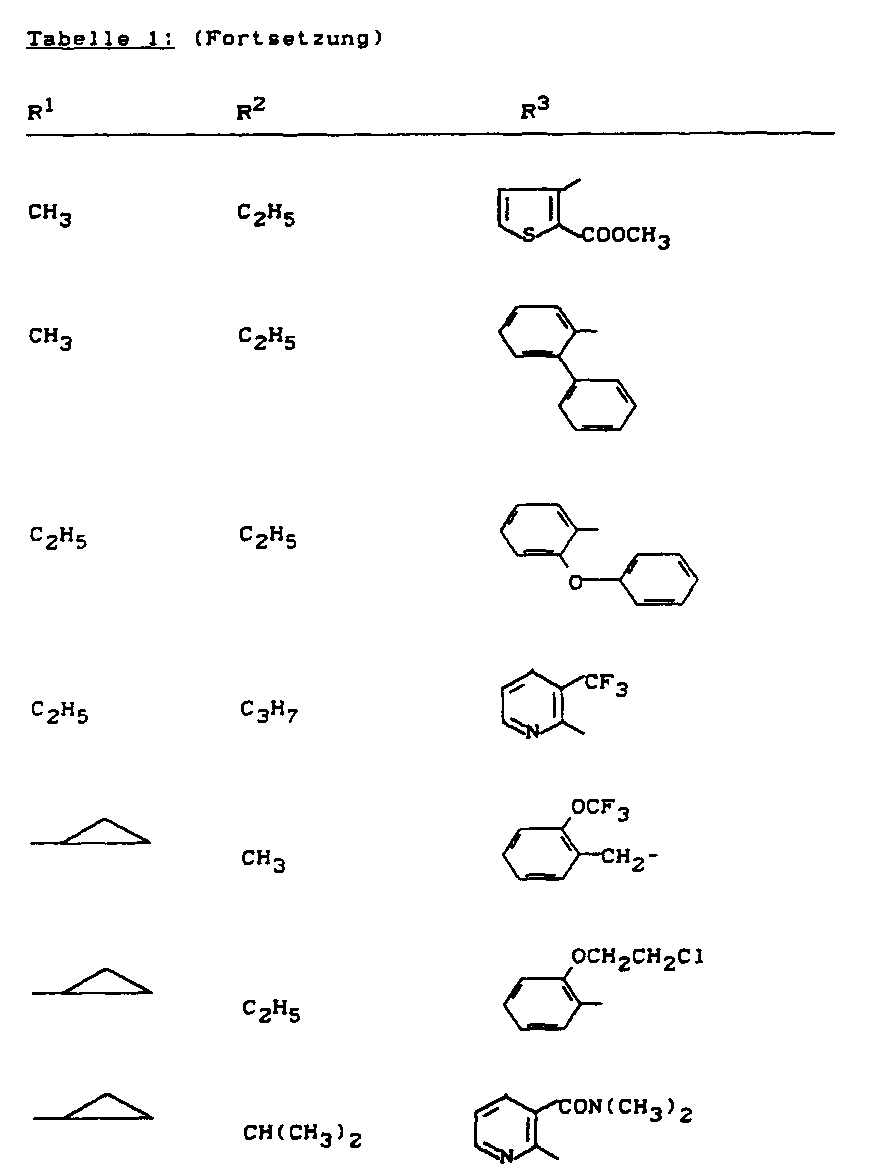

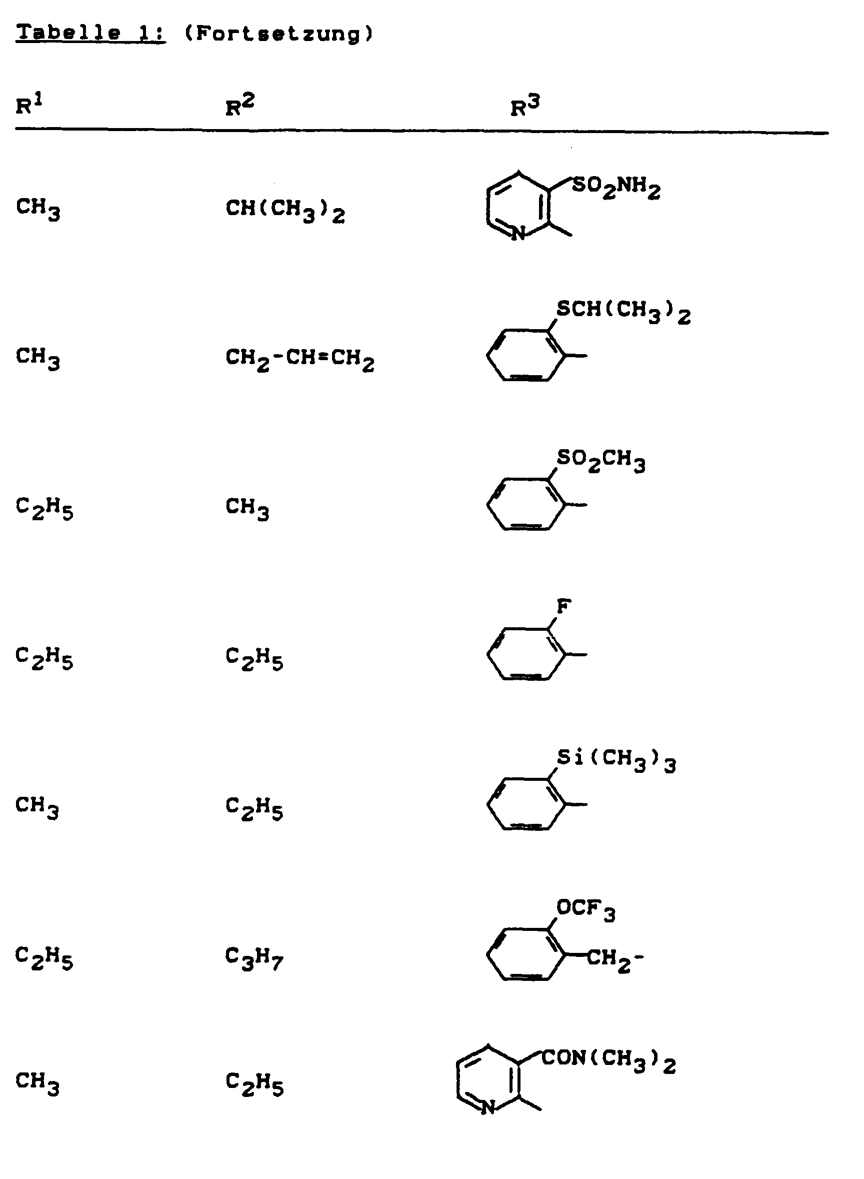

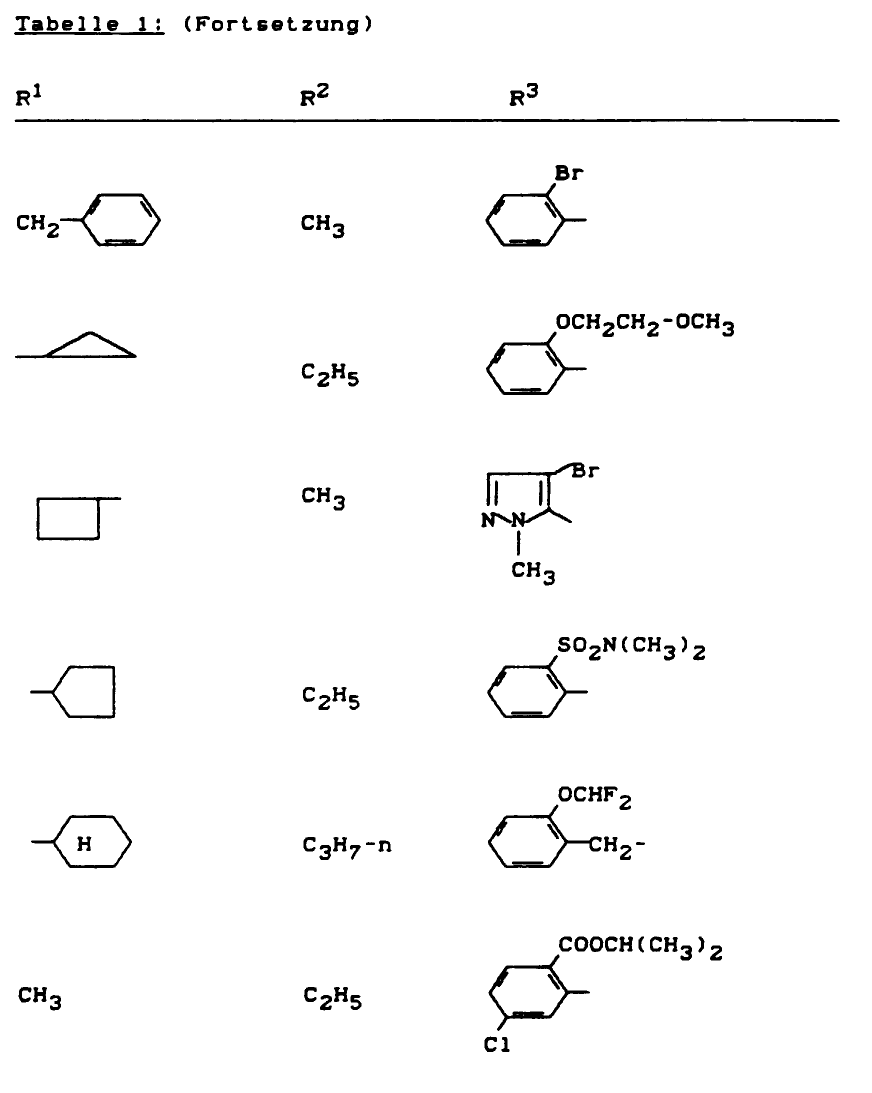

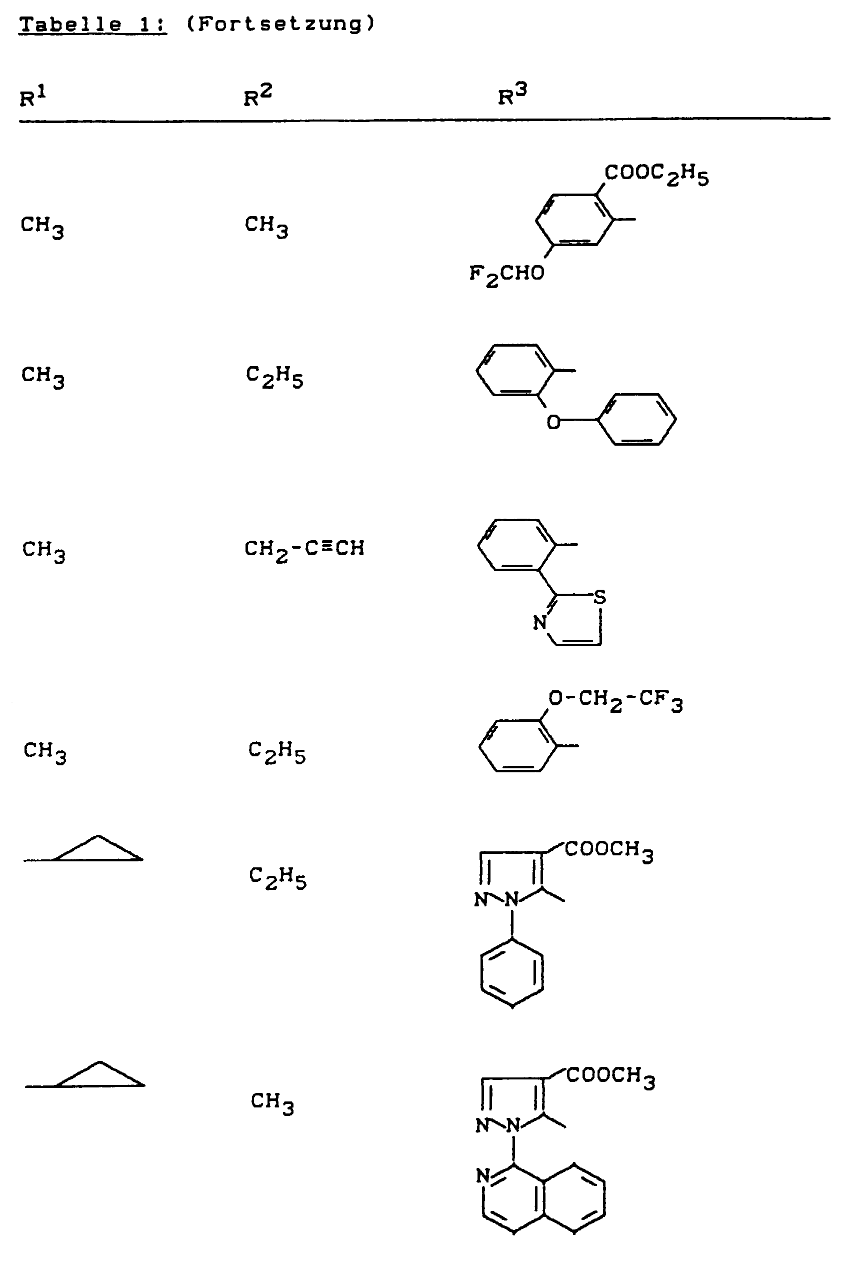

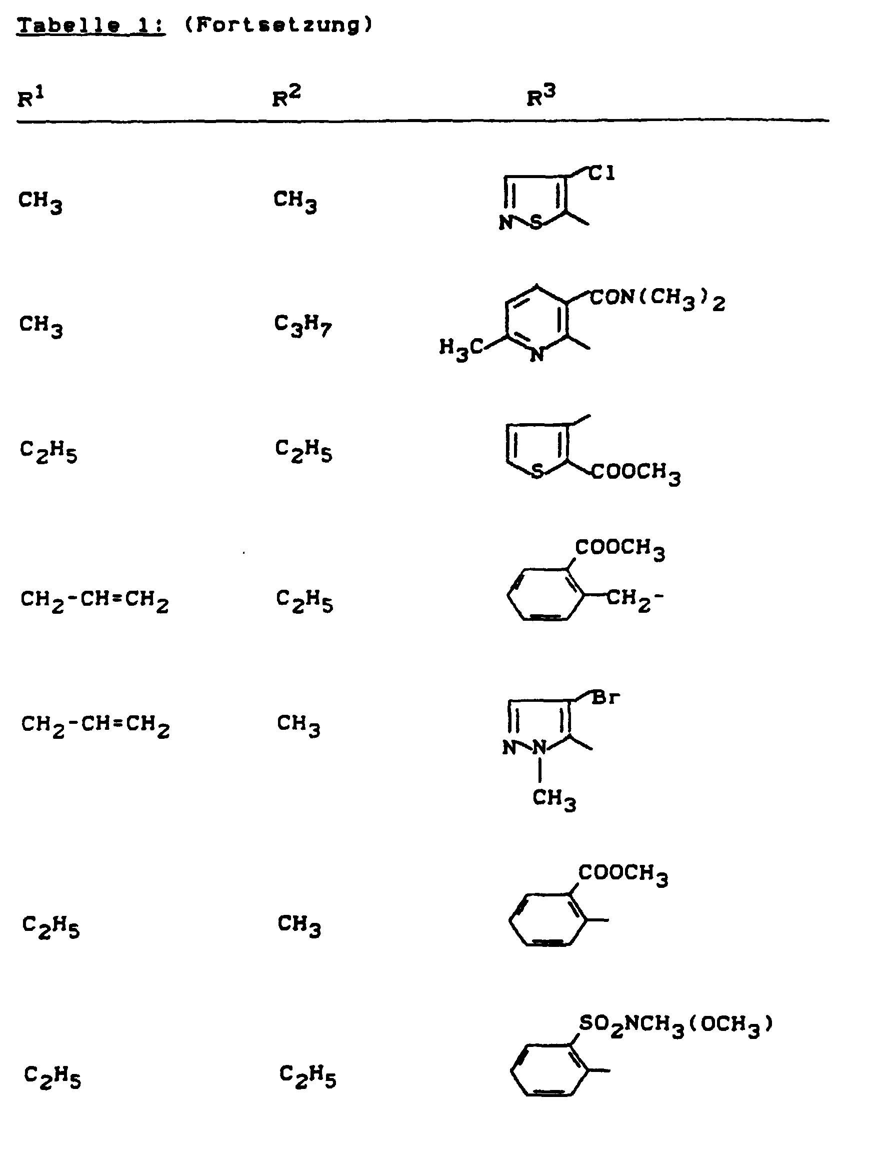

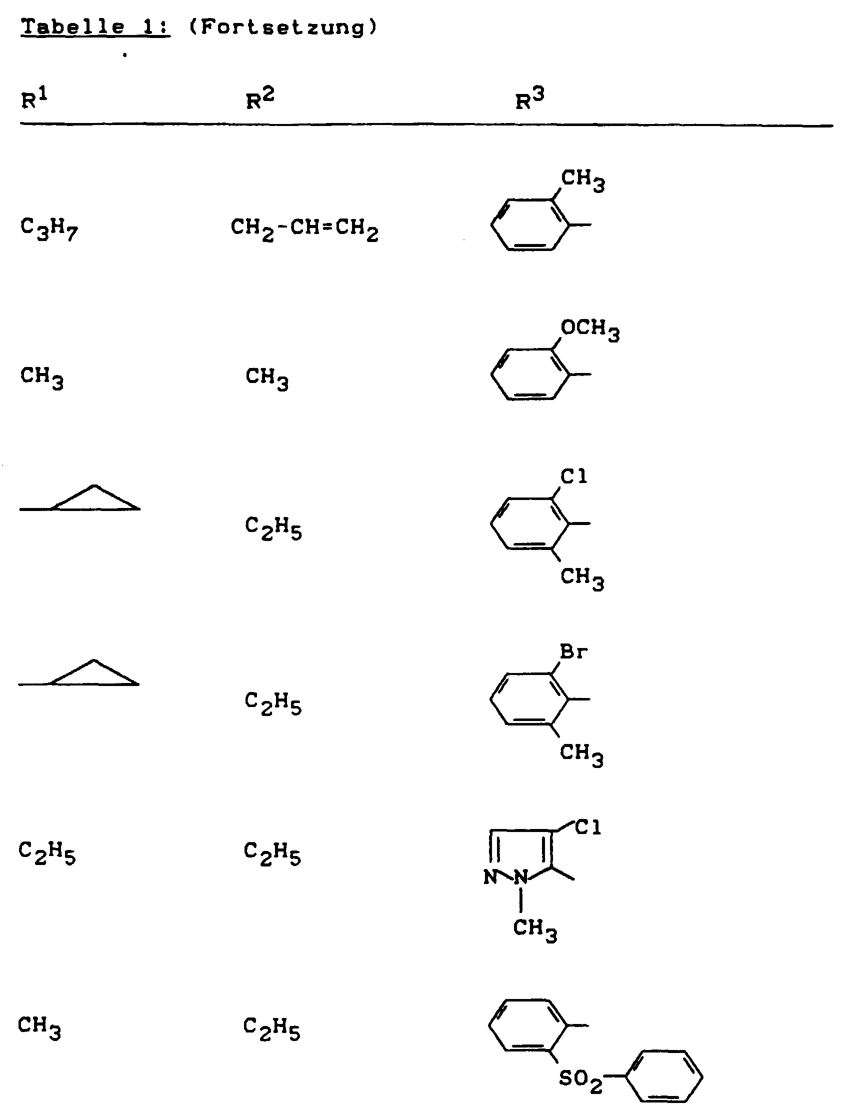

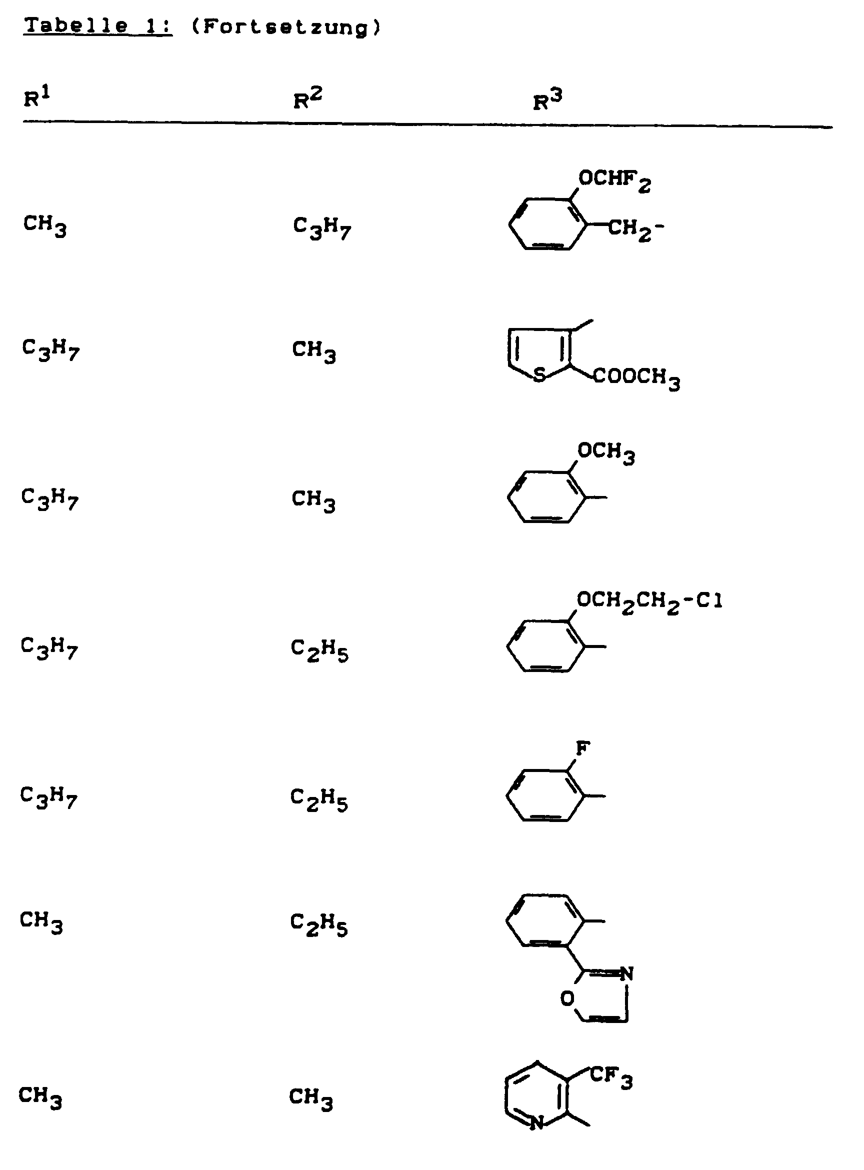

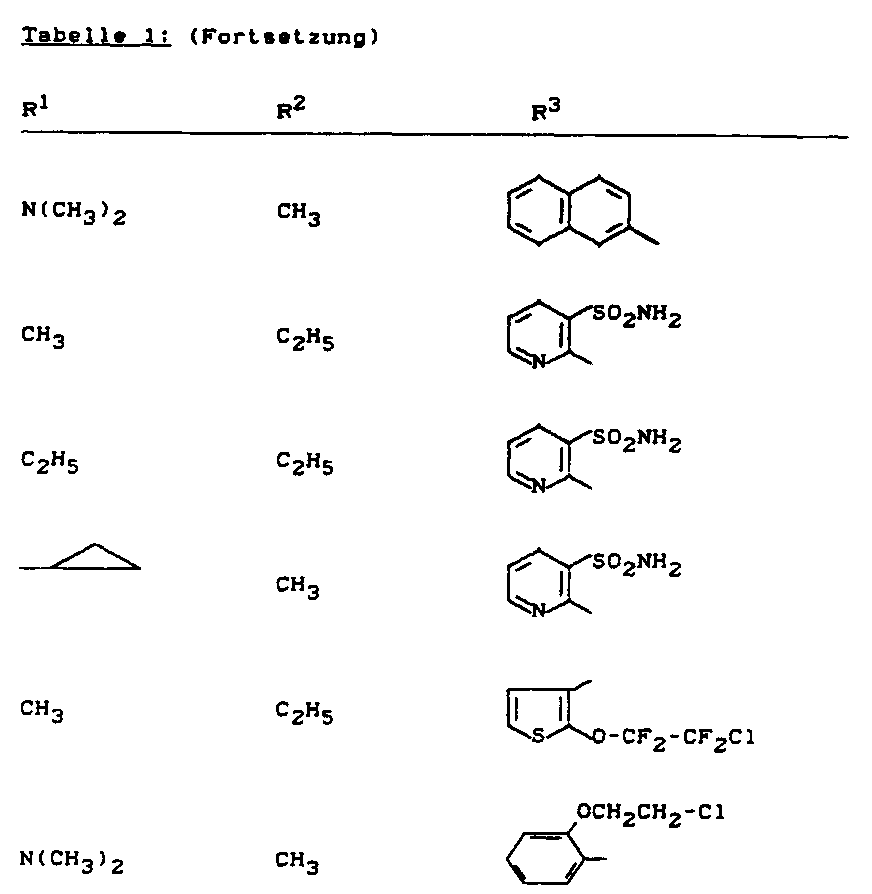

Verwendet man beispielsweise 2,6-Difluor-phenylsulfonylisocyanat und 5-Ethoxy-4-methyl-2,4-dihydro-3H-1,2,4-triazol-3-on als Ausgangsstoffe, so kann der Reaktionsablauf beim erfindungsgemäßen Verfahren (a) durch folgendes Formelschema skizziert werden:

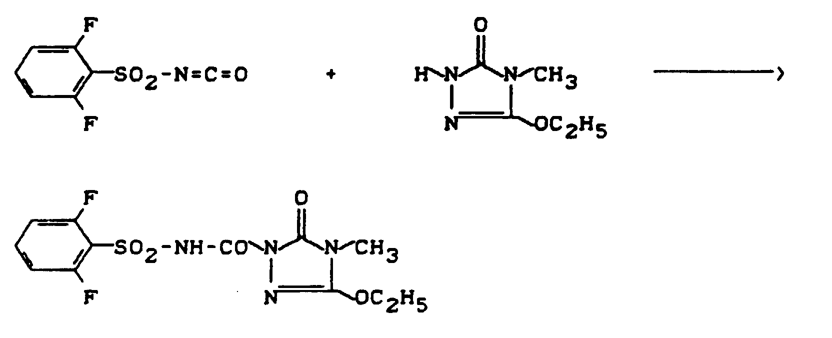

Verwendet man beispielsweise 2-Methylthio-benzolsulfonsäureamid und 2-Chlorcarbonyl-4-dimethylamino-5-propyloxy-2,4-dihydro-3H-1,2,4-triazol-3-on als Ausgangsstoffe, so kann der Reaktionsablauf beim erfindungsgemäßen Verfahren (b) durch folgendes Formelschema skizziert werden:

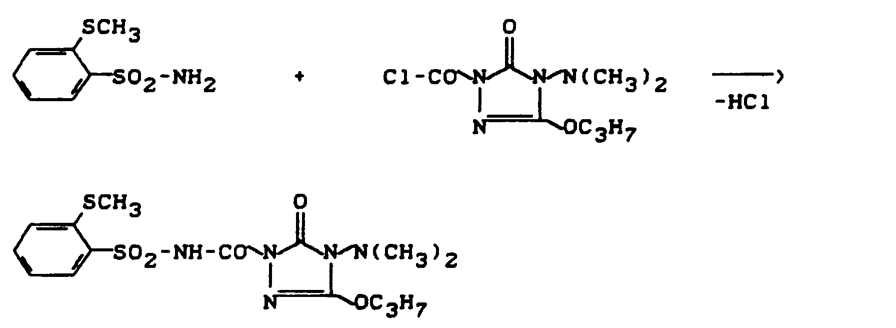

Verwendet man beispielsweise N-Methoxycarbonyl-2-methoxy-benzolsulfonsäureamid und 5-Methoxy-4-difluormethyl-2,4-dihydro-3H-1,2,4-triazol-3-on als Ausgangsstoffe, so kann der Reaktionsablauf beim erfindungsgemäßen Verfahren (c) durch das folgende Formelschema skizziert werden:

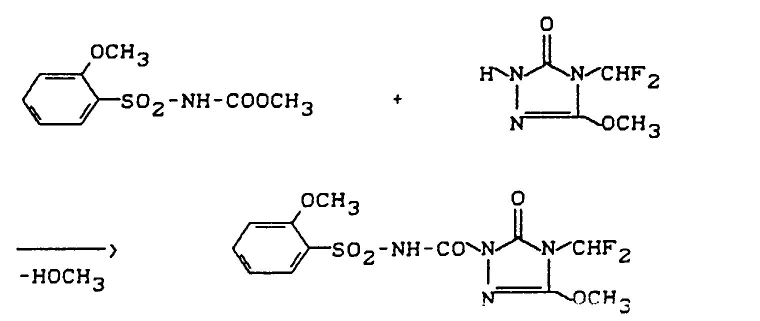

Die bei den erfindungsgemäßen Verfahren (a) und (c) zur Herstellung von Verbindungen der Formel (I) als Ausgangsstoffe zu verwendenden Triazolinone sind durch die Formel (II) allgemein definiert.

In Formel (II) haben R¹ und R² vorzugsweise diejenigen Bedeutungen, die bereits oben im Zusammenhang mit der Beschreibung der erfindungsgemäßen Verbindungen der Formel (I) vorzugsweise für R¹ und R² angegeben wurden.

Beispiele für die Ausgangsstoffe der Formel (II) sind in der nachstehenden Tabelle 2 aufgeführt.

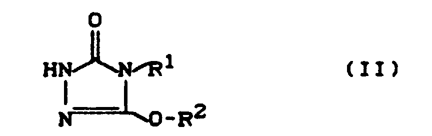

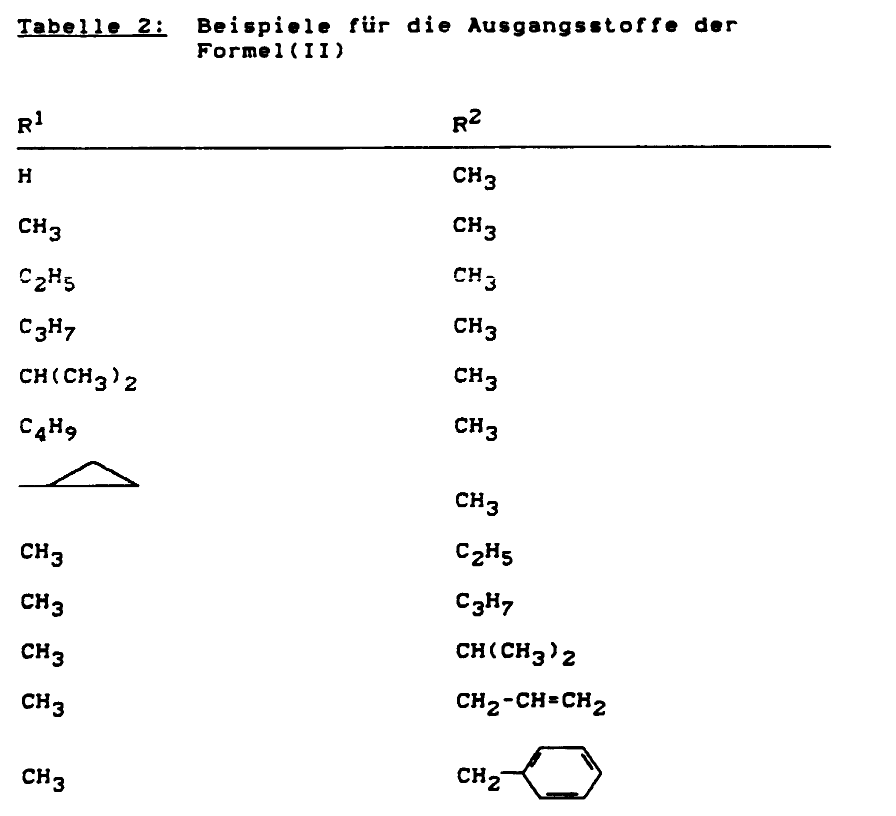

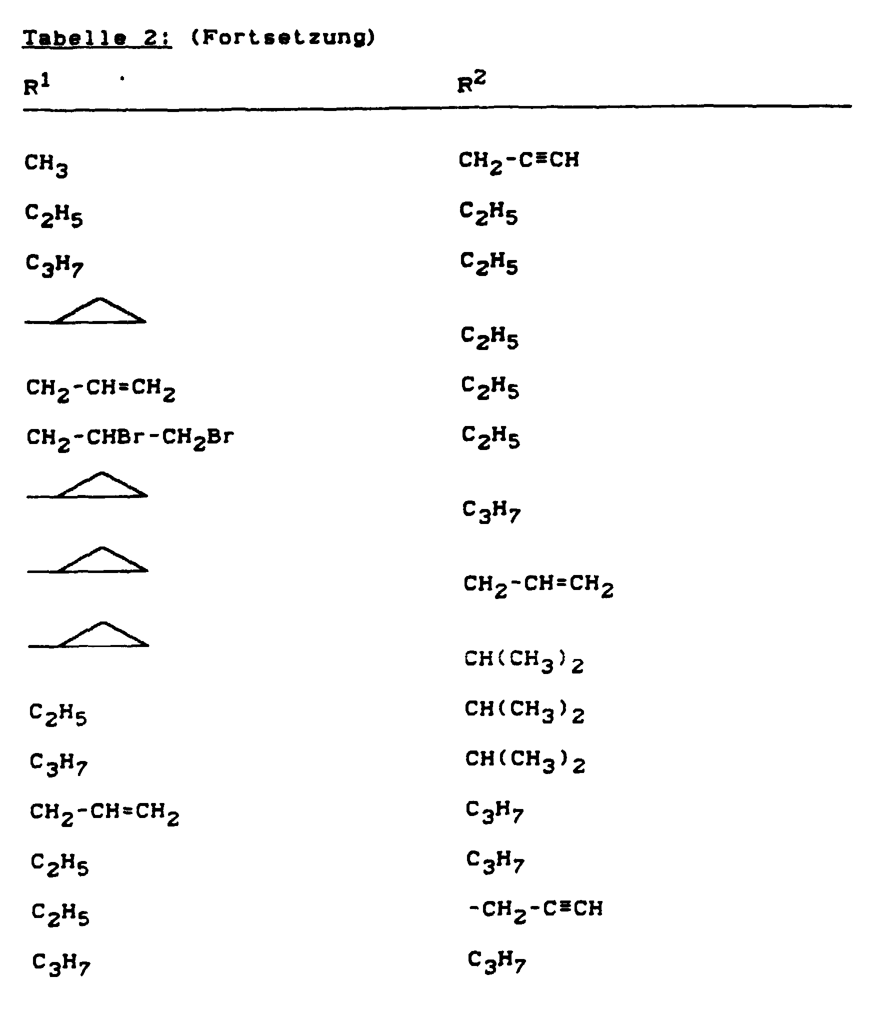

Die Ausgangsstoffe der Formel (II) sind zum Teil Gegenstand einer vorgängigen, jedoch nicht vorveröffentlichten Patentanmeldung (vgl. DE-P 4030063 vom 22.09.1990).

Neu und Gegenstand der vorliegenden Patentanmeldung sind diejenigen Verbindungen der allgemeinen Formel (II), bei welchen R¹ die oben angegebene Bedeutung hat und R² für jeweils gegebenenfalls substituiertes Cycloalkyl, Cycloalkenyl, Aralkyl oder Aryl steht (wobei die exakte Definition dieser Reste oben bei Formel (I) angegeben ist). Man erhält die Verbindungen der Formel (II), wenn man Hydrazinoameisensäureester der allgemeinen Formel (VII)

H₂N-NH-CO-O-R²⁷ (VII)

in welcher
- R²⁷: für Methyl, Ethyl oder Phenyl steht,
mit Alkyliminokohlensäurediestern der allgemeinen Formel (VIII) 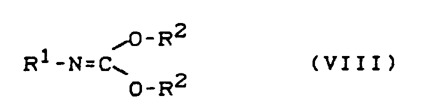 in welcher
- R¹ und R²: die oben angegebene Bedeutung haben,
gegebenenfalls in Gegenwart eines Verdünnungsmittels, wie z.B. Methanol, bei Temperaturen zwischen 0°C und 50°C umsetzt, die hierbei gebildeten Verbindungen der allgemeinen Formel (IX) 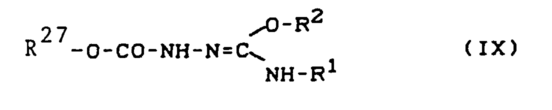 in welcher
- R¹ und R²: die oben angegebene Bedeutung haben und
- R²⁷: für Methyl, Ethyl oder Phenyl steht,
gegebenenfalls nach üblichen Methoden isoliert und auf Temperaturen zwischen 50°C und 150°C, gegebenenfalls in Gegenwart eines Verdünnungsmittels, wie z.B. Toluol, Xylol oder o-Dichlorbenzol, erhitzt (vgl. die Herstellungsbeispiele).

Die Ausgangsstoffe der Formeln (VII) und (VIII) sind bekannte Chemikalien.

Die Zwischenprodukte der Formel (IX) sind neue Verbindungen,

Die beim erfindungsgemäßen Verfahren (a) zur Herstellung von Verbindungen der Formel (I) weiter als Ausgangsstoffe zu verwendenden Sulfonylisocyanate sind durch die Formel (III) allgemein definiert.

In Formel (III) hat R³ vorzugsweise diejenige Bedeutung, die bereits oben im Zusammenhang mit der Beschreibung der erfindungsgemäßen Verbindungen der Formel (I) vorzugsweise für R³ angegeben wurde.

Als Beispiele für die Ausgangsstoffe der Formel (III) seien genannt:
2-Fluor-, 2-Chlor-, 2-Brom-, 2-Methyl-, 2-Methoxy-, 2-Trifluormethyl-, 2-Difluor-methoxy-, 2-Trifluormethoxy-, 2-Methylthio-, 2-Ethylthio-, 2-Propylthio-, 2-Methylsulfinyl-, 2-Methylsulfonyl-, 2-Dimethylaminosulfonyl-, 2-Diethylaminosulfonyl-, 2-(N-Methoxy-N-methyl)-aminosulfonyl-, 2-Phenyl-, 2-Phenoxy-, 2-Methoxycarbonyl-, 2-Ethoxycarbonyl-, 2-Propoxycarbonyl- und 2-Isopropoxycarbonyl-phenylsulfonylisocyanat, 2-Fluor-, 2-Chlor-, 2-Difluormethoxy-, 2-Trifluormethoxy-, 2-Methoxycarbonyl- und 2-Ethoxycarbonyl-benzylsulfonylisocyanat, 2-Methoxycarbonyl-3-thienyl-sulfonyl isocyanat, 4-Methoxycarbonyl- und 4-Ethoxycarbonyl-1-methyl-pyrazol-5-yl-sulfonylisocyanat.

Die Sulfonylisocyanate der Formel (III) sind bekannt und/oder können nach an sich bekannten Verfahren hergestellt werden (vgl. US-P 4 127 405, 4 169 719, 4 371 391; EP-A 7 687, 13 480, 21 641, 23 141, 23 422, 30 139, 35 893, 44 808, 44 809, 48 143, 51 466, 64 322, 70 041, 173 312).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 10 °C und 80 °C.

Das erfindungsgemäße Verfahren (a) wird im allgemeinen bei Normaldruck durchgeführt.

Zur Durchführung des erfindungsgemäßen Verfahrens (a) setzt man je Mol Triazolinon der Formel (II) im allgemeinen zwischen 1 und 3 Mol, vorzugsweise zwischen 1 und 2 Mol, Sulfonylisocyanat der Formel (III) ein.

Die Reaktionskomponenten können in beliebiger Reihenfolge zusammengegeben werden. Das Reaktionsgemisch wird bis zum Ende der Umsetzung gerührt und das Produkt durch Absaugen isoliert. In einer anderen Aufarbeitungsvariante wird eingeengt und das im Rückstand verbleibende Rohprodukt mit einem geeigneten Lösungsmittel, wie z. B. Diethylether, zur Kristallisation gebracht. Das hierbei kristallin angefallene Produkt der Formel (I) wird durch Absaugen isoliert.

Die beim erfindungsgemäßen Verfahren (b) zur Herstellung von Verbindungen der Formel (I) als Ausgangsstoffe zu verwendenden Triazolinon-Derivate sind durch die Formel (IV) allgemein definiert.

In Formel (IV) haben R¹ und R² vorzugsweise diejenigen Bedeutungen, die bereits oben im Zusammenhang mit der Beschreibung der erfindungsgemäßen Verbindungen der Formel (I) vorzugsweise für R¹ und R² angegeben wurden und
- Z: steht für Chlor, C₁-C₄-Alkoxy, Benzyloxy oder Phenoxy, vorzugsweise für Methoxy oder Phenoxy.

Mögliche Beispiele für die Ausgangsstoffe der Formel (IV) sind die aus den in Tabelle 2 aufgeführten Verbindungen der Formel (II) und Phosgen, Chlorameisensäuremethylester, Chlorameisensäure-benzylester, Chlorameisensäurephenylester oder Diphenylcarbonat herzustellenden Verbindungen der Formel (IV).

Die Ausgangsstoffe der Formel (IV) sind noch nicht bekannt.

Man erhält die neuen Triazolinon-Derivate der Formel (IV), wenn man Triazolinone der allgemeinen Formel (II) 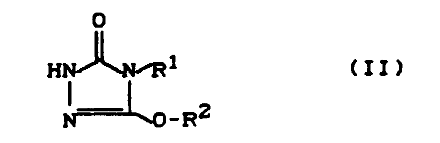 in welcher
- R¹ und R²: die oben angegebenen Bedeutungen haben,
mit Kohlensäurederivaten der allgemeinen Formel (X)

Z-CO-Z¹ (X) (X)

in welcher
- Z: die oben angegebene Bedeutung hat und
- Z¹: für eine Abgangsgruppe wie Chlor, Methoxy, Benzyloxy oder Phenoxy steht,
gegebenenfalls in Gegenwart eines Verdünnungsmittels, wie z.B. Tetrahydrofuran, und gegebenenfalls in Gegenwart eines Säureakzeptors, wie z.B. Natriumhydrid oder Kalium-tert-butylat, bei Temperaturen zwischen -20°C und +100°C umsetzt.

Die beim erfindungsgemäßen Verfahren (b) zur Herstellung von Verbindungen der Formel (I) weiter als Ausgangsstoffe zu verwendenden Sulfonsäureamide sind durch die Formel (V) allgemein definiert.

In Formel (V) hat R³ vorzugsweise diejenige Bedeutung, die bereits oben im Zusammenhang mit der Beschreibung der erfindungsgemäßen Verbindungen der Formel (I) vorzugsweise für R³ angegeben wurde.

Als Beispiele für die Ausgangsstoffe der Formel (V) seien genannt:
2-Fluor-, 2-Chlor-, 2-Brom-, 2-Methyl-, 2-Methoxy-, 2-Trifluormethyl-, 2-Difluor-methoxy-, 2-Trifluormethoxy-, 2-Methylthio-, 2-Ethylthio-, 2-Propylthio-, 2-Methylsulfinyl-, 2-Methylsulfonyl-, 2-Dimethylaminosulfonyl-, 2-Diethylaminosulfonyl-, 2-(N-Methoxy-N-methyl)-aminosulfonyl-, 2-Phenyl-, 2-Phenoxy-, 2-Methoxycarbonyl-, 2-Ethoxycarbonyl-, 2-Propoxycarbonyl- und 2-Isopropoxycarbonyl-benzolsulfonsäureamid, 2-Fluor-, 2-Chlor-, 2-Difluormethoxy-, 2-Trifluormethoxy-, 2-Methoxycarbonyl- und 2-Ethoxycarbonyl-phenylmethansulfonsäureamid, 2-Methoxycarbonyl-3-thiophensulfonsäureamid, 4-Methoxycarbonyl-und 4-Ethoxycarbonyl-1-methyl-pyrazol-5-sulfonsäureamid.

Die Sulfonsäureamide der Formel (V) sind bekannt und/ oder können nach an sich bekannten Verfahren hergestellt werden (vgl. US-P 4 127 405, 4 169 719, 4 371 391; EP-A 7 687, 13 480, 21 641, 23 141, 23 422, 30 139, 35 893, 44 808, 44 809, 48 143, 51 466, 64 322, 70 041, 173 312).

Das erfindungsgemäße Verfahren (b) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Betracht, wie sie beispielsweise oben für das erfindungsgemäße Verfahren (a) angegeben sind.

Als Säureakzeptoren können bei dem erfindungsgemäßen Verfahren (b) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen 0 °C und 100 °C, vorzugsweise bei Temperaturen zwischen 10 °C und 60 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden.

Die beim erfindungsgemäßen Verfahren (c) zur Herstellung von Verbindungen der Formel (I) als Ausgangsstoffe zu verwendenden Triazolinone der Formel (II) sind bereits als Ausgangsstoffe für das erfindungsgemäße Verfahren (a) beschrieben worden.

Die beim erfindungsgemäßen Verfahren (c) zur Herstellung von Verbindungen der Formel (I) weiter als Ausgangsstoffe zu verwendenden Sulfonsäureamid-Derivate sind durch die Formel (VI) allgemein definiert.

In Formel (VI) haben R³ und Z vorzugsweise diejenigen Bedeutungen, die bereits oben im Zusammenhang mit der Beschreibung der erfindungsgemäßen Verbindungen der Formel (I) bzw. (IV) vorzugsweise für R³ und Z angegeben wurden.

Das erfindungsgemäße Verfahren (c) wird vorzugsweise unter Verwendung von Verdünnungsmitteln durchgeführt. Es kommen hierbei die gleichen organischen Lösungsmittel in Betracht, die oben im Zusammenhang mit der Beschreibung des erfindungsgemäßen Verfahrens (a) genannt wurden.

Verfahren (c) wird gegebenenfalls in Gegenwart eines Säureakzeptors durchgeführt. Es kommen hierbei die gleichen Säurebindemittel in Betracht, die oben im Zusammenhang mit der Beschreibung des erfindungsgemäßen Verfahrens (b) genannt wurden.

Die Reaktionstemperaturen können bei dem erfindungsgemäßen Verfahren (c) in einem größeren Bereich variiert werden. Im allgemeinen arbeitet man bei Temperaturen zwischen 0 °C und 100 °C, vorzugsweise bei Temperaturen zwischen 10 °C und 60 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (c) jeweils nach üblichen Methoden.

Zur Überführung in Salze werden die Verbindungen der Formel (I) mit geeigneten Salzbildnern, wie z.B. Natrium- oder Kalium-hydroxid, -methylat oder -ethylat, Ammoniak, Isopropylamin, Dibutylamin oder Triethylamin, in geeigneten Verdünnungsmitteln, wie z.B. Wasser, Methanol oder Ethanol, verrührt. Die Salze können - dann gegebenenfalls nach Einengen - als kristalline Produkte isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich teilweise zur totalen oder semitotalen Unkrautbekämpfung, teilweise zur selektiven Bekämpfung von monokotylen und dikotylen Unkräutern in monokotylen und dikotylen Kulturen, sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.
Als feste Trägerstoffe kommen infrage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitrobenzoesäure (ACIFLUORFEN); Chloressigsäure-N-(methoxymethyl)-2,6-diethylanilid (ALACHLOR); Methyl-6,6-dimethyl-2,4-dioxo-3-[1-(2-propenyloxyamino)-butyliden]-cyclohexancarbonsäure (ALLOXYDIM); 4-Amino-benzolsulfonyl-methylcarbamat (ASULAM); 2-Chlor-4-ethylamino-6-isopropylamino-1,3,5-triazin (ATRAZIN); 2-[[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); 5-Amino-4-chlor-2-phenyl-2,3-dihydro-3-oxy-pyridazin (CHLORIDAZON); Efhyl-2-{[(4-chlor-6-methoxy-2-pyrimidinyl)-aminocarbonyl]-aminosulfonyl}-benzoat (CHLORIMURON); N-(3-Chlorphenyl)-isopropylcarbamat (CHLORPROPHAM); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); N,S-Diethyl-N-cyclohexyl-thiolcarbamat (CYCLOATE); 2-[1-(Ethoximino)-butyl]-3-hydroxy-5-[tetrahydro-(2H)-thiopyran-3-yl]-2-cyclohexen-1-on (CYCLOXYDIM); 2-[4-(2,4-Dichlorphenoxy)-phenoxy]-propionsäure, deren Methyl- oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); [(4-Amino-3,5-dichlor-6-fluor-2-pyridinyl)-oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); N-Phosphonomethylglycin (GLYPHOSATE); 2-{4-[(3-Chlor-5-(trifluormethyl)-2-pyridinyl)-oxy]-phenoxy}-propansäure bzw. deren Ethylester (HALOXYFOP); 3-Cyclohexyl-6-dimethylamino-1-methyl-1,3,5-triazin-2,4-dion (HEXAZINONE); Methyl-2-[4,5-dihydro-4-methyl-4-(1-methylethyl)-5-oxo-1H-imidazol-2-yl]-4(5)-methylbenzoat (IMAZAMETHABENZ); 2-(4,5-Dihydro-4-methyl-4-isopropyl-5-oxo-1H-imidazol-2-yl)-pyridin-3-carbonsäure (IMAZAPYR); 2-(5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 3,5-Diiod-4-hydroxybenzonitril (IOXYNIL); N,N-Dimethyl-N'-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylen-thiolcarbamat (MOLINATE); 1-(3-Trifluormethyl-phenyl)-4-methylamino-5-chlor-6-pyridazon (NORFLURAZON); 4-(Di-n-propylamino)-3,5-dinitrobenzolsulfonamid (ORYZALIN); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); 3-(Ethoxycarbonylaminophenyl)-N-(3'-methylphenyl)-carbamat (PHENMEDIPHAM); 4-Amino-3,5,6-trichlorpyridin-2-carbonsäure (PICLORAM); α-Chlor-2,6'-diethyl-N-(2-propoxyethyl)-acetanilid (PRETILACHLOR); 2-Chlor-N-isopropylacetanilid (PROPACHLOR); Isopropyl-N-phenyl-carbamat (PROPHAM); O-(6-Chlor-3-phenyl-pyridazin-4-yl)-S-octyl-thiocarbonat (PYRIDATE); 2-[4-(6-Chlor-chinoxalin-2-yl-oxy)-phenoxy]-propionsäure-ethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); Methyl-2-{[(4,6-dimethyl-2-pyrimidinyl)-aminocarbonyl]-aminosulfonyl}-benzoat (SULFOMETURON); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); S-[(4-Chlorphenyl)-methyl]-N,N-diethylthiocarbamat (THIOBENCARB); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe kennen sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken, Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

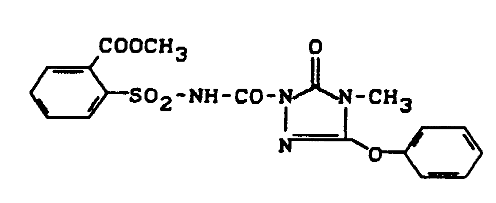

### (Verfahren (a))

Eine Mischung aus 2,0 g (10,5 mMol) 4-Methyl-5-phenoxy-2,4-dihydro-3H-1,2,4-triazol-3-on, 3,5 g (14,5 mMol) 2-Methoxycarbonyl-phenylsulfonylisocyanat und 60 ml Acetonitril wird 6 Stunden bei 20°C gerührt und anschließend im Wasserstrahlvakuum eingeengt. Der Rückstand wird mit Diethylether verrührt und das kristallin angefallene Produkt durch Absaugen isoliert.

Man erhält 4,1 g (90% der Theorie) 2-(2-Methoxycarbonylphenylsulfonylaminocarbonyl)-4-methyl-5-phenoxy-2,4-dihydro-3H-1,2,4-triazol-3-on vom Schmelzpunkt 161°C.

Analog Beispiel 1 und entsprechend der allgemeinen Beschreibung der erfindungsgemäßen Herstellungsverfahren können beispielsweise auch die in der nachstehenden Tabelle 3 aufgeführten Verbindungen der Formel (I) hergestellt werden.

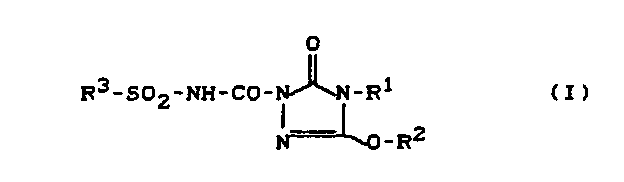

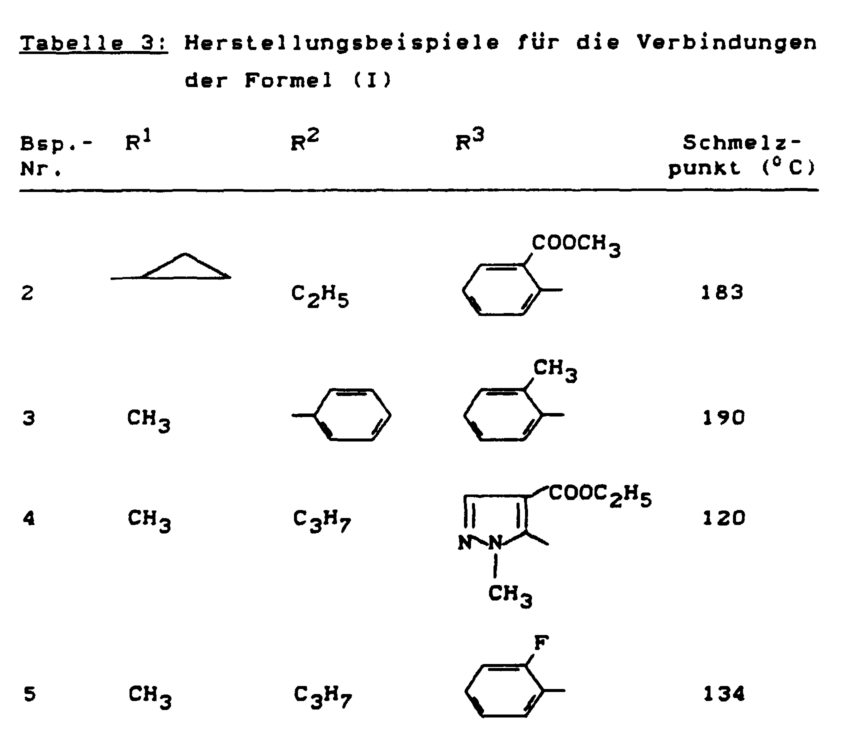

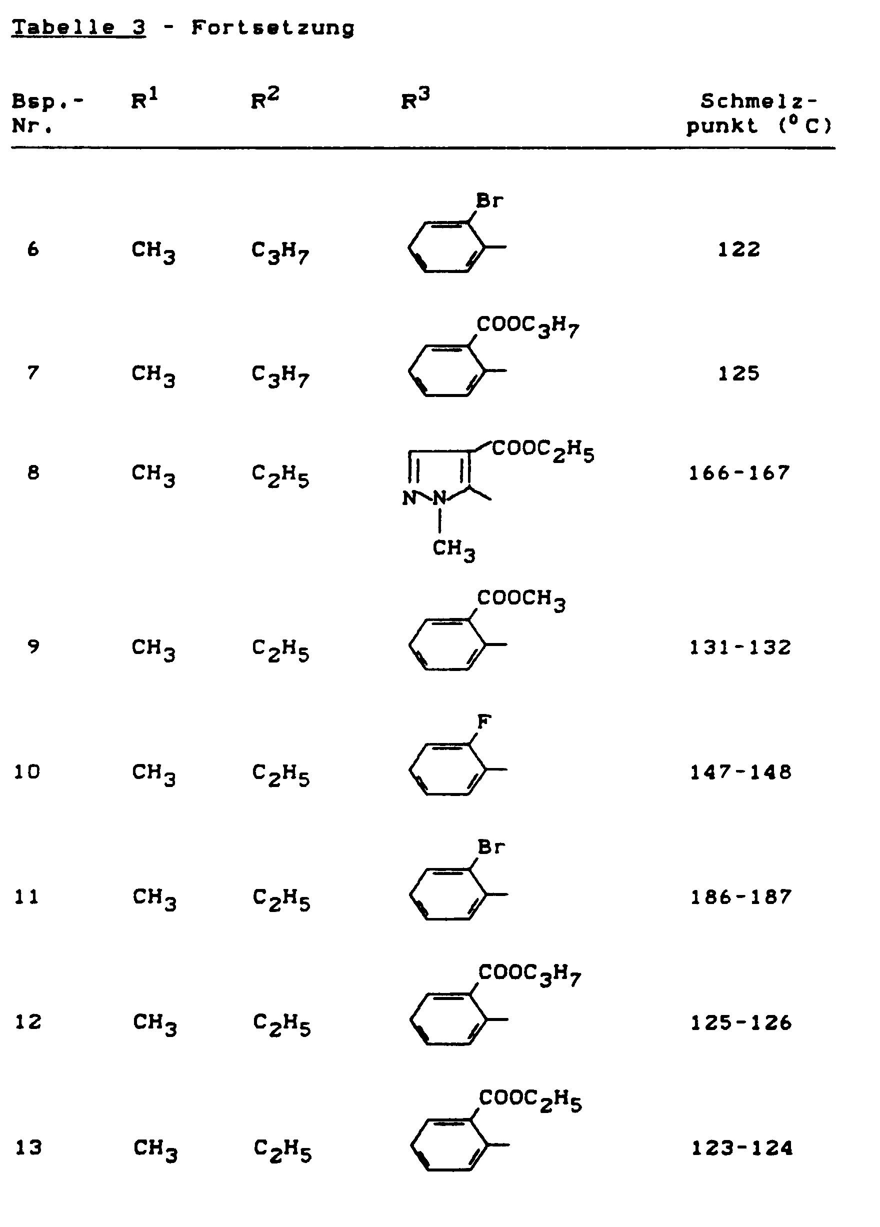

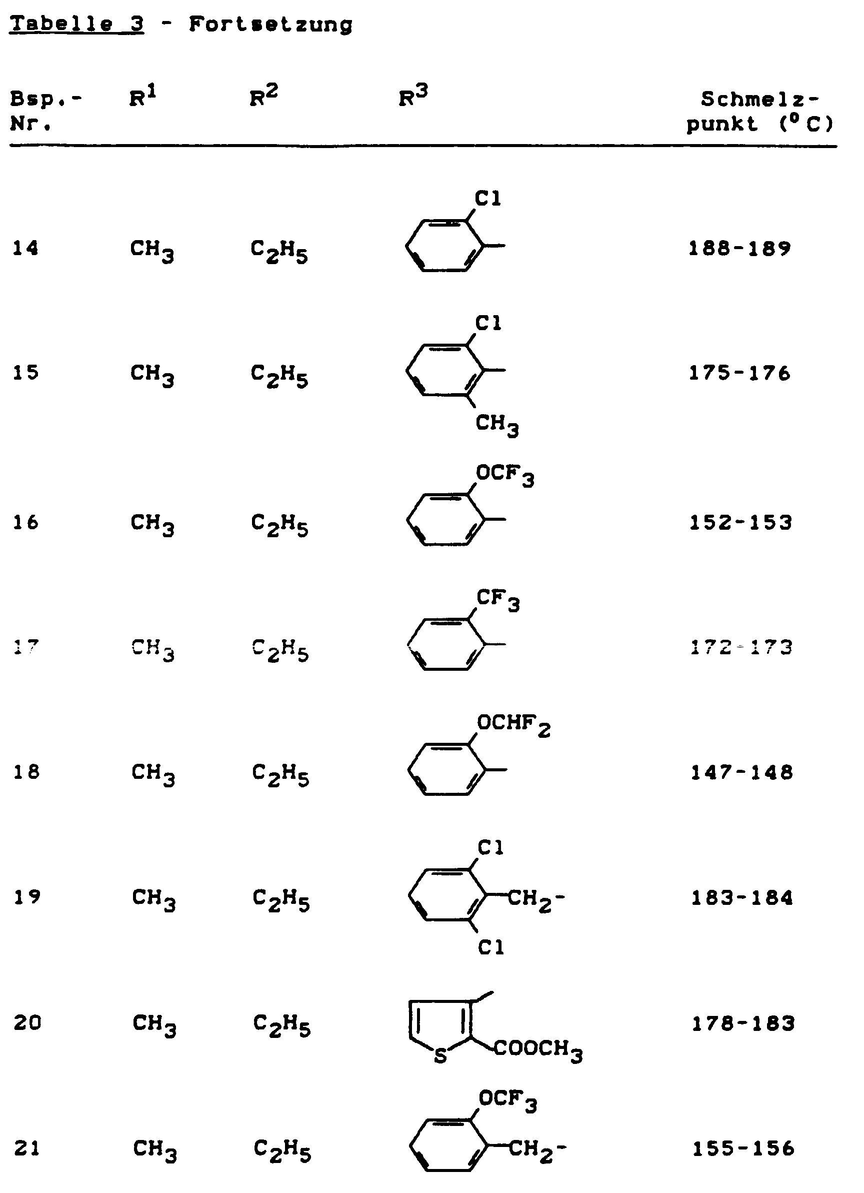

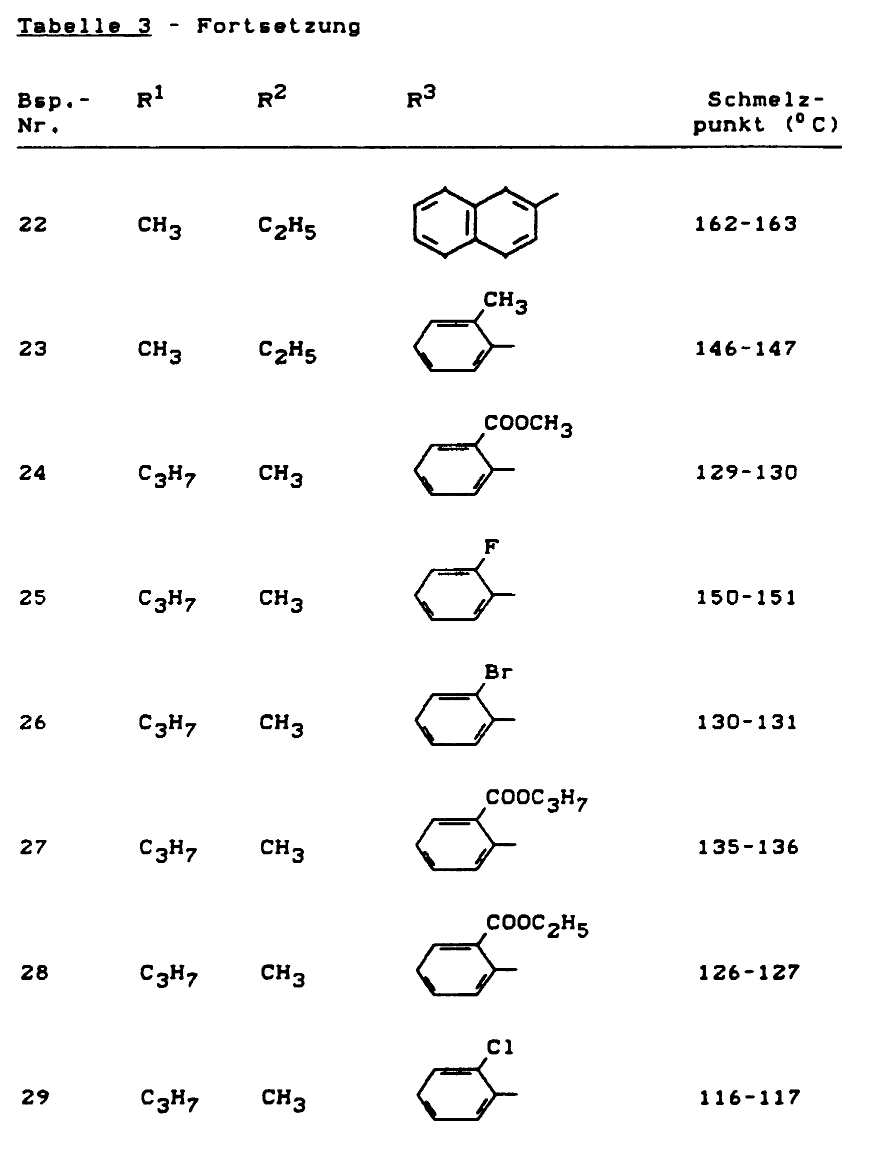

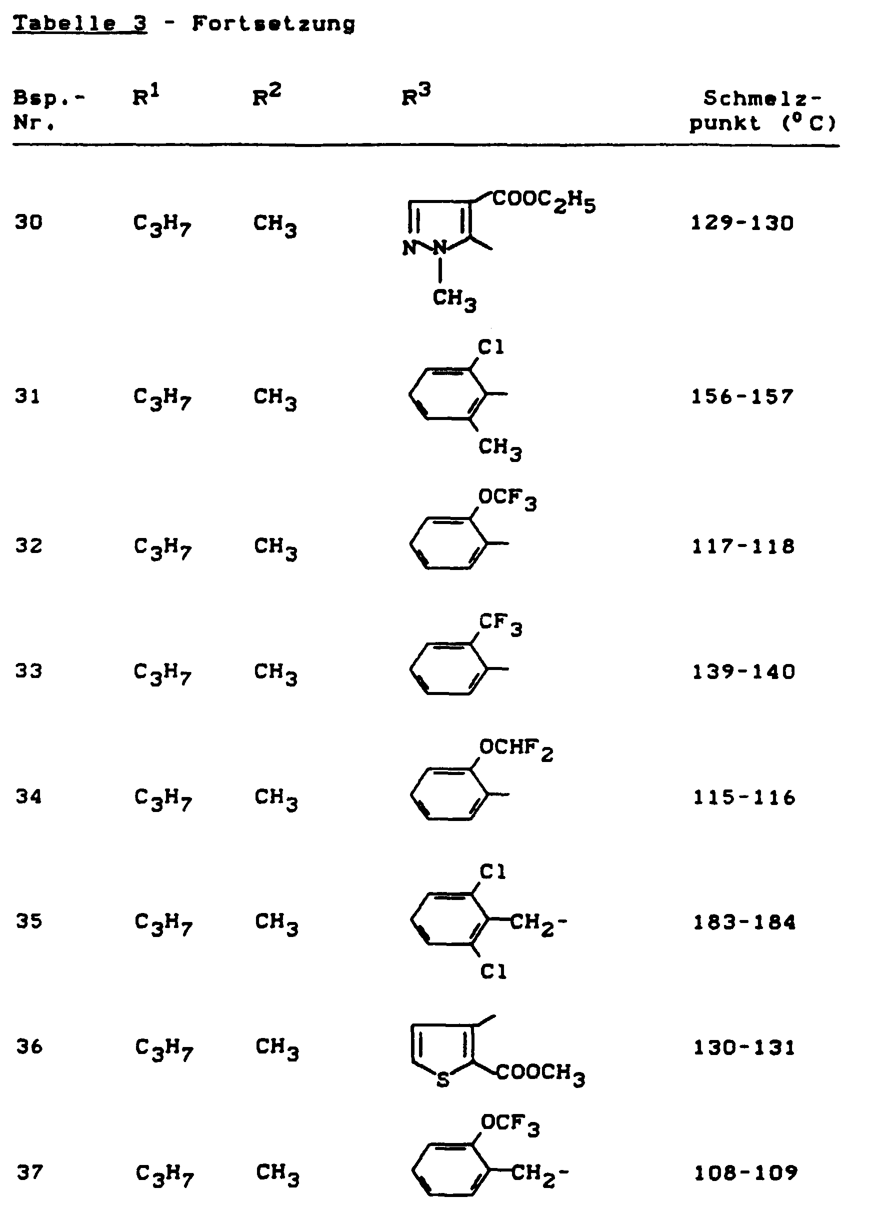

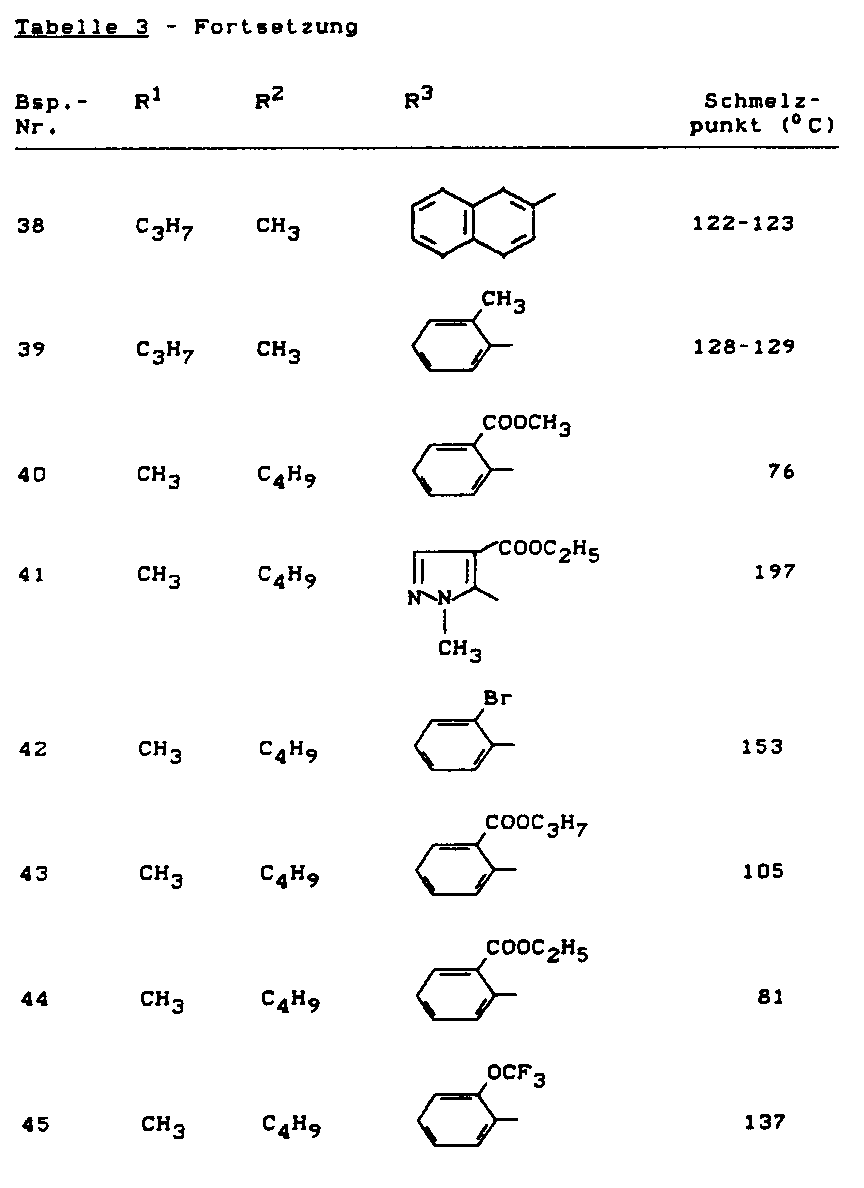

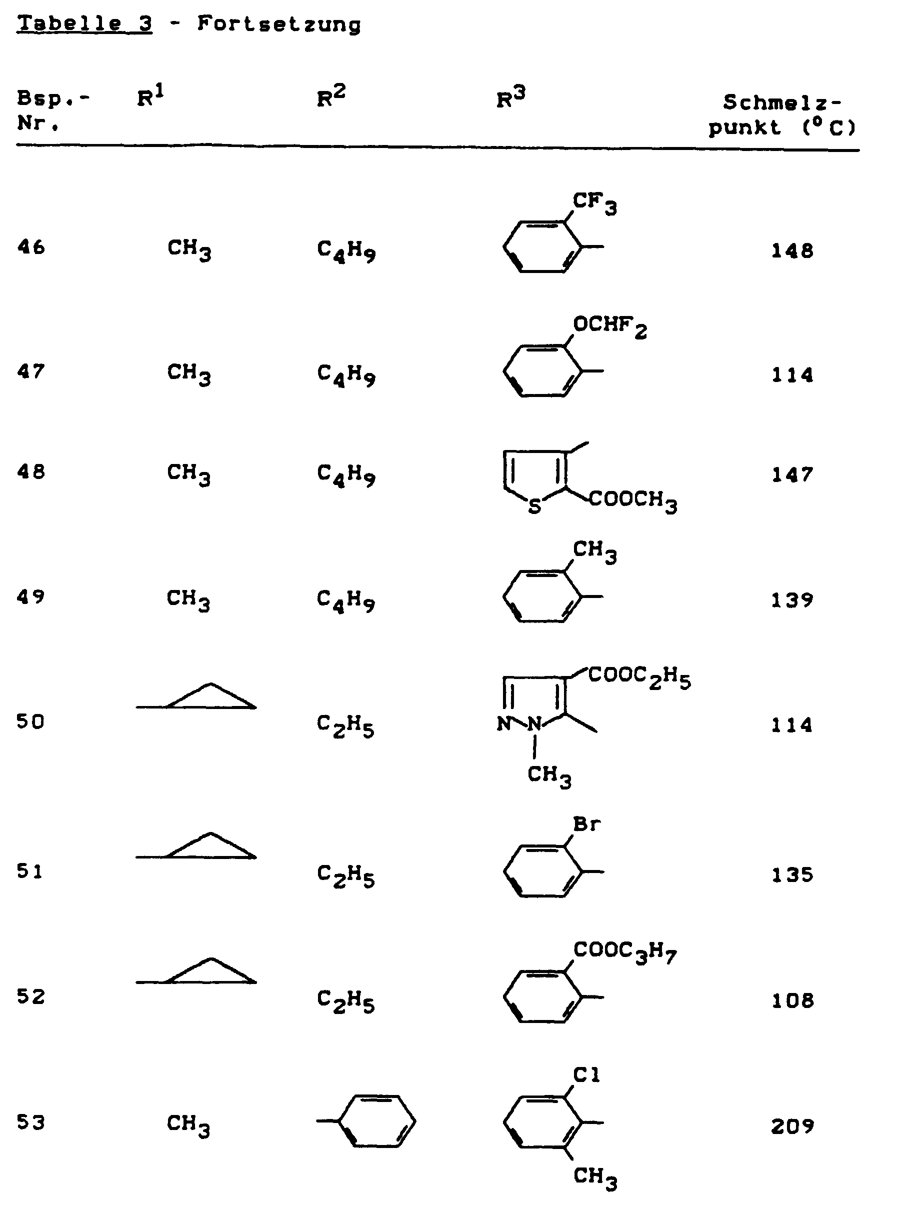

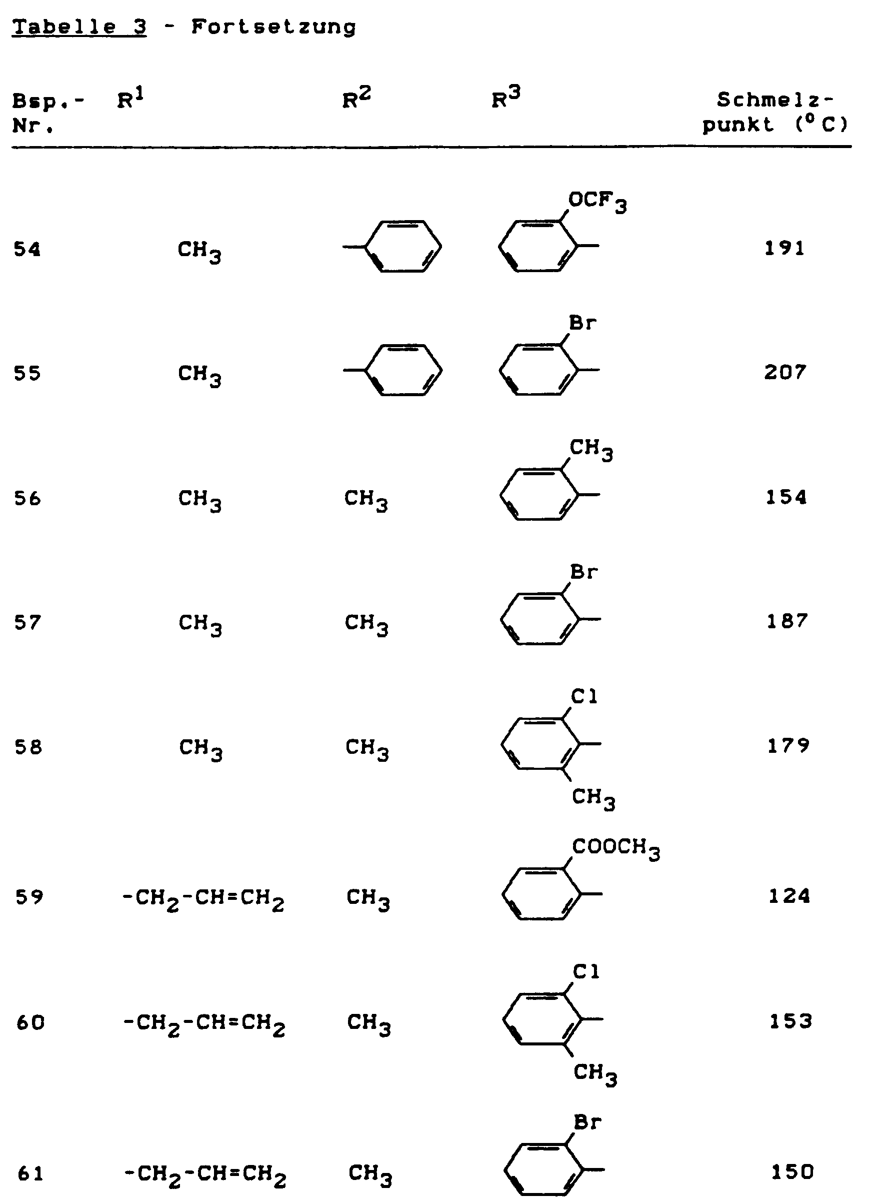

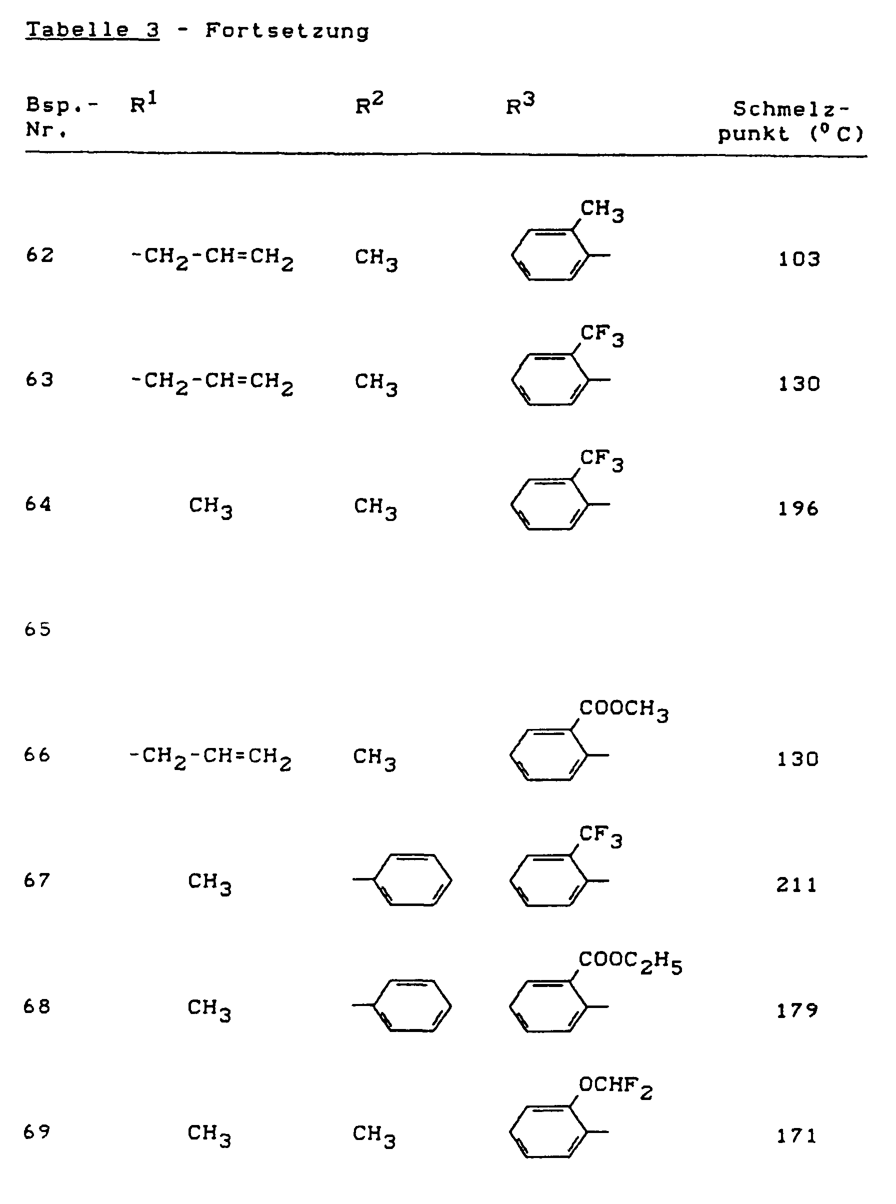

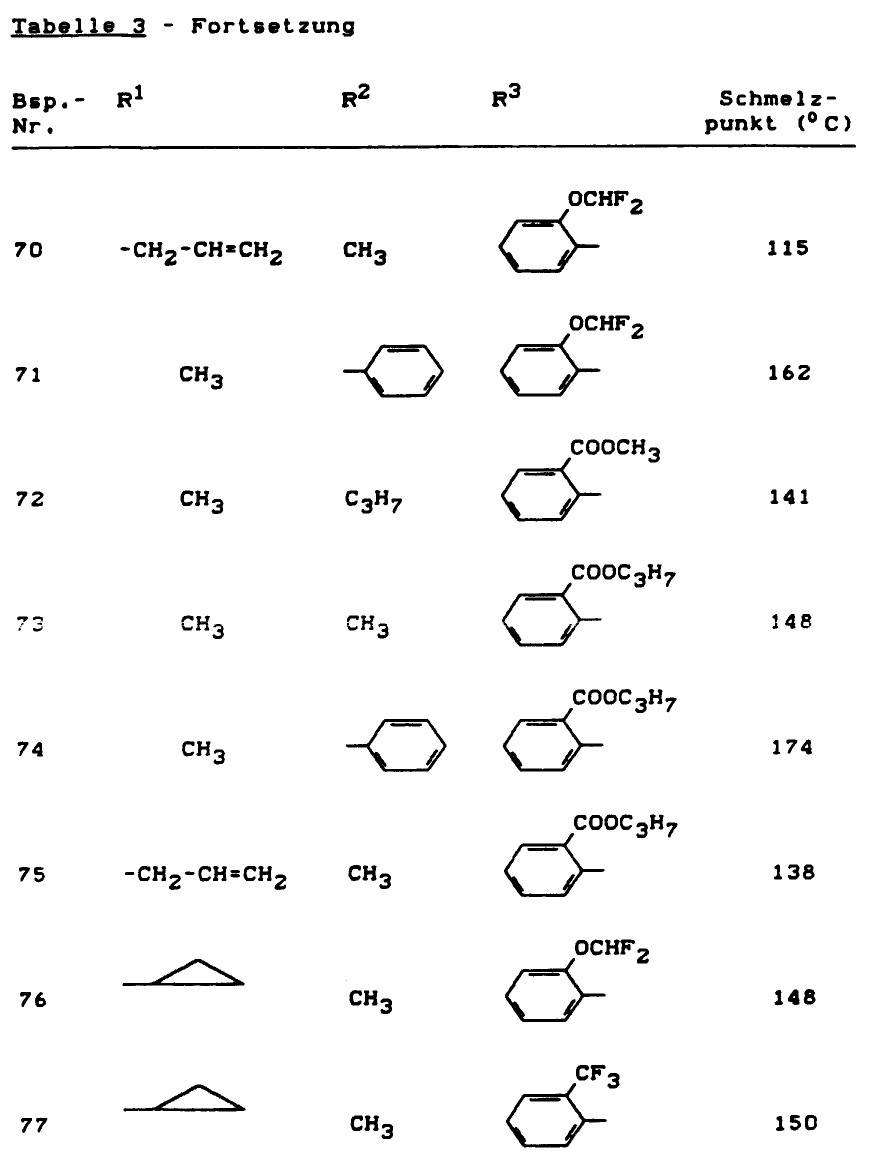

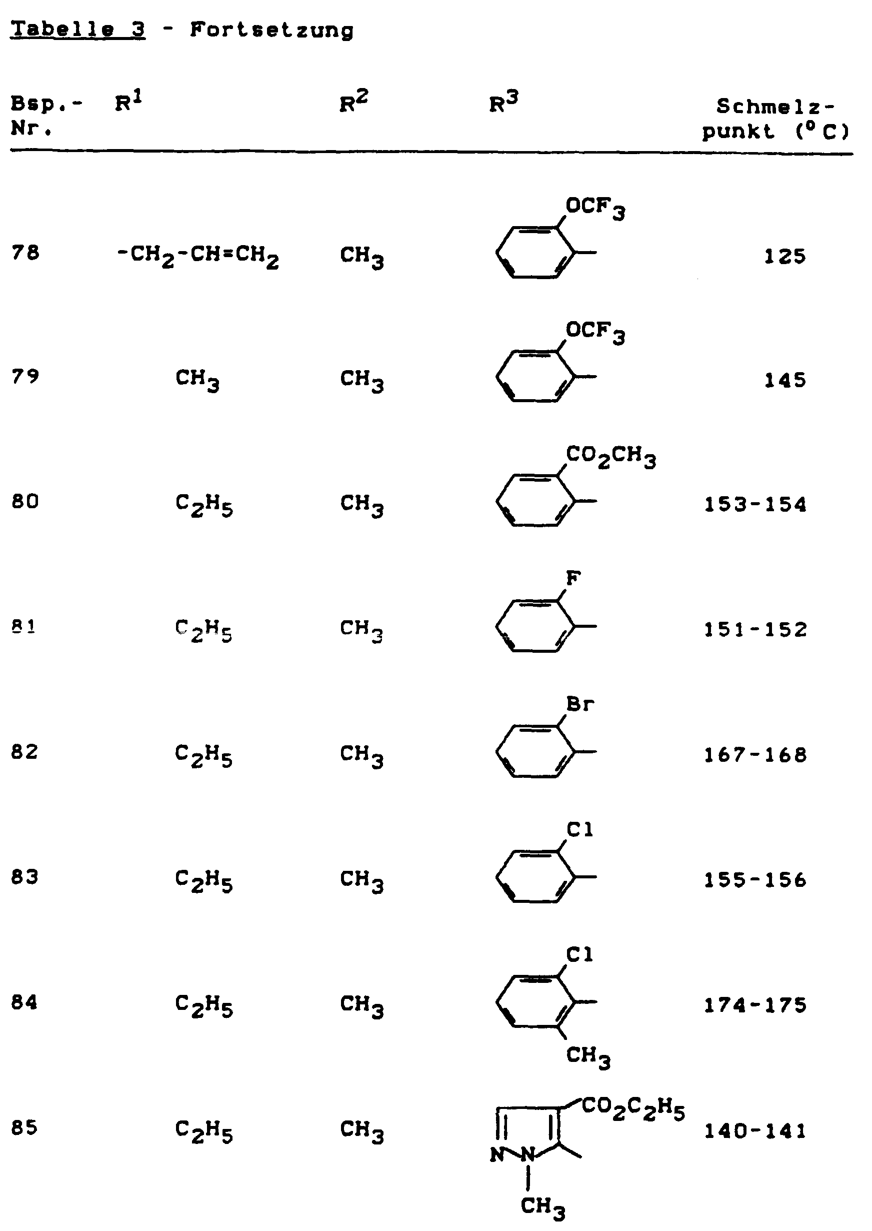

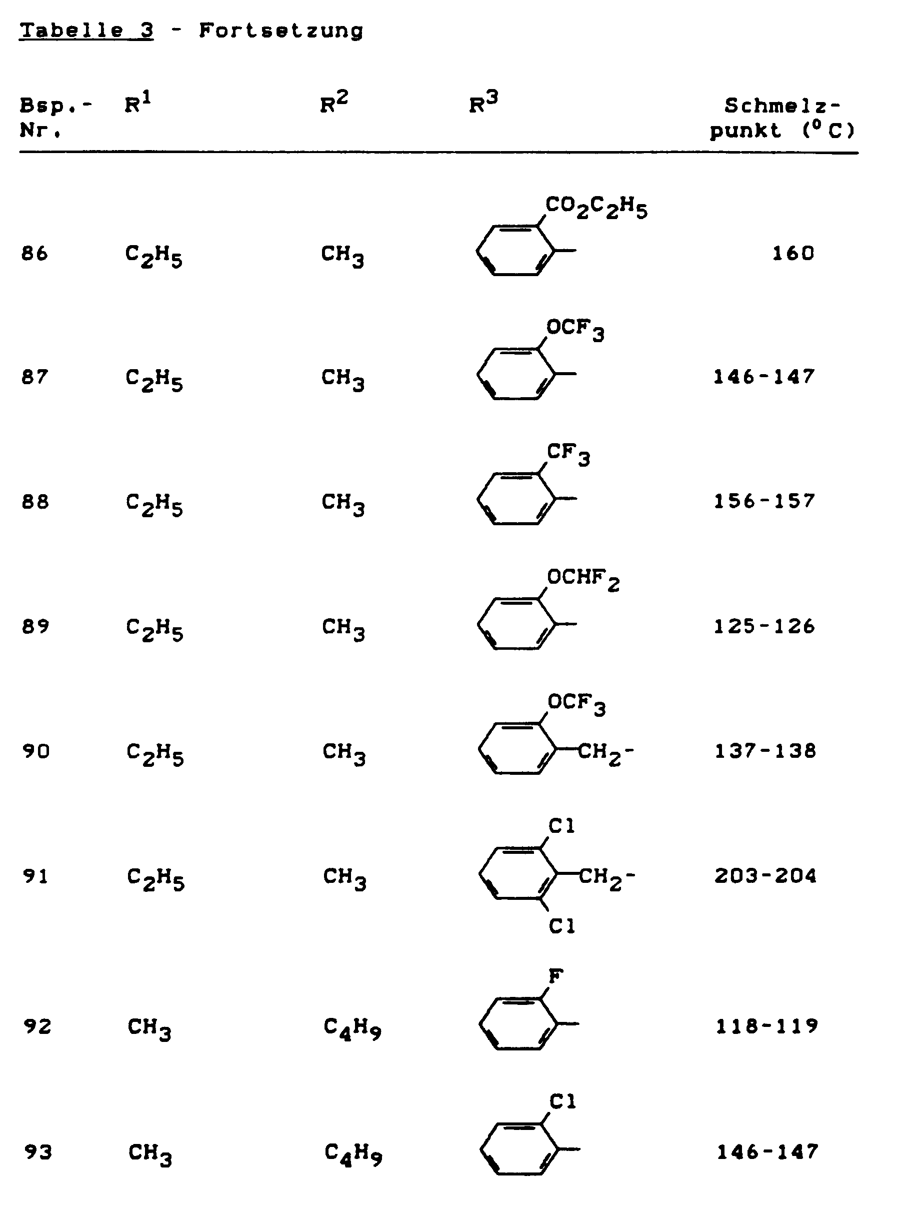

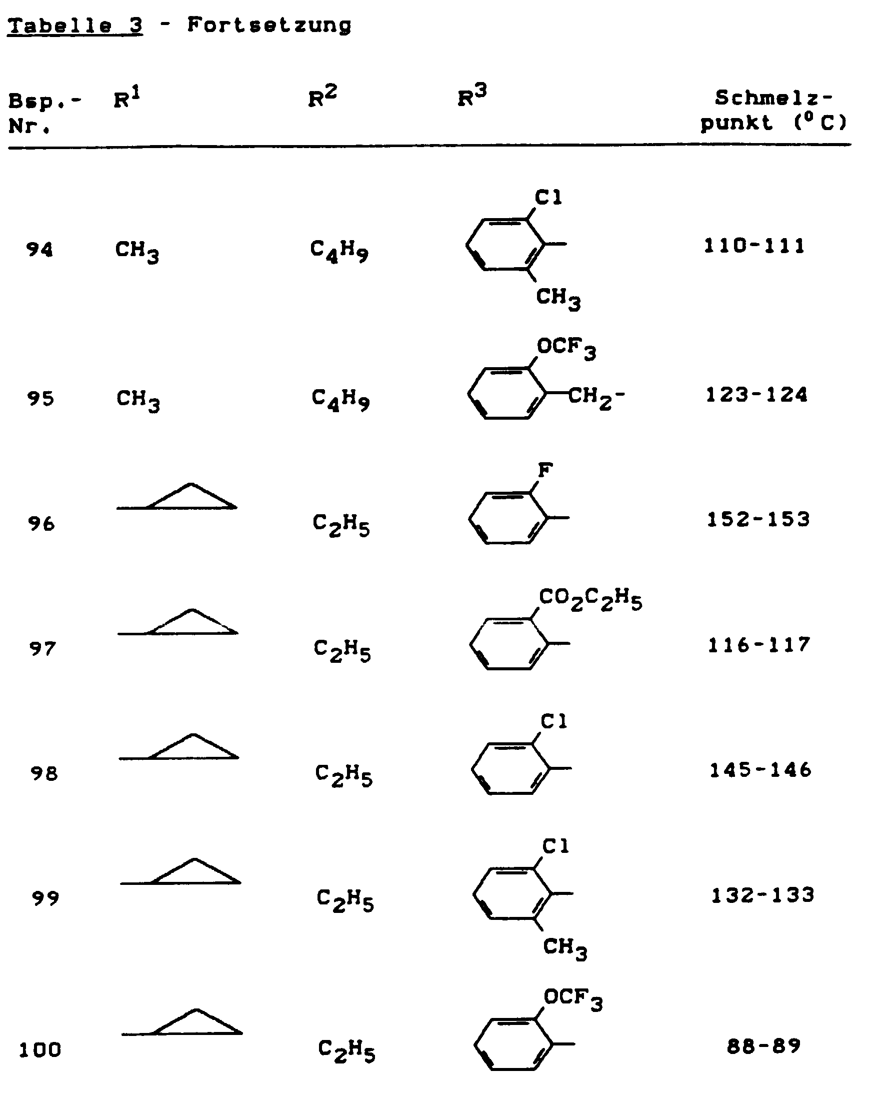

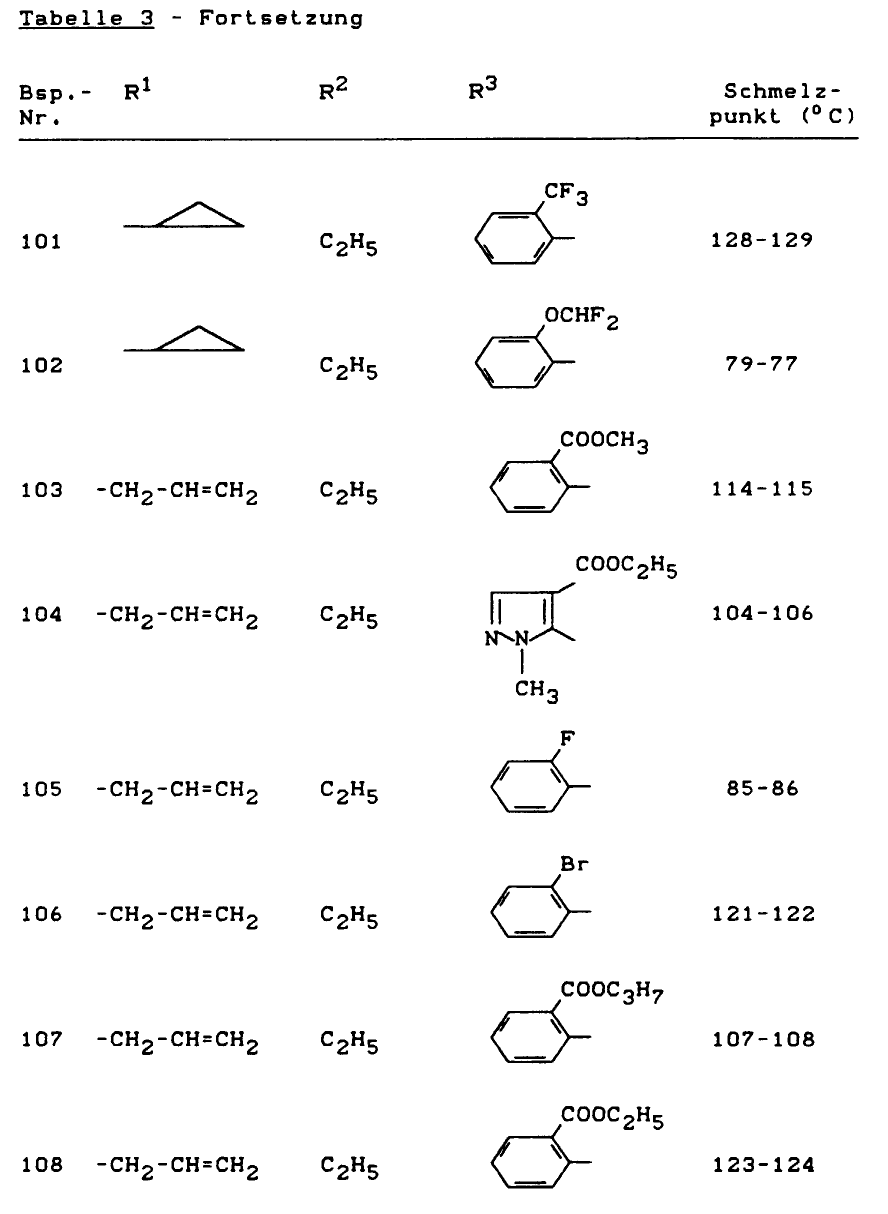

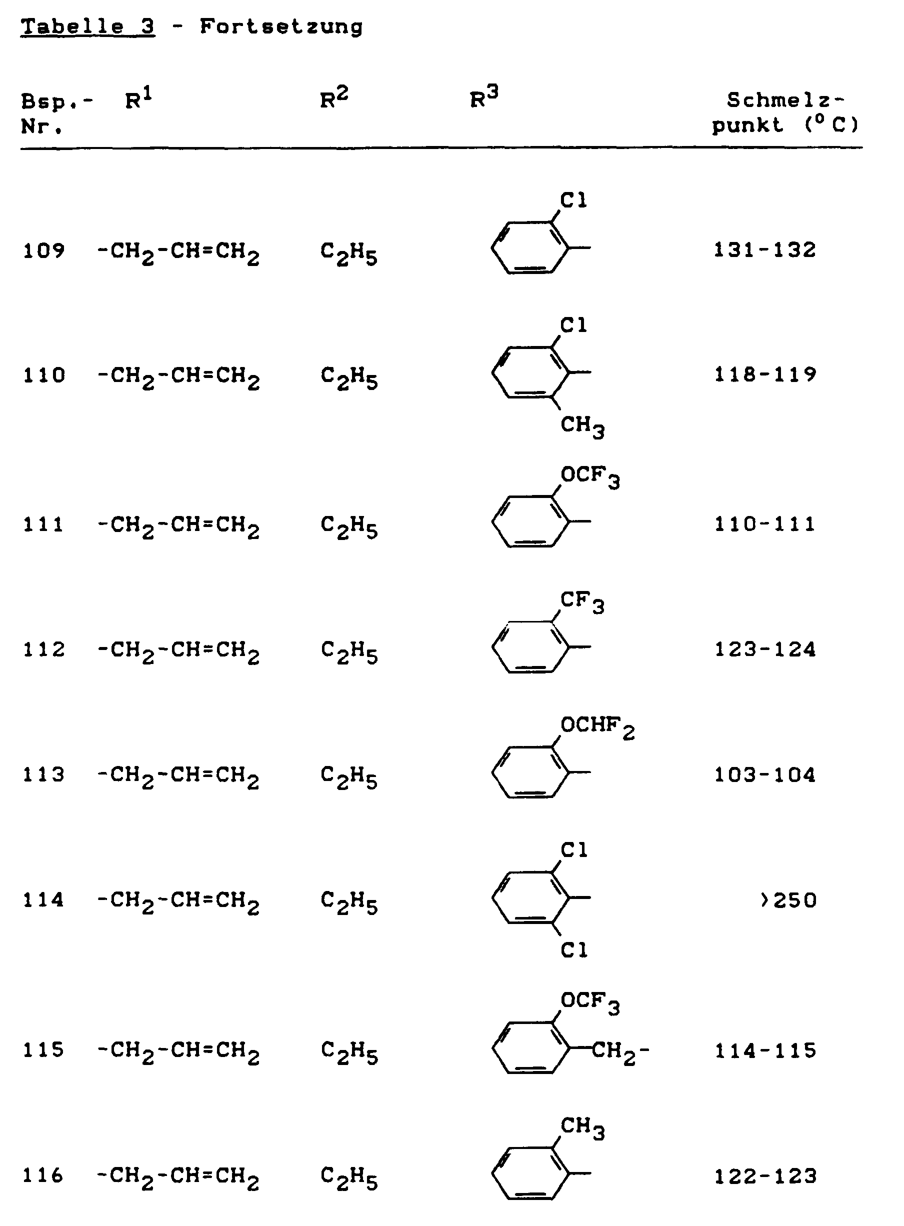

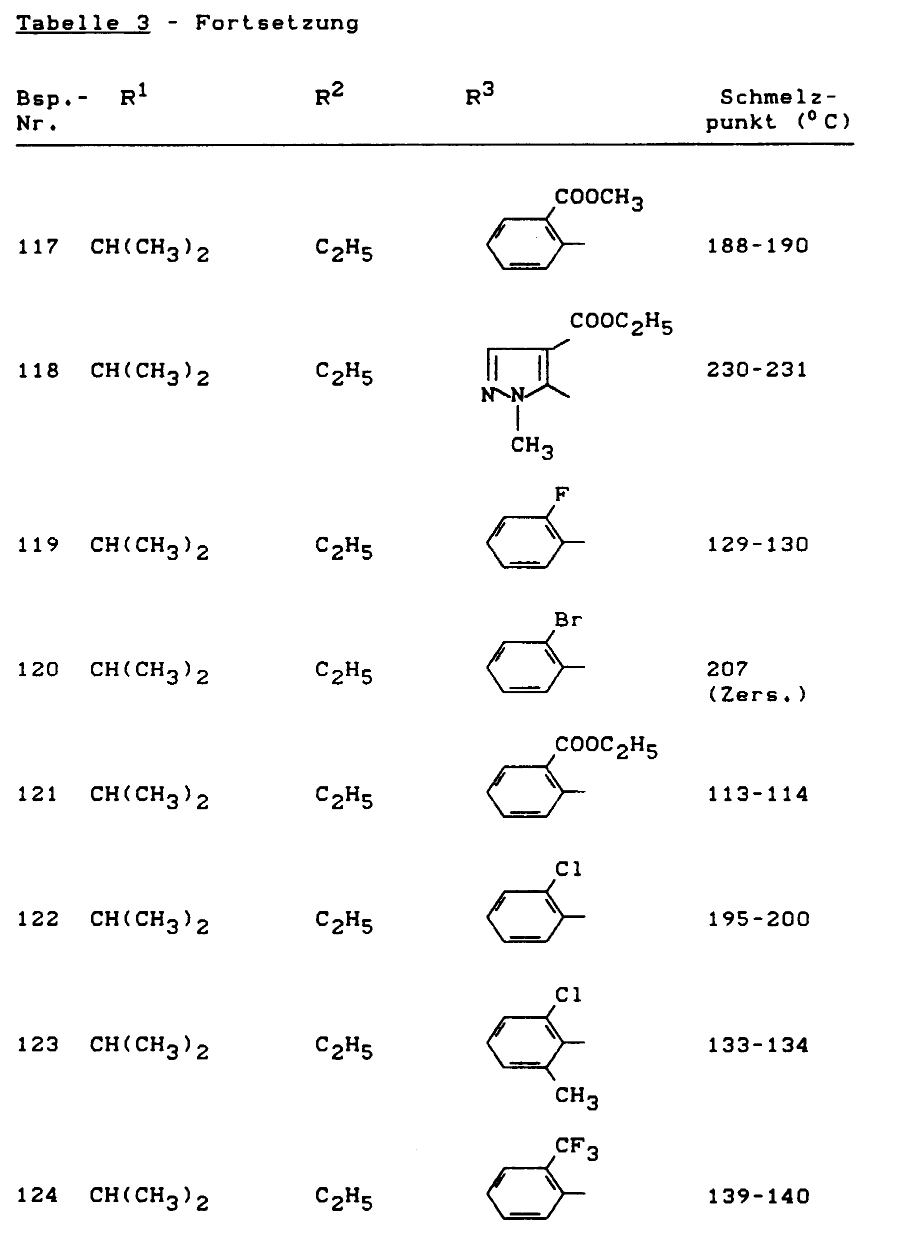

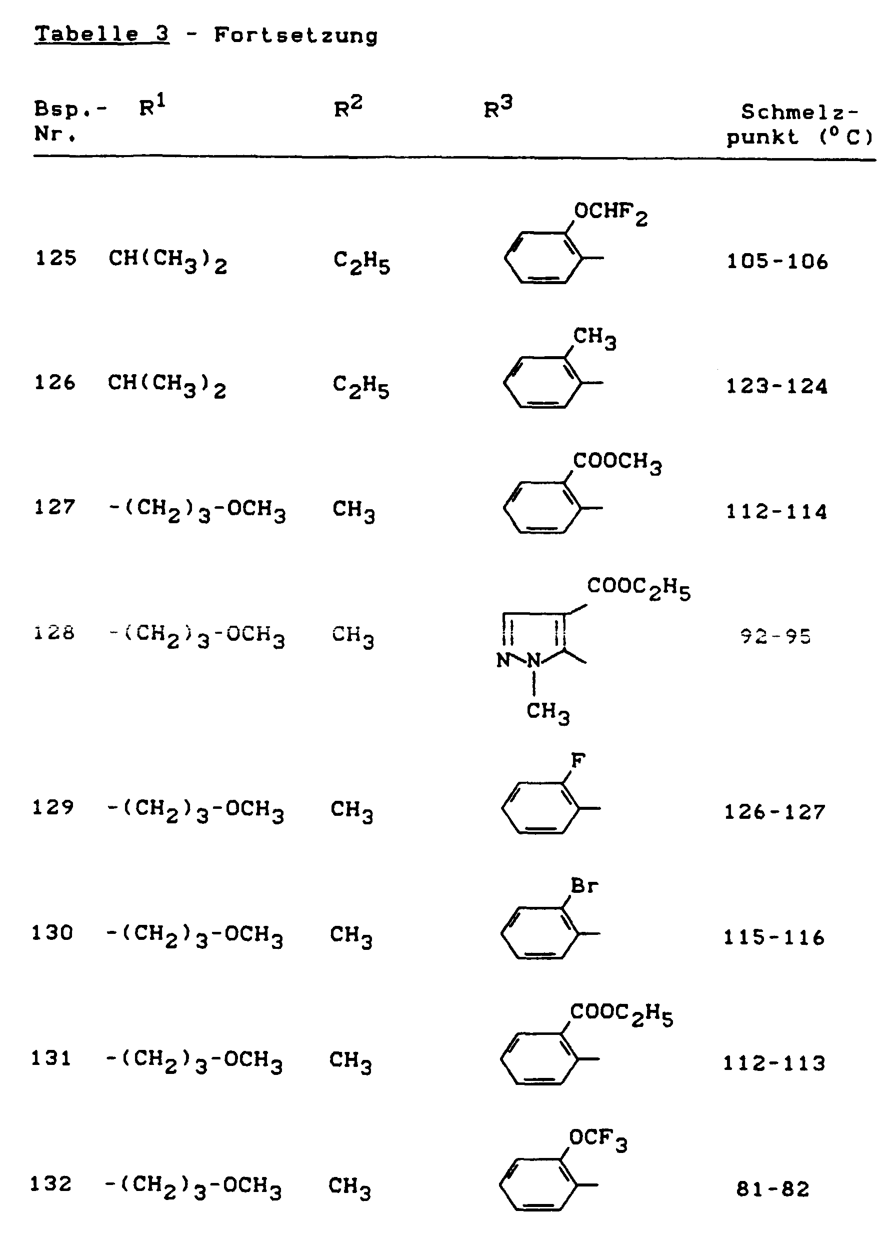

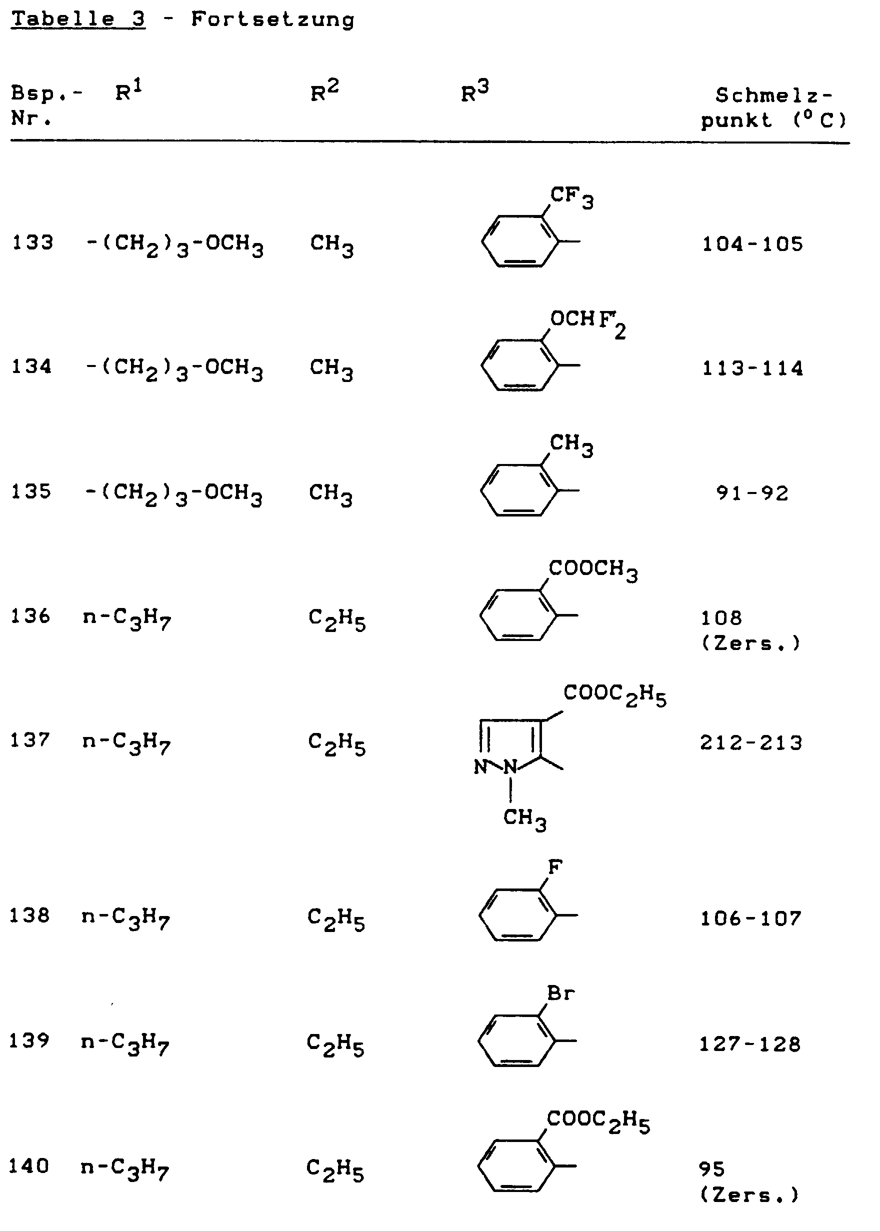

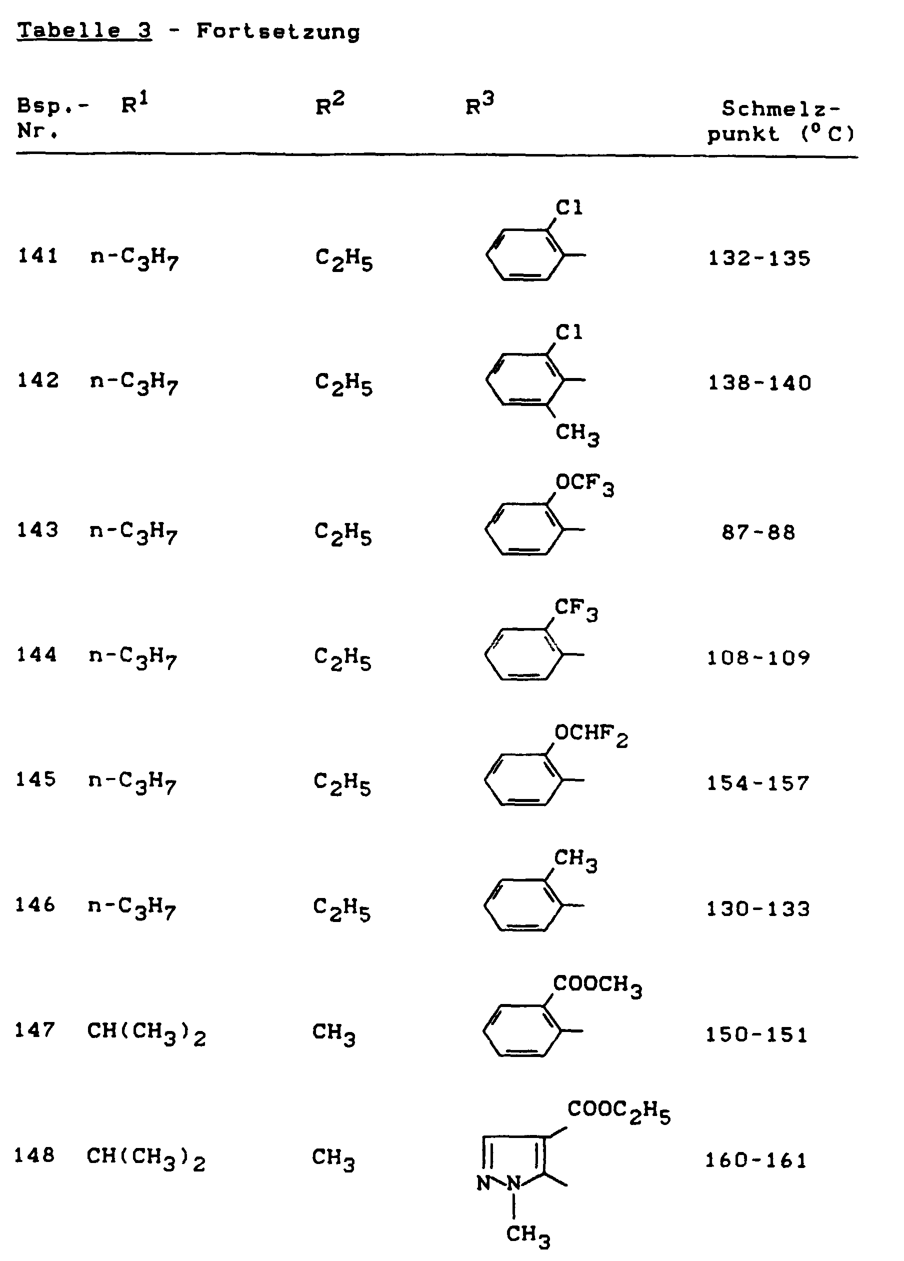

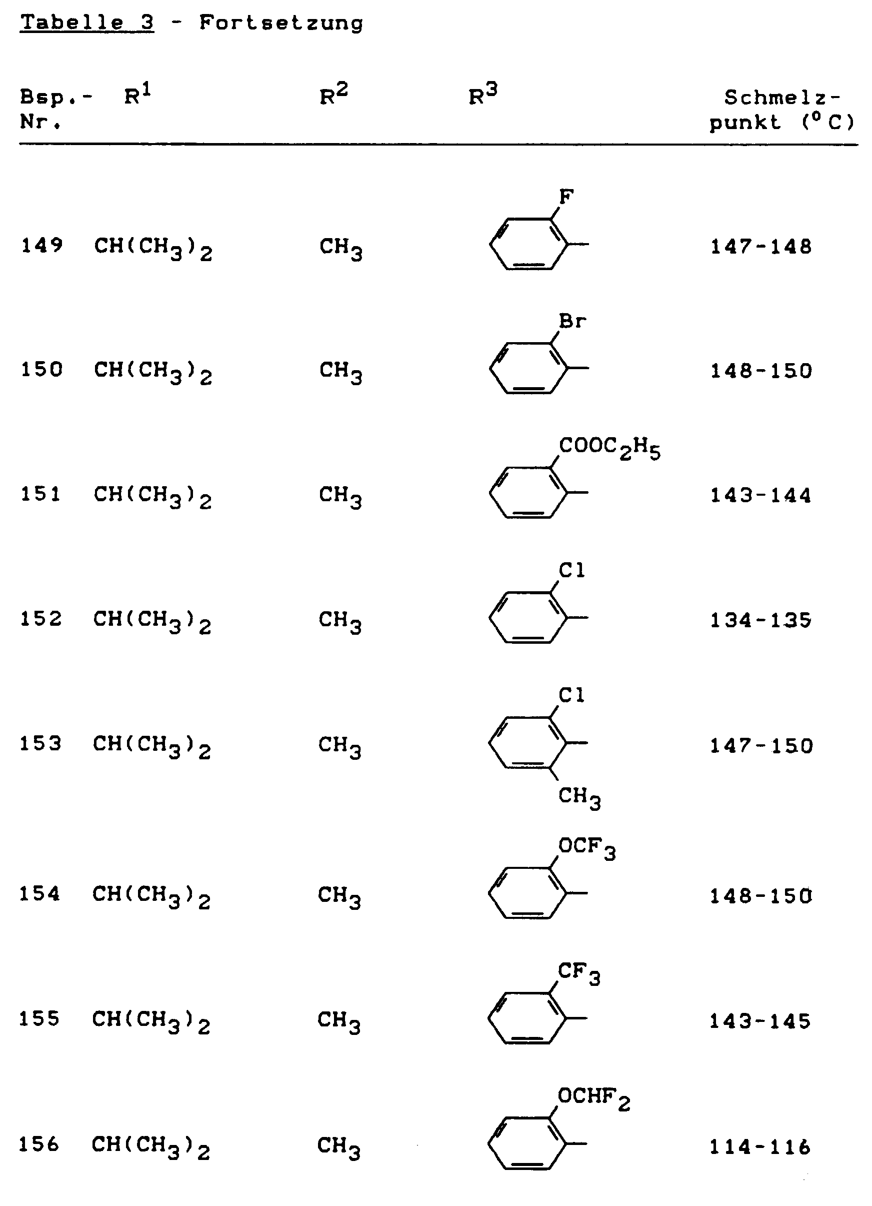

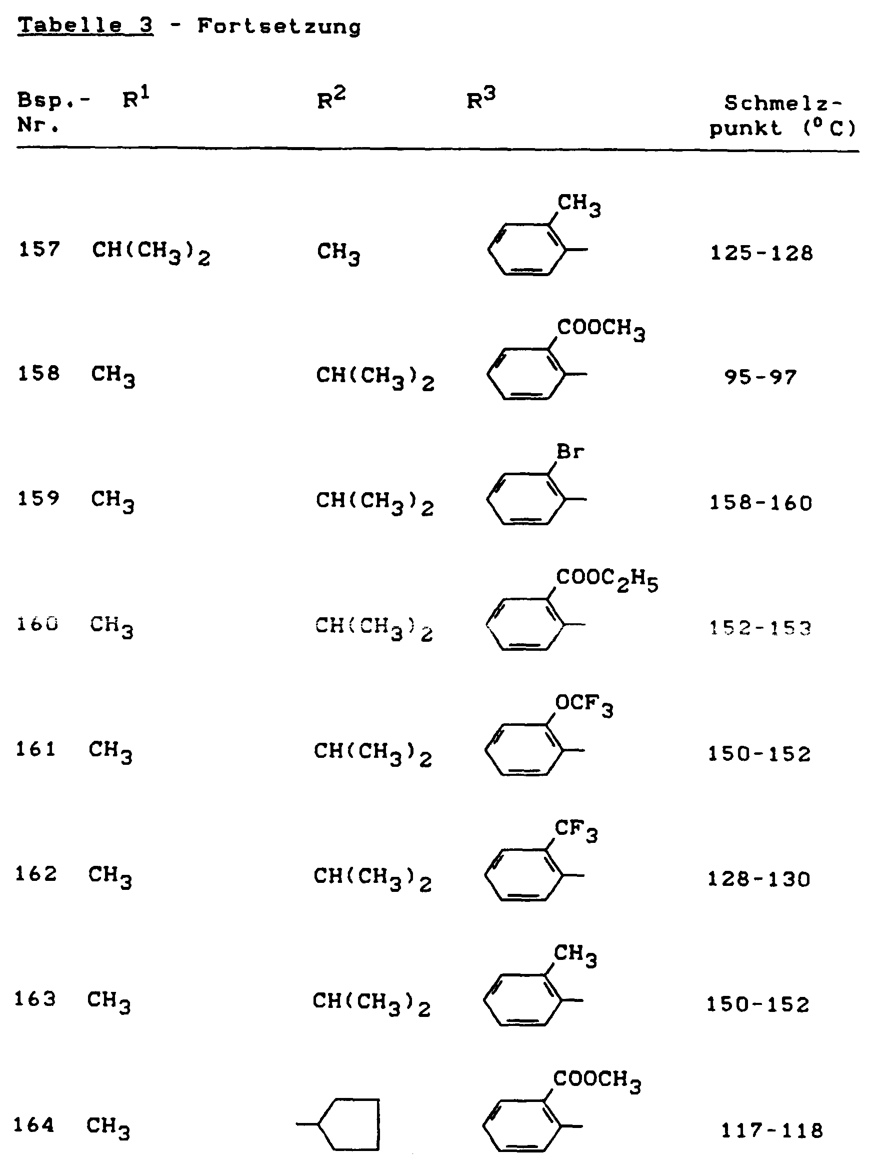

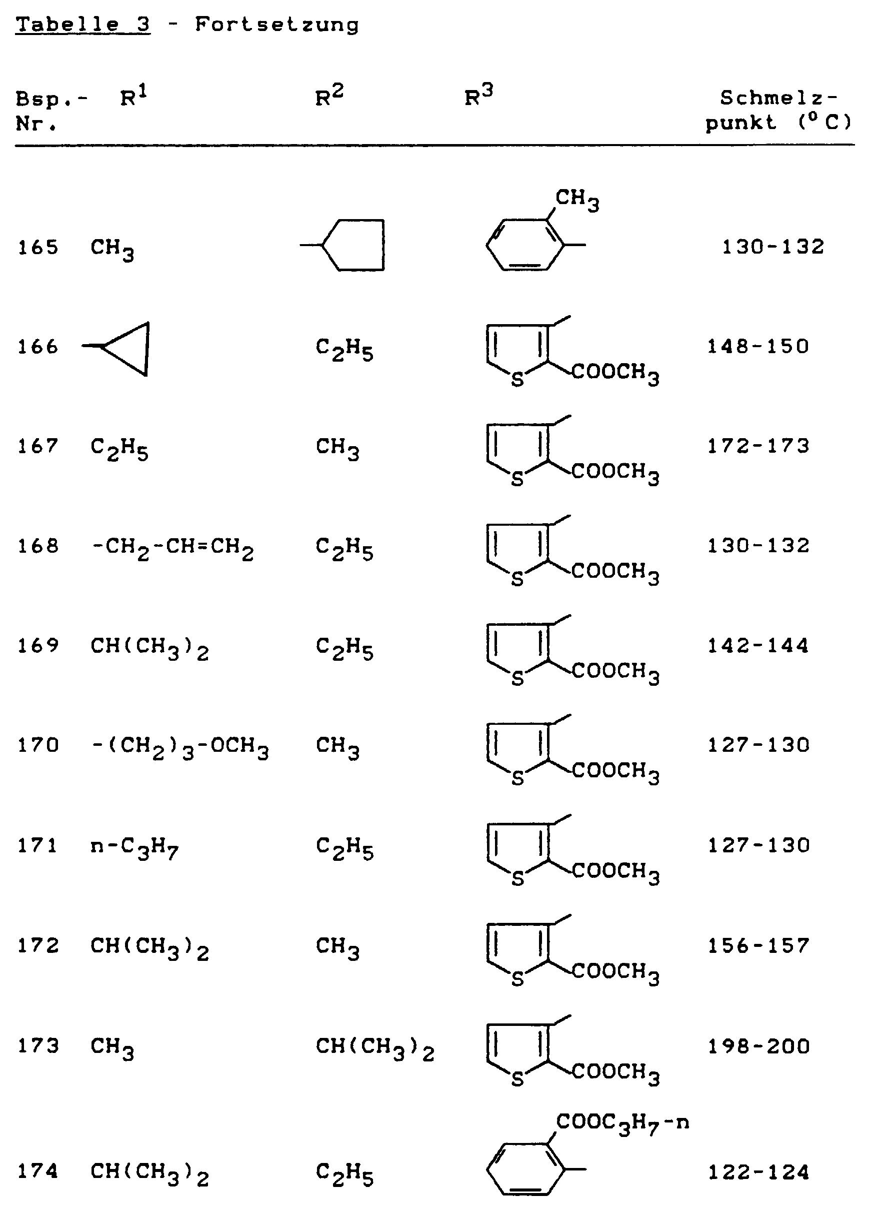

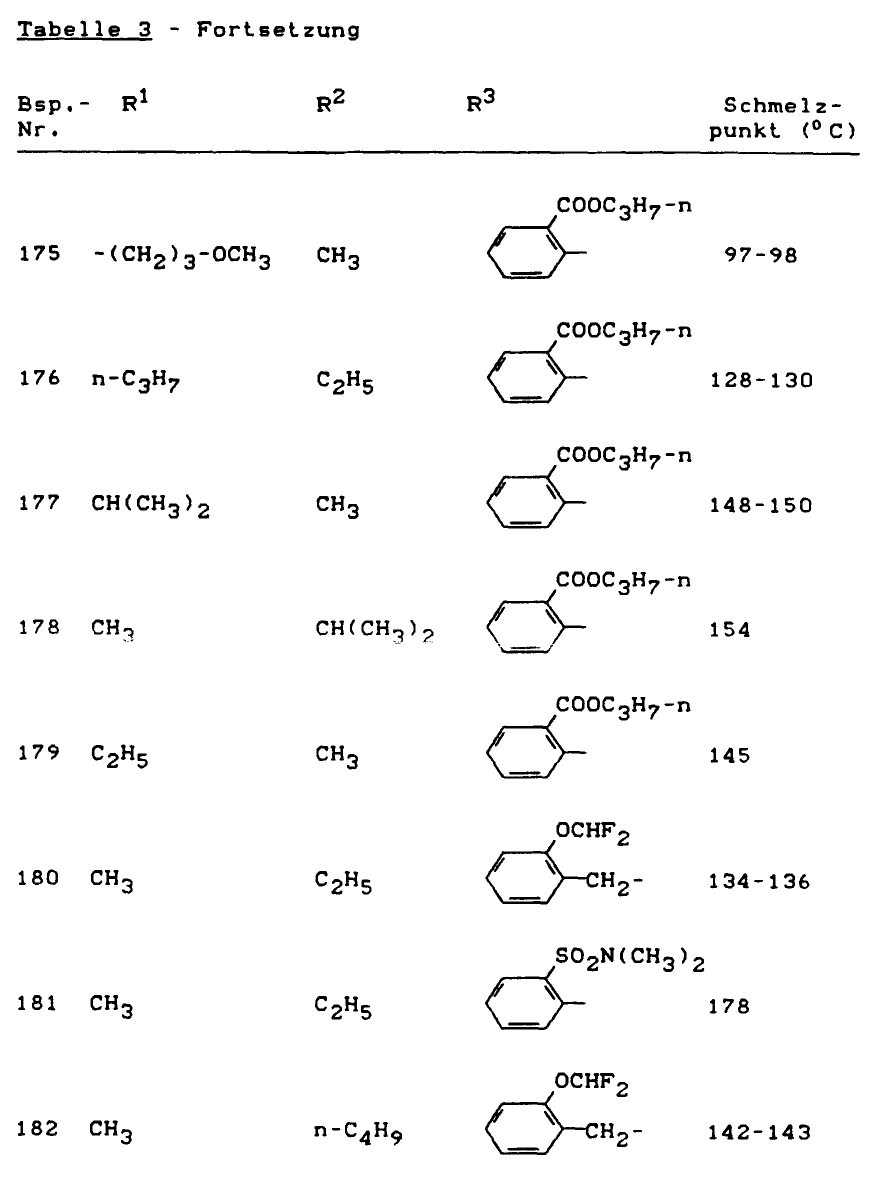

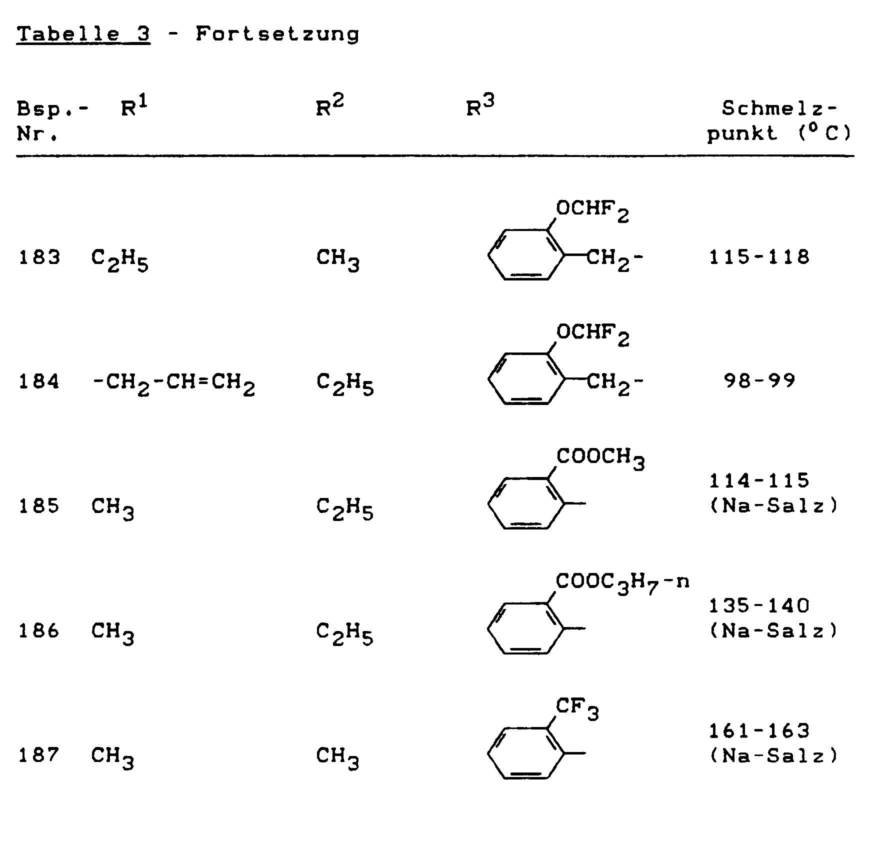

### Ausgangsstoffe der Formel (II):

### Beispiel (II-1)

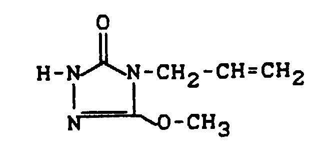

Eine Mischung aus 5,0 g (0,02 Mol) 1-Phenoxycarbonyl-4-allyl-O-methyl-isosemicarbazid und 30 ml Toluol wird 15 Minuten unter Rückfluß erhitzt und anschließend im Wasserstrahlvakuum eingeengt. Der Rückstand wird mit Diethylether/Petrolether verrieben und das kristallin angefallene Produkt durch Absaugen isoliert.

Man erhält 2,0 g (64% der Theorie) 4-Allyl-5-methoxy-2,4-dihydro-3H-1,2,4-triazol-3-on vom Schmelzpunkt 111°C.

### Beispiel (II-2)

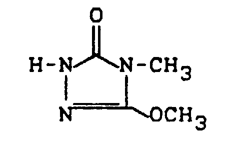

50,2 g (0,33 Mol) Hydrazinoameisensäurephenylester und 36,6 g (0,33 Mol, 93%ig) Trimethyliminocarbonat werden in 100 ml absolutem o-Dichlorbenzol auf 60°C erwärmt und 2 Stunden gerührt, wobei eine klare Lösung entsteht. Im Verlauf von zwei Stunden wird auf 120°C erwärmt, wobei Methanol abdestilliert. Es wird vorsichtig Vakuum angelegt, wobei weiteres Methanol und zuletzt Phenol abdestilliert. Bei weiterem Destillieren entsteht eine Fraktion, die in der Vorlage kristallin erstarrt.

Nach Umkristallisieren aus Toluol werden 7,0 g (0,054 Mol, 16% der Theorie) 4-Methyl-5-methoxy-2,4-dihydro-3H-1,2,4-triazol-3-on in Form farbloser Kristalle vom Schmelzpunkt 142-144°C erhalten.

Analog zu den Beispielen (II-1) und (II-2) können beispielsweise auch die in der nachstehenden Tabelle 4 aufgeführten Verbindungen der Formel (II) hergestellt werden.

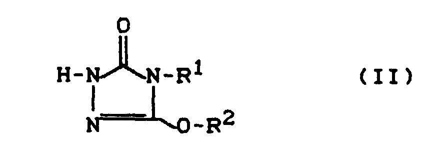

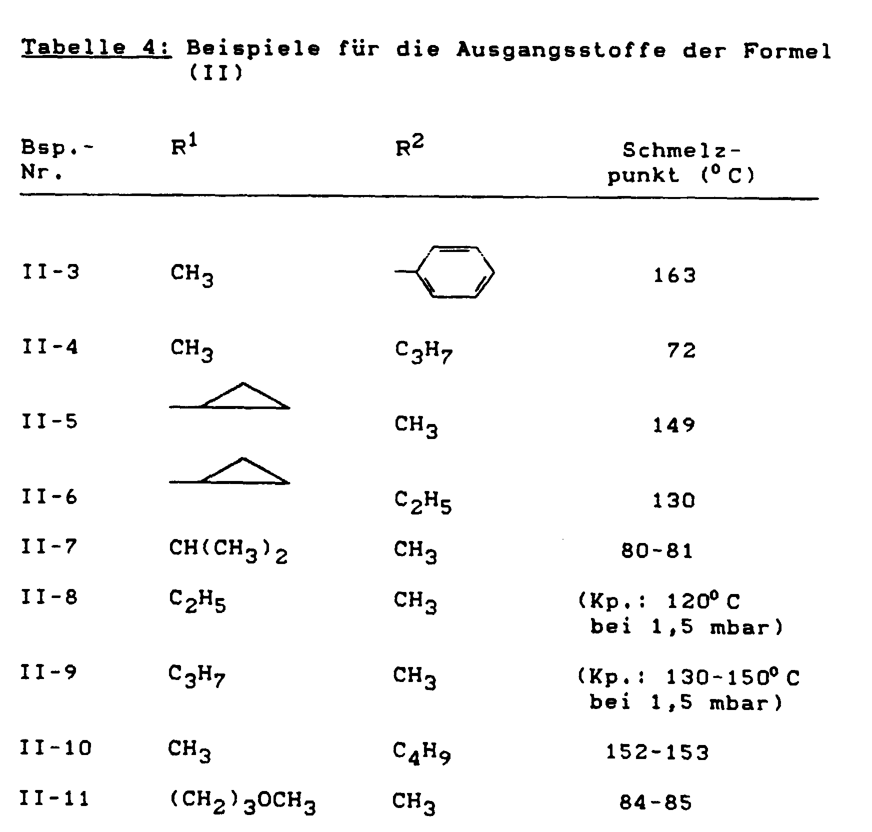

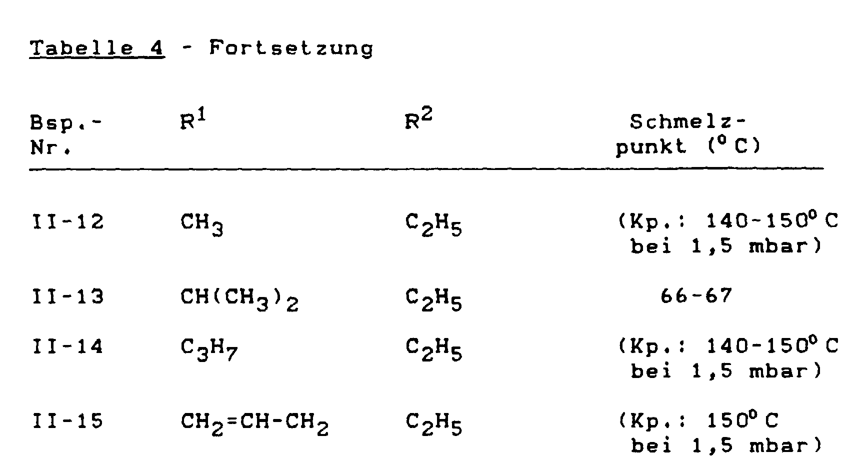

### Zwischenprodukte der Formel (IX):

### Beispiel (IX-1)

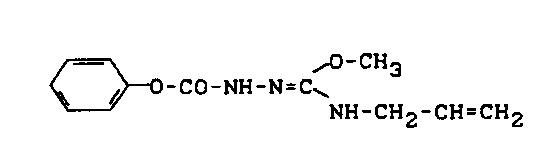

Eine Mischung aus 30,4 g (0,2 Mol) Hydrazinoameisensäurephenylester, 26,0 g (0,2 Mol) N-Allyl-iminokohlensäuredimethylester und 150 ml Methanol wird 12 Stunden bei 20°C gerührt und anschließend im Wasserstrahlvakuum eingeengt. Der Rückstand wird mit Diethylether/Ethanol (Vol. 1/1) verrieben und das kristalline Produkt durch Absaugen isoliert.

Man erhält 11,0 g (22% der Theorie) 1-Phenoxycarbonyl-4-allyl-O-methyl-isosemicarbazid vom Schmelzpunkt 114°C.

Analog Beispiel (IX-1) können beispielsweise auch die in der nachstehenden Tabelle 5 aufgeführten Verbindungen der Formel (IX) hergestellt werden.

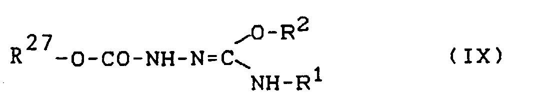

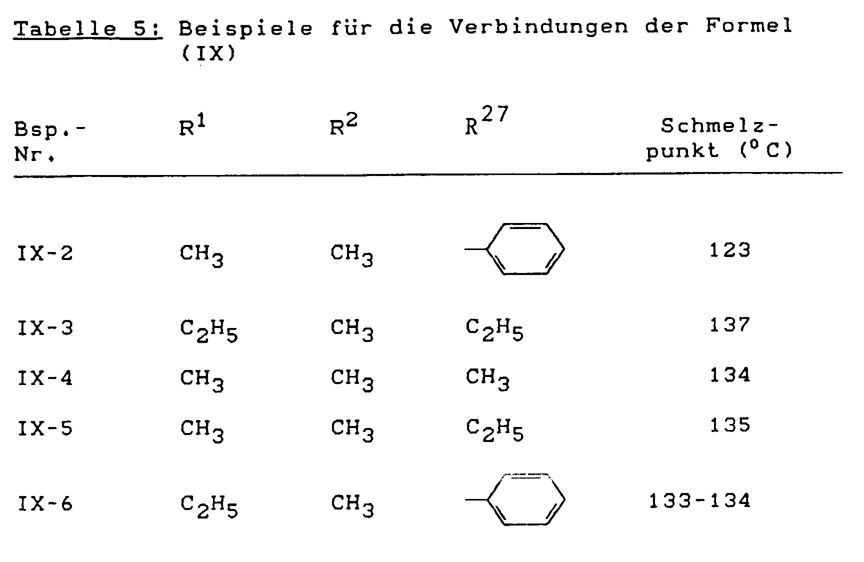

### Anwendungsbeispiele:

In den Anwendungsbeispielen wird die folgende Verbindung (A) als Vergleichssubstanz herangezogen: 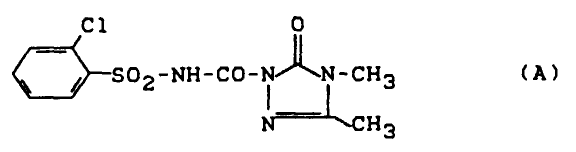 2-(2-Chlor-phenylsulfonylaminocarbonyl)-4,5-dimethyl-2,4-dihydro-3H-1,2,4-triazol-3-on
(bekannt aus EP-A 341489).

### Beispiel A

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
- 0 % =: keine Wirkung (wie unbehandelte Kontrolle)
- 100 % =: totale Vernichtung

In diesem Test zeigen beispielsweise die Verbindungen gemäß den Herstellungsbeispielen 1, 2, 3, 53, 54, 55, 56, 57, 58, 64, 65 und 67 bei teilweise guter Nutzpflanzenverträglichkeit wesentlich stärkere Wirkung gegen Unkräuter als die bekannte Verbindung (A).

### Beispiel B

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
- 0 % =: keine Wirkung (wie unbehandelte Kontrolle)
- 100 % =: totale Vernichtung

In diesem Test zeigen beispielsweise die Verbindungen gemäß den Herstellungsbeispielen 2, 54 und 69 bei teilweise guter Nutzpflanzenverträglichkeit wesentlich stärkere Wirkung gegen Unkräuter als die bekannte Verbindung (A).

## Patentansprüche

1. Sulfonylaminocarbonyltriazolinone mit über Sauerstoff gebundenen Substituenten der allgemeinen Formel (I), 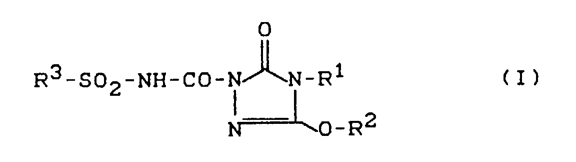 in welcher
R¹ für Wasserstoff, Amino, für gegebenenfalls durch Fluor, Chlor, Brom, Cyano, C₁-C₄-Alkoxy, C₁-C₄-Alkylcarbonyl oder C₁-C₄-Alkoxy-carbonyl substituiertes C₁-C₆-Alkyl, für jeweils gegebenenfalls durch Fluor, Chlor und/oder Brom substituiertes C₃-C₆-Alkenyl oder C₃-C₆-Alkinyl, für gegebenenfalls durch Fluor, Chlor, Brom und/oder C₁-C₄-Alkyl substituiertes C₃-C₆-Cycloalkyl, für gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl-C₁-C₃-alkyl, für gegebenenfalls durch Fluor, Chlor, Brom, Cyano, Nitro, C₁-C₄-Alkyl, Trifluormethyl, C₁-C₄-Alkoxy, Fluor- und/oder Chlor-substituiertes C₁-C₃-Alkoxy, C₁-C₄-Alkylthio-Fluor- und/oder Chlor- substituiertes C₁-C₃-Alkylthio, C₁-C₄-Alkylsulfinyl, C₁-C₄-Alkylsulfonyl und/oder C₁-C₄-Alkoxy-carbonyl substituiertes Phenyl, für gegebenenfalls durch Fluor, Cyano, C₁-C₄-Alkoxy oder C₁-C₄-Alkoxy-carbonyl substituiertes C₁-C₄-Alkylamino, C₃-C₆-Cycloalkylamino oder für Di-(C₁-C₄-alkyl)-amino steht,
R² für gegebenenfalls durch Fluor, Chlor, Brom, Cyano, C₃-C₆-Cycloalkyl, C₁-C₄-Alkoxy oder C₁-C₄-Alkoxy-carbonyl substituiertes C₁-C₆-Alkyl, für jeweils gegebenenfalls durch Fluor, Chlor und/oder Brom substituiertes C₃-C₆-Alkenyl oder C₃-C₆-Alkinyl, für gegebenenfalls durch Fluor, Chlor, Brom und/oder C₁-C₄-Alkyl substituiertes C₃-C₆-Cycloalkyl, für Cyclohexenyl, für gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl-C₁-C₃-alkyl, oder für gegebenenfalls durch Fluor, Chlor, Brom, Cyano, Nitro, C₁-C₄-Alkyl, Trifluormethyl, C₁-C₄-Alkoxy, Fluor- und/oder Chlor-substituiertes C₁-C₃-Alkoxy, C₁-C₄-Alkylthio, Fluor-und/oder Chlor-substituiertes C₁-C₃-Alkylthio, C₁-C₄-Alkyl-sulfinyl, C₁-C₄-Alkylsulfonyl und/oder C₁-C₄-Alkoxy-carbonyl substituiertes Phenyl steht und
R³ für die Gruppierung 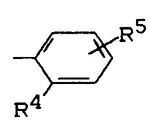 steht, worin
R⁴ und R⁵ gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxycarbonyl, C₁-C₄-Alkylamino-carbonyl, Di-(C₁-C₄-alkyl)amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist), für C₂-C₆-Alkenyl (welches gegebenenfalls durch Fluor, Chlor, Brom, Cyano, C₁-C₄-Alkoxy-carbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxy-carbonyl, C₁-C₄-Alkylthio, C₁-C₄-Alkylsulfinyl oder C₁-C₄-Alkylsulfonyl substituiert ist), für C₃-C₆-Alkenyloxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxycarbonyl substituiert ist), C₃-C₆-Alkinyloxy, C₃-C₆-Alkinylthio oder für den Rest -S(O)ₚ-R⁶ stehen, wobei
p für die Zahlen 1 oder 2 steht und
R⁶ für C₁-C₄-Alkyl (welches gegebenenfalls durch Fluor, Chlor, Brom, Cyano oder C₁-C₄-Alkoxy-carbonyl substituiert ist), C₃-C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
R⁷ für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkyl-carbonyl, C₁-C₄-Alkoxycarbonyl, C₁-C₄-Alkylamino-carbonyl oder Di-(C₁-C₄-alkyl)-amino-carbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-Cycloalkyl-C₁-C₂-alkyl, Phenyl-C₁-C₂-alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio, Trifluormethylthio oder C₁-C₄-Alkoxy-carbonyl substituiert ist) steht,
R⁴ und/oder R⁵ weiterhin für Phenyl oder Phenoxy, für C₁-C₄-Alkylcarbonylamino, C₁-C₄-Alkoxy-carbonylamino, C₁-C₄-Alkylamino-carbonyl-amino, Di-(C₁-C₄-alkyl)-amino-carbonylamino, oder für den Rest -CO-R⁸ stehen, wobei
R⁸ für C₁-C₆-Alkyl, C₁-C₆-Alkoxy, C₃-C₆-Cycloalkoxy, C₃-C₆-Alkenyloxy, C₁-C₄ Alkylthio, C₁-C₄-Alkylamino, C₁-C₄-Alkoxyamino, C₁-C₄-Alkoxy-C₁-C₄-alkyl-amino oder Di-(C₁-C₄-alkyl)-amino steht (welche gegebenenfalls durch Fluor und/oder Chlor substituiert sind),
R⁴ und/oder R⁵ weiterhin für Trimethylsilyl, Thiazolinyl, C₁-C₄-Alkylsulfonyloxy, Di-(C₁-C₄-alkyl)-aminosulfonylamino oder für den Rest -CH=N-R⁹ stehen, wobei
R⁹ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/oder Chlor substituiertes C₁-C₆-Alkoxy, C₃-C₆-Alkenoxy, C₃-C₆-Alkinoxy oder Benzyloxy, für Amino, C₁-C₄-Alkylamino, Di-(C₁-C₄-alkyl)-amino, Phenylamino, C₁-C₄-Alkyl-carbonyl-amino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht,
weiterhin
R³ für den Rest 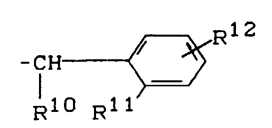 steht, worin
R¹⁰ für Wasserstoff oder C₁-C₄-Alkyl steht,
R¹¹ und R¹² gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), Carboxy, C₁-C₄-Alkoxy-carbonyl, Dimethylaminocarbonyl, C₁-C₄-Alkylsulfonyl oder Di-(C₁-C₄-alkyl)-aminosulfonyl stehen;
weiterhin
R³ für den Rest 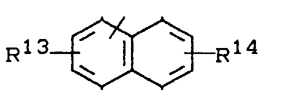 steht, worin
R¹³ und R¹⁴ gleich oder verschieden sind und für Wasserstoff, Fluor, Chlor, Brom, Nitro, Cyano, C₁-C₄-Alkyl (welches gegebenenfalls durch Fluor und/oder Chlor substituiert ist) oder C₁-C₄-Alkoxy (welches gegebenenfalls durch Fluor und/oder Chlor substituiert ist), stehen;
weiterhin
R³ für den Rest 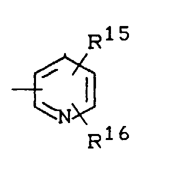 steht, worin
R¹⁵ und R¹⁶ gleich oder verschieden sind und für Wasserstoff, Fluor, Chlor, Brom, Nitro, Cyano, C₁-C₄-Alkyl (welches gegebenenfalls durch Flu or und/oder Chlor substituiert ist), C₁-C₄-Alkoxy (welches gegebenenfalls durch Fluor und/ oder Chlor substituiert ist), für C₁-C₄-Alkylthio, C₁-C₄-Alkylsulfinyl oder C₁-C₄-Alkylsulfonyl (welche gegebenenfalls durch Fluor und/ oder Chlor substituiert sind), für Aminosulfonyl, Mono-(C₁-C₄-alkyl)-aminosulfonyl, für Di-(C₁-C₄-alkyl)-aminosulfonyl oder C₁-C₄-Alkoxy-carbonyl oder Dimethylaminocarbonyl stehen;
weiterhin
R³ für den Rest 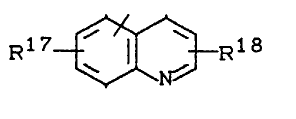 steht, worin
R¹⁷ und R¹⁸ gleich oder verschieden sind und für Wasserstoff, Fluor, Chlor, Brom, C₁-C₄-Alkyl (welches gegebenenfalls durch Fluor und/oder Brom substituiert ist), C₁-C₄-Alkoxy (welches gegebenenfalls durch Fluor und/oder Chlor substituiert ist), für C₁-C₄-Alkylthio, C₁-C₄-Alkylsulfinyl oder C₁-C₄-Alkylsulfonyl (welche gegebenenfalls durch Fluor und/oder Chlor substituiert sind), oder für Di-(C₁-C₄-Alkyl)-aminosulfonyl stehen;
weiterhin
R³ für den Rest 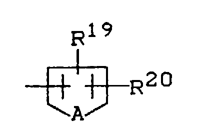 steht, worin
R¹⁹ und R²⁰ gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), C₁-C₄-Alkylthio, C₁-C₄-Alkylsulfinyl oder C₁-C₄-Alkylsulfonyl (welches gegebenenfalls durch Fluor und/oder Chlor substituiert ist), Di-(C₁-C₄-alkyl)-amino-sulfonyl, C₁-C₄-Alkoxy-carbonyl oder Dimethylaminocarbonyl stehen, und
A für Sauerstoff, Schwefel oder die Gruppierung N-Z¹ steht, wobei
Z¹ für Wasserstoff, C₁-C₄-Alkyl (welches gegebenenfalls durch Fluor, Chlor, Brom oder Cyano substituiert ist), C₃-C₆-Cycloalkyl, Benzyl, Phenyl (welches gegebenenfalls durch Fluor, Chlor, Brom oder Nitro substituiert ist), C₁-C₄-Alkylcarbonyl, C₁-C₄-Alkoxy-carbonyl oder Di-(C₁-C₄-alkyl)-aminocarbonyl steht;
weiterhin
R³ für den Rest 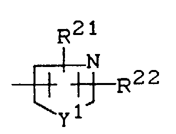 steht, worin
R²¹ und R²² gleich oder verschieden sind und für Wasserstoff, C₁-C₄-Alkyl, Halogen, C₁-C₄-Alkoxycarbonyl, C₁-C₄-Alkoxy oder C₁-C₄-Halogenalkoxy stehen,
Y¹ für Schwefel oder die Gruppierung N-R²³ steht, wobei
R²³ für Wasserstoff oder C₁-C₄-Alkyl steht;
weiterhin
R³ für den Rest 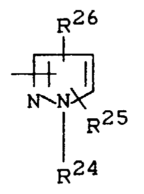 steht, worin
R²⁴ für Wasserstoff, C₁-C₄-Alkyl, Benzyl, Pyridyl, Chinolinyl oder Phenyl steht,
R²⁵ für Wasserstoff, Halogen, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Dioxolanyl oder C₁-C₄-Alkoxy-carbonyl steht und
R²⁶ für Wasserstoff, Halogen oder C₁-C₄-Alkyl steht;
weiterhin
R³ für eine der nachstehend aufgeführten Gruppierungen steht, 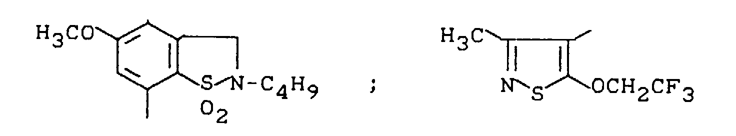 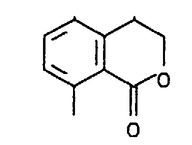
sowie Salze von Verbindungen der Formel (I), ausgenommen die Verbindungen
2-(2-Methoxycarbonyl-phenylsulfonylaminocarbonyl)-4-methyl-5-methoxy-2,4-dihydro-3H-1,2,4-triazol-3-on,
2-(2-Trifluormethoxy-phenylsulfonylaminocarbonyl)-4-cyclopropyl-5-methoxy-2,4-dihydro-3H-1,2,4-triazol-3-on und
2-(Difluormethoxy-phenylsulfonylaminocarbonyl)-4-cyclobutyl-5-ethoxy-2,4-dihydro-3H-1,2,4-triazol-3-on.

2. Sulfonylaminocarbonyltriazolinone der Formel (I) gemäß Anspruch 1, dadurch gekennzeichnet, daß darin
R¹ für Wasserstoff, Amino, für gegebenenfalls durch Fluor, Cyano, Methoxy oder Ethoxy substituiertes C₁-C₄-Alkyl, für Allyl, für C₃-C₆-Cycloalkyl, für Benzyl, für Phenyl, für C₁-C₃-Alkylamino, C₃-C₆-Cycloalkylamino oder für Di-(C₁-C₃-alkyl)-amino steht,
R² für Wasserstoff, für gegebenenfalls durch Fluor und/oder Chlor, Methoxy oder Ethoxy substituiertes C₁-C₄-Alkyl, für gegebenenfalls durch Fluor und/ oder Chlor substituiertes C₃-C₄-Alkenyl, für C₃-C₆-Cycloalkyl oder für gegebenenfalls durch Fluor, Chlor und/oder Methyl substituiertes Benzyl steht, und
R³ für die Gruppierung 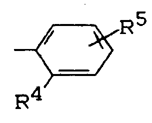 steht, worin
R⁴ für Fluor, Chlor, Brom, Methyl, Trifluormethyl, Methoxy, Difluormethoxy, Trifluormethoxy, 2-Chlor-ethoxy, 2-Methoxy-ethoxy, C₁-C₃-Alkylthio, C₁-C₃-Alkylsulfinyl, C₁-C₃-Alkylsulfonyl, Dimethylaminosulfonyl, Diethylaminosulfonyl, N-Methoxy-N-methylaminosulfonyl, Methoxyaminosulfonyl, Phenyl, Phenoxy oder C₁-C₃-Alkoxy-carbonyl steht und
R⁵ für Wasserstoff, Fluor, Chlor oder Brom steht;
weiterhin
R³ für den Rest 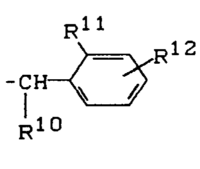 steht, worin
R¹⁰ für Wasserstoff steht,
R¹¹ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und
R¹² für Wasserstoff steht;
weiterhin
R³ für den Rest 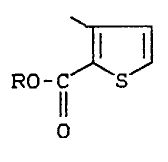 steht,
worin R für C₁-C₄-Alkyl steht, oder
für den Rest 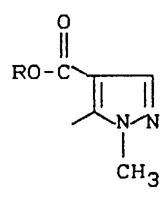 steht, worin R für C₁-C₄-Alkyl steht,
sowie Salze von Verbindungen der Formel (I),
wobei dieselben drei Verbindungen ausgeschlossen sind wie bei Anspruch 1.

3. Die Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, C₅- oder C₆-Cycloalkyl-ammonium- und Di-(C₁-C₂-alkyl)-benzylammonium-Salze von Verbindungen der Formel (I) gemäß Anspruch 1.

4. Verfahren zur Herstellung von Sulfonylaminotriazolinonen der allgemeinen Formel (I) 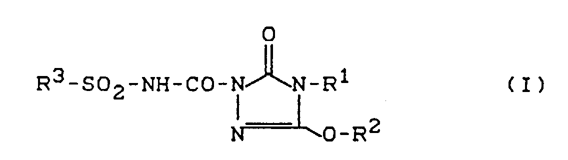 in welcher
R¹ für Wasserstoff, Amino, für gegebenenfalls durch Fluor, Chlor, Brom, Cyano, C₁-C₄-Alkoxy, C₁-C₄-Alkylcarbonyl oder C₁-C₄-Alkoxy-carbonyl substituiertes C₁-C₆-Alkyl, für jeweils gegebenenfalls durch Fluor, Chlor und/oder Brom substituiertes C₃-C₆-Alkenyl oder C₃-C₆-Alkinyl, für gegebenenfalls durch Fluor, Chlor, Brom und/oder C₁-C₄-Alkyl substituiertes C₃-C₆-Cycloalkyl, für gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl-C₁-C₃-alkyl, für gegebenenfalls durch Fluor, Chlor, Brom, Cyano, Nitro, C₁-C₄-Alkyl, Trifluormethyl, C₁-C₄-Alkoxy, Fluor- und/oder Chlor-substituiertes C₁-C₃-Alkoxy, C₁-C₄-Alkylthio, Fluor- und/oder Chlor- substituiertes C₁-C₃-Alkylthio, C₁-C₄-Alkylsulfinyl, C₁-C₄-Alkylsulfonyl und/oder C₁-C₄-Alkoxy-carbonyl substituiertes Phenyl, für gegebenenfalls durch Fluor, Cyano, C₁-C₄-Alkoxy oder C₁-C₄-Alkoxycarbonyl substituiertes C₁-C₄-Alkylamino, C₃-C₆-Cycloalkylamino oder für Di-(C₁-C₄-alkyl)-amino steht,
R² für gegebenenfalls durch Fluor, Chlor, Brom, Cyano, C₃-C₆-Cycloalkyl, C₁-C₄-Alkoxy oder C₁-C₄-Alkoxy-carbonyl substituiertes C₁-C₆-Alkyl, für jeweils gegebenenfalls durch Fluor, Chlor und/oder Brom substituiertes C₃-C₆-Alkenyl oder C₃-C₆-Alkinyl, für gegebenenfalls durch Fluor, Chlor, Brom und/oder C₁-C₄-Alkyl substituiertes C₃-C₆-Cycloalkyl, für Cyclohexenyl, für gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl-C₁-C₃-alkyl, oder für gegebenenfalls durch Fluor, Chlor, Brom, Cyano, Nitro, C₁-C₄-Alkyl, Trifluormethyl, C₁-C₄-Alkoxy, Fluor- und/oder Chlor-substituiertes C₁-C₃-Alkoxy, C₁-C₄-Alkylthio, Fluor-und/oder Chlor-substituiertes C₁-C₃-Alkylthio, C₁-C₄-Alkyl-sulfinyl, C₁-C₄-Alkylsulfonyl und/oder C₁-C₄-Alkoxy-carbonyl substituiertes Phenyl steht und
R³ für die Gruppierung 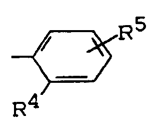 steht, worin
R⁴ und R⁵ gleich oder verschieden sind und für Wasserstoff, Fluor, Chlor, Brom, Iod, Nitro, C₁-C₆-Alkyl (welches gegebenenfalls durch Fluor, Chlor, Brom, Cyano, Carboxy, C₁-C₄-Alkoxycarbonyl, C₁-C₄-Alkylamino-carbonyl, Di-(C₁-C₄-alkyl)amino-carbonyl, Hydroxy, C₁-C₄-Alkoxy, Formyloxy, C₁-C₄-Alkyl-carbonyloxy, C₁-C₄-Alkoxy-carbonyloxy, C₁-C₄-Alkylamino-carbonyloxy, C₁-C₄-Alkylthio, C₁-C₄-Alkylsulfinyl, C₁-C₄-Alkylsulfonyl, Di-(C₁-C₄-alkyl)-aminosulfonyl, C₃-C₆-Cycloalkyl oder Phenyl substituiert ist), für C₂-C₆-Alkenyl (welches gegebenenfalls durch Fluor, Chlor, Brom, Cyano, C₁-C₄-Alkoxy-carbonyl, Carboxy oder Phenyl substituiert ist), für C₂-C₆-Alkinyl (welches gegebenenfalls durch Fluor, Chlor, Brom, Cyano, C₁-C₄-Alkoxy-carbonyl, Carboxy oder Phenyl substituiert ist), für C₁-C₄-Alkoxy (welches gegebenenfalls durch Fluor, Chlor, Brom, Cyano, Carboxy, C₁-C₄-Alkoxy-carbonyl, C₁-C₄-Alkoxy, C₁-C₄-Alkylthio, C₁-C₄-Alkylsulfinyl oder C₁-C₄-Alkylsulfonyl substituiert ist), für C₁-C₄-Alkylthio (welches gegebenenfalls durch Fluor, Chlor, Brom, Cyano, Carboxy, C₁-C₄-Alkoxy-carbonyl, C₁-C₄-Alkylthio, C₁-C₄-Alkylsulfinyl oder C₁-C₄-Alkylsulfonyl substituiert ist), für C₃-C₆-Alkenyloxy (welches gegebenenfalls durch Fluor, Chlor, Brom, Cyano oder C₁-C₄-Alkoxy-carbonyl substituiert ist), für C₂-C₆-Alkenylthio (welches gegebenenfalls durch Fluor, Chlor, Brom, Cyano, Nitro, C₁-C₃-Alkylthio oder C₁-C₄-Alkoxycarbonyl substituiert ist), C₃-C₆-Alkinyloxy, C₃-C₆-Alkinylthio oder für den Rest -S(O)ₚ-R⁶ stehen, wobei
p für die Zahlen 1 oder 2 steht und
R⁶ für C₁-C₄-Alkyl (welches gegebenenfalls durch Fluor, Chlor, Brom, Cyano oder C₁-C₄-Alkoxy-carbonyl substituiert ist), C₃-C₆-Alkenyl, C₃-C₆-Alkinyl, C₁-C₄-Alkoxy, C₁-C₄-Alkoxy-C₁-C₄-alkylamino, C₁-C₄-Alkylamino, Di-(C₁-C₄-alkyl)-amino, Phenyl oder für den Rest -NHOR⁷ steht, wobei
R⁷ für C₁-C₁₂-Alkyl (welches gegebenenfalls durch Fluor, Chlor, Cyano, C₁-C₄-Alkoxy, C₁-C₄-Alkylthio, C₁-C₄-Alkylsulfinyl, C₁-C₄-Alkylsulfonyl, C₁-C₄-Alkyl-carbonyl, C₁-C₄-Alkoxy-carbonyl, C₁-C₄-Alkylamino-carbonyl oder Di-(C₁-C₄-alkyl)-amino-carbonyl substituiert ist), für C₃-C₆-Alkenyl (welches gegebenenfalls durch Fluor, Chlor oder Brom substituiert ist), C₃-C₆-Alkinyl, C₃-C₆-Cycloalkyl, C₃-C₆-Cycloalkyl-C₁-C₂-alkyl, Phenyl-C₁-C₂-alkyl (welches gegebenenfalls durch Fluor, Chlor, Nitro, Cyano, C₁-C₄-Alkyl, C₁-C₄-Alkoxy oder C₁-C₄-Alkoxy-carbonyl substituiert ist), für Benzhydryl oder für Phenyl (welches gegebenenfalls durch Fluor, Chlor, Nitro, Cyano, C₁-C₄-Alkyl, Trifluormethyl, C₁-C₄-Alkoxy, C₁-C₂-Fluoralkoxy, C₁-C₄-Alkylthio, Trifluormethylthio oder C₁-C₄-Alkoxy-carbonyl substituiert ist) steht,
R⁴ und/oder R⁵ weiterhin für Phenyl oder Phenoxy, für C₁-C₄-Alkylcarbonylamino, C₁-C₄-Alkoxy-carbonylamino, C₁-C₄-Alkylamino-carbonyl-amino, Di-(C₁-C₄-alkyl)-amino-carbonylamino, oder für den Rest -CO-R⁸ stehen, wobei
R⁸ für C₁-C₆-Alkyl, C₁-C₆-Alkoxy, C₃-C₆-Cycloalkoxy, C₃-C₆-Alkenyloxy, C₁-C₄ Alkylthio, C₁-C₄-Alkylamino, C₁-C₄-Alkoxyamino, C₁-C₄-Alkoxy-C₁-C₄-alkyl-amino oder Di-(C₁-C₄-alkyl)-amino steht (welche gegebenenfalls durch Fluor und/oder Chlor substituiert sind),
R⁴ und/oder R⁵ weiterhin für Trimethylsilyl, Thiazolinyl, C₁-C₄-Alkylsulfonyloxy, Di-(C₁-C₄-alkyl)-aminosulfonylamino oder für den Rest -CH=N-R⁹ stehen, wobei
R⁹ für gegebenenfalls durch Fluor, Chlor, Cyano, Carboxy, C₁-C₄-Alkoxy, C₁-C₄-Alkylthio, C₁-C₄-Alkylsulfinyl oder C₁-C₄-Alkylsulfonyl substituiertes C₁-C₆-Alkyl, für gegebenenfalls durch Fluor oder Chlor substituiertes Benzyl, für gegebenenfalls durch Fluor oder Chlor substituiertes C₃-C₆-Alkenyl oder C₃-C₆-Alkinyl, für gegebenenfalls durch Fluor, Chlor, Brom, C₁-C₄-Alkyl, C₁-C₄-Alkoxy, Trifluormethyl, Trifluormethoxy oder Trifluormethylthio substituiertes Phenyl, für gegebenenfalls durch Fluor und/oder Chlor substituiertes C₁-C₆-Alkoxy, C₃-C₆-Alkenoxy, C₃-C₆-Alkinoxy oder Benzyloxy, für Amino, C₁-C₄-Alkylamino, Di-(C₁-C₄-alkyl)-amino, Phenylamino, C₁-C₄-Alkyl-carbonyl-amino, C₁-C₄-Alkoxy-carbonylamino, C₁-C₄-Alkyl-sulfonylamino oder für gegebenenfalls durch Fluor, Chlor, Brom oder Methyl substituiertes Phenylsulfonylamino steht,
weiterhin
R³ für den Rest 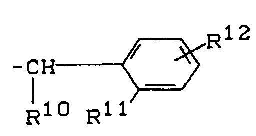 steht, worin
R¹⁰ für Wasserstoff oder C₁-C₄-Alkyl steht,
R¹¹ und R¹² gleich oder verschieden sind und für Wasserstoff, Fluor, Chlor, Brom, Nitro, Cyano, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), Carboxy, C₁-C₄-Alkoxy-carbonyl, Dimethylaminocarbonyl, C₁-C₄-Alkylsulfonyl oder Di-(C₁-C₄-alkyl)-aminosulfonyl stehen;
weiterhin
R³ für den Rest 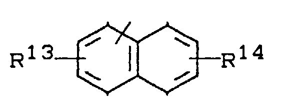 steht, worin
R¹³ und R¹⁴ gleich oder verschieden sind und für Wasserstoff, Fluor, Chlor, Brom, Nitro, Cyano, C₁-C₄-Alkyl (welches gegebenenfalls durch Fluor und/oder Chlor substituiert ist) oder C₁-C₄-Alkoxy (welches gegebenenfalls durch Fluor und/oder Chlor substituiert ist), stehen;
weiterhin
R³ für den Rest 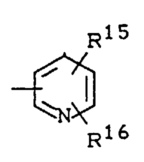 steht, worin
R¹⁵ und R¹⁶ gleich oder verschieden sind und für Wasserstoff, Fluor, Chlor, Brom, Nitro, Cyano, C₁-C₄-Alkyl (welches gegebenenfalls durch Flu or und/oder Chlor substituiert ist), C₁-C₄-Alkoxy (welches gegebenenfalls durch Fluor und/oder Chlor substituiert ist), für C₁-C₄-Alkyl-thio, C₁-C₄-Alkylsulfinyl oder C₁-C₄-Alkylsulfonyl (welche gegebenenfalls durch Fluor und/ oder Chlor substituiert sind), für Aminosulfonyl, Mono-(C₁-C₄-alkyl)-aminosulfonyl, für Di-(C₁-C₄-alkyl)-aminosulfonyl oder C₁-C₄-Alkoxy-carbonyl oder Dimethylaminocarbonyl stehen;
weiterhin
R³ für den Rest 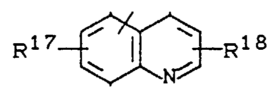 steht, worin
R¹⁷ und R¹⁸ gleich oder verschieden sind und für Wasserstoff, Fluor, Chlor, Brom, C₁-C₄-Alkyl (welches gegebenenfalls durch Fluor und/oder Brom substituiert ist), C₁-C₄-Alkoxy (welches gegebenenfalls durch Fluor und/oder Chlor substituiert ist), für C₁-C₄-Alkylthio, C₁-C₄-Alkylsulfinyl oder C₁-C₄-Alkylsulfonyl (welche gegebenenfalls durch Fluor und/oder Chlor substituiert sind), oder für Di-(C₁-C₄-alkyl)-aminosulfonyl stehen;
weiterhin
R³ für den Rest 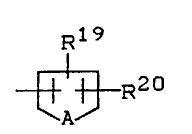 steht, worin
R¹⁹ und R²⁰ gleich oder verschieden sind und für Wasserstoff, Fluor, Chlor, Brom, Cyano, Nitro, C₁-C₄-Alkyl (welches gegebenenfalls durch Fluor und/oder Chlor substituiert ist), C₁-C₄-Alkoxy (welches gegebenenfalls durch Fluor und/ oder Chlor substituiert ist), C₁-C₄-Alkylthio-C₁-C₄-Alkylsulfinyl oder C₁-C₄-Alkylsulfonyl (welches gegebenenfalls durch Fluor und/oder Chlor substituiert ist), Di-(C₁-C₄-alkyl)-amino-sulfonyl, C₁-C₄-Alkoxy-carbonyl oder Dimethylaminocarbonyl stehen, und
A für Sauerstoff, Schwefel oder die Gruppierung N-Z¹ steht, wobei
Z¹ für Wasserstoff, C₁-C₄-Alkyl (welches gegebenenfalls durch Fluor, Chlor, Brom oder Cyano substituiert ist), C₃-C₆-Cycloalkyl, Benzyl, Phenyl (welches gegebenenfalls durch Fluor, Chlor, Brom oder Nitro substituiert ist), C₁-C₄-Alkylcarbonyl, C₁-C₄-Alkoxy-carbonyl oder Di-(C₁-C₄-alkyl)-aminocarbonyl steht;
weiterhin
R³ für den Rest 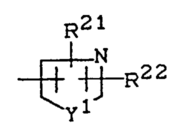 steht, worin
R²¹ und R²² gleich oder verschieden sind und für Wasserstoff, C₁-C₄-Alkyl, Halogen, C₁-C₄-Alkoxycarbonyl, C₁-C₄-Alkoxy oder C₁-C₄-Halogenalkoxy stehen,
Y¹ für Schwefel oder die Gruppierung N-R²³ steht, wobei
R²³ für Wasserstoff oder C₁-C₄-Alkyl steht;
weiterhin
R³ für den Rest 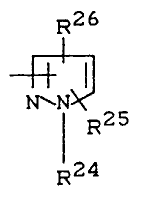 steht, worin
R²⁴ für Wasserstoff, C₁-C₄-Alkyl, Benzyl, Pyridyl, Chinolinyl oder Phenyl steht,
R²⁵ für Wasserstoff, Halogen, Cyano, Nitro, C₁-C₄-Alkyl (welches gegebenenfalls durch Fluor und/oder Chlor substituiert ist), C₁-C₄-Alkoxy (welches gegebenenfalls durch Fluor und/oder Chlor substituiert ist), Dioxolanyl oder C₁-C₄-Alkoxy-carbonyl steht und
R²⁶ für Wasserstoff, Halogen oder C₁-C₄-Alkyl steht;
weiterhin
R³ für eine der nachstehend aufgeführten Gruppierungen steht, 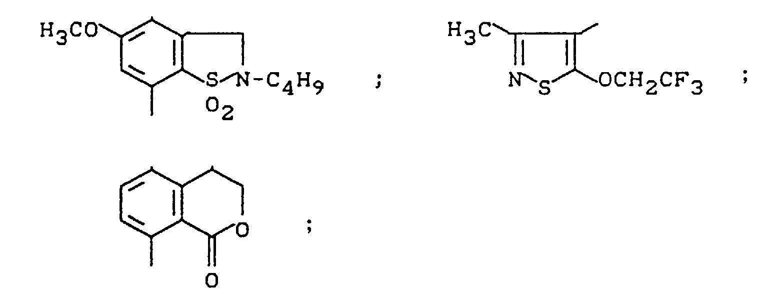
sowie von Salzen der Verbindungen der Formel (I), wobei jedoch die Verbindungen
2-(2-Methoxycarbonyl-phenylsulfonylaminocarbonyl)-4-methyl-5-methoxy-2,4-dihydro-3H-1,2,4-triazol-3-on,
2-(2-Trifluormethoxy-phenyl sulfonylaminocarbonyl)-4-cyclopropyl-5-methoxy-2,4-dihydro-3H-1,2,4-triazol-3-on und
2-(Difluormethoxy-phenyl sulfonyl aminocarbonyl)-4-cyclobutyl-5-ethoxy-2,4-dihydro-3H-1,2,4-triazol-3 -on
ausgeschlossen sind,
dadurch gekennzeichnet, daß man
(a) Triazolinone der allgemeinen Formel (II) 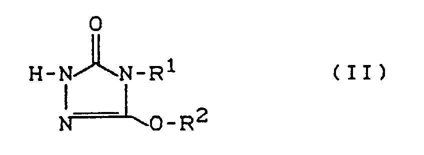 in welcher
R¹ und R² die oben bei Formel (I) angegebenen Bedeutungen haben,
mit Sulfonylisocyanaten der allgemeinen Formel (III)
R³-SO₂-N=C=O (III)
in welcher
R³ die oben bei Formel (I) angegebene Bedeutung hat,
gegebenenfalls in Gegenwart eines Verdünnungsmittels umsetzt, oder daß man
(b) Triazolinon-Derivate der allgemeinen Formel (IV) 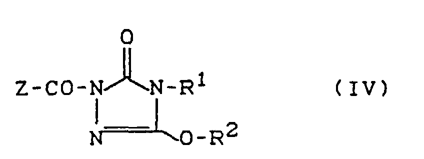 in welcher
R¹ und R² die bei Formel (I) angegebenen Bedeutungen haben und
Z für Chlor, C₁-C₄-Alkoxy, Benzyloxy oder Phenoxy steht,
mit Sulfonsäureamiden der allgemeinen Formel (V)
R³-SO₂-NH₂ (V)
in welcher
R³ die bei Formel (I) angegebene Bedeutung hat,
gegebenenfalls in Gegenwart eines Säureakzeptors und gegebenenfalls in Gegenwart eines Verdünnungsmittels umsetzt, oder daß man
(c) Triazolinone der allgemeinen Formel (II) 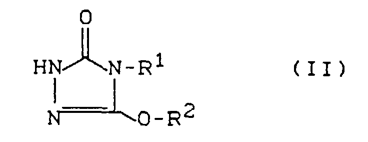 in welcher
R¹ und R² die bei Formel (I) angegebenen Bedeutungen haben,
mit Sulfonsäureamid-Derivaten der allgemeinen Formel (VI)
R³-SO₂-NH-CO-Z (VI)
in welcher
R³ die bei Formel (I) angegebene Bedeutung hat und
Z für Chlor, C₁-C₄-Alkoxy, Benzyloxy oder Phenoxy steht,
gegebenenfalls in Gegenwart eines Säureakzeptors und gegebenenfalls in Gegenwart eines Verdünnungsmittels umsetzt
und gegebenenfalls aus den nach Verfahren (a), (b) oder (c) hergestellten Verbindungen der Formel (I) nach üblichen Methoden Salze erzeugt.

5. Herbizide Mittel, gekennzeichnet durch einen Gehalt an mindestens einem Sulfonylaminocarbonyltriazolinon der Formel (I) gemäß Anspruch 1 oder 4.

6. Verwendung von Sulfonylaminocarbonyltriazolinonen der allgemeinen Formel (I) gemäß Anspruch 1 oder 4 zur Bekämpfung von unerwünschtem Pflanzenwachstum.

7. Verfahren zur Bekämpfung von Unkräutern, dadurch gekennzeichnet, daß man Sulfonylaminocarbonyltriazolinone der allgemeinen Formel (I) gemäß Anspruch 1 oder 4 auf die Unkräuter oder ihren Lebensraum einwirken läßt.

8. Verfahren zur Herstellung von herbiziden Mitteln, dadurch gekennzeichnet, daß man Sulfonylaminocarbonyitriazolinone der allgemeinen Formel (I) gemäß Anspruch 1 oder 4 mit Streckmitteln und/oder oberflächenaktiven Mitteln vermischt.

9. Triazolinone der allgemeinen Formel (II) 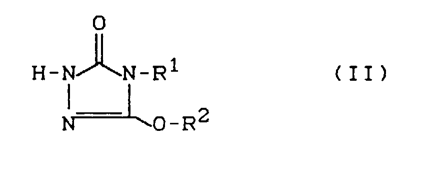 in welcher
R¹ für Amino, für gegebenenfalls durch Fluor, Chlor, Brom, Cyano, C₁-C₄-Alkoxy, C₁-C₄-Alkylcarbonyl oder C₁-C₄-Alkoxy-carbonyl substituiertes C₁-C₆-Alkyl, für jeweils gegebenenfalls durch Fluor, Chlor und/oder Brom substituiertes C₃-C₆-Alkenyl oder C₃-C₆-Alkinyl, für gegebenenfalls durch Fluor, Chlor, Brom und/oder C₁-C₄-Alkyl substituiertes C₃-C₆-Cycloalkyl, für gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl-C₁-C₃-alkyl, für gegebenenfalls durch Fluor, Chlor, Brom, Cyano, Nitro, C₁-C₄-Alkyl, Trifluormethyl, C₁-C₄-Alkoxy, Fluor- und/oder Chlor-substituiertes C₁-C₃-Alkoxy, C₁-C₄-Alkylthio, Fluor- und/oder Chlor-substituiertes C₁-C₃-Alkylthio, C₁-C₄-Alkylsulfinyl, C₁-C₄-Alkylsulfonyl und/oder C₁-C₄-Alkoxy-carbonyl substituiertes Phenyl, für gegebenenfalls durch Fluor, Cyano, C₁-C₄-Alkoxy oder C₁-C₄-Alkoxy-carbonyl substituiertes C₁-C₄-Alkylamino, C₃-C₆-Cycloalkylamino oder für Di-(C₁-C₄-alkyl)-amino steht und
R² für gegebenenfalls durch Fluor, Chlor, Brom, und/oder C₁-C₄-Alkyl substituiertes C₃-C₆-Cycloalkyl, für Cyclohexenyl, für gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl-C₁-C₃-alkyl, oder für gegebenenfalls durch Fluor, Chlor, Brom, Cyano, Nitro, C₁-C₄-Alkyl, Trifluormethyl, C₁-C₄-Alkoxy, Fluor- und/oder Chlor-substituiertes C₁-C₃-Alkoxy, C₁-C₄-Alkylthio, Fluor-und/oder Chlor-substituiertes C₁-C₃-Alkylthio, C₁-C₄-Alkyl-sulfinyl, C₁-C₄-Alkylsulfonyl und/oder C₁-C₄-Alkoxy-carbonyl substituiertes Phenyl steht.

10. Verfahren zur Herstellung von Triazolinonen der allgemeinen Formel (II) 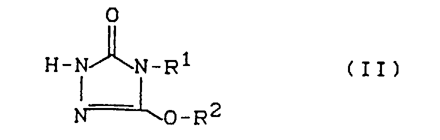 in welcher
R¹ für Amino, für gegebenenfalls durch Fluor, Chlor, Brom, Cyano, C₁-C₄-Alkoxy, C₁-C₄-Alkylcarbonyl oder C₁-C₄-Alkoxy-carbonyl substituiertes C₁-C₆-Alkyl, für jeweils gegebenenfalls durch Fluor, Chlor und/oder Brom substituiertes C₃-C₆-Alkenyl oder C₃-C₆-Alkinyl, für gegebenenfalls durch Fluor, Chlor, Brom und/oder C₁-C₄-Alkyl substituiertes C₃-C₆-Cycloalkyl, für gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl-C₁-C₃-alkyl, für gegebenenfalls durch Fluor, Chlor, Brom, Cyano, Nitro, C₁-C₄-Alkyl, Trifluormethyl, C₁-C₄-Alkoxy, Fluor- und/oder Chlor-substituiertes C₁-C₃-Alkoxy, C₁-C₄-Alkylthio, Fluor- und/oder Chlor-substituiertes C₁-C₃-Alkylthio, C₁-C₄-Alkylsulfinyl, C₁-C₄-Alkylsulfonyl und/oder C₁-C₄-Alkoxy-carbonyl substituiertes Phenyl, für gegebenenfalls durch Fluor, Cyano, C₁-C₄-Alkoxy oder C₁-C₄-Alkoxy-carbonyl substituiertes C₁-C₄-Alkylamino, C₃-C₆-Cycloalkylamino oder für Di-(C₁-C₄-alkyl)-amino steht und
R² für gegebenenfalls durch Fluor, Chlor, Brom, und/oder C₁-C₄-Alkyl substituiertes C₃-C₆-Cycloalkyl, für Cyclohexenyl, für gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl-C₁-C₃-alkyl, oder für gegebenenfalls durch Fluor, Chlor, Brom, Cyano, Nitro, C₁-C₄-Alkyl, Trifluormethyl, C₁-C₄-Alkoxy, Fluor- und/oder Chlor-substituiertes C₁-C₃-Alkoxy, C₁-C₄-Alkylthio, Fluor-und/oder Chlor-substituiertes C₁-C₃-Alkylthio, C₁-C₄-Alkyl-sulfinyl, C₁-C₄-Alkylsulfonyl und/oder C₁-C₄-Alkoxy-carbonyl substituiertes Phenyl steht ,
dadurch gekennzeichnet, daß man Hydrazinoameisensäureester der allgemeinen Formel (VII)
H₂N-NH-CO-O-R²⁷ (VII),
in welcher
R²⁷ für Methyl, Ethyl oder Phenyl steht,
mit Alkyliminokohlensäurediestern der allgemeinen Formel (VIII) 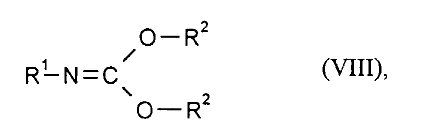 in welcher
R¹ und R² die oben angegebene Bedeutung haben,
gegebenenfalls in Gegenwart eines Verdünnungsmittels bei Temperaturen zwischen 0°C und 50°C umsetzt, die hierbei gebildeten Verbindungen der allgemeinen Formel (IX) 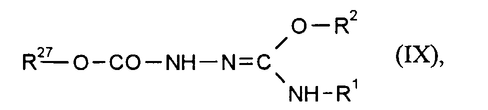 in welcher
R¹, R² und R²⁷ die oben angegebene Bedeutung haben,
gegebenenfalls nach üblichen Methoden isoliert und auf Temperaturen zwischen 50°C und 150°C, gegebenenfalls in Gegenwart eines Verdünnungsmittels, erhitzt.

11. Verbindungen der allgemeinen Formel (IX) 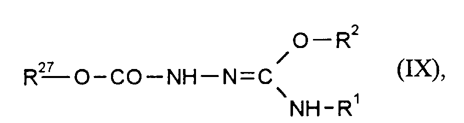 in welcher
R¹ und R² die in Anspruch 9 angegebenen Bedeutungen haben und
R²⁷ für Methyl, Ethyl oder Phenyl steht.

12. Verfahren nach Anspruch 10, dadurch gekennzeichnet, daß man als Hydrazinoameisensäureester der Formel (VII) Hydrazinoameisensäurephenylester und als Alkyliminokohlensäurediester der Formel (VIII) Trimethyliminocarbonat einsetzt, wobei man 4-Methyl-5-methoxy-2,4-dihydro-3H-1,2,4-triazol-3-on (II-2) erhält.

## Claims

1. Sulphonylaminocarbonyltriazolinones having substituents bonded by oxygen, of the general formula (I) 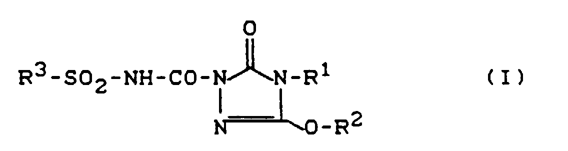 in which
R¹ represents hydrogen, amino, or represents C₁-C₆-alkyl which is optionally substituted by fluorine, chlorine, bromine, cyano, C₁-C₄-alkoxy, C₁-C₄-alkylcarbonyl or C₁-C₄-alkoxycarbonyl, or represents C₃-C₆-alkenyl or C₃-C₆-alkinyl, each of which is optionally substituted by fluorine, chlorine and/or bromine, or represents C₃-C₆-cycloalkyl which is optionally substituted by fluorine, chlorine, bromine and/or C₁-C₄-alkyl, or represents phenyl-C₁-C₃-alkyl which is optionally substituted by fluorine, chlorine, bromine, cyano, -nitro, C₁-C₄-alkyl, trifluoromethyl, C₁-C₄-alkoxy and/or C₁-C₄-alkoxy-carbonyl, or represents phenyl which is optionally substituted by fluorine, chlorine, bromine, cyano, nitro, C₁-C₄-alkyl, trifluoromethyl, C₁-C₄-alkoxy, fluorine- and/or chlorine-substituted C₁-C₃-alkoxy, C₁-C₄-alkylthio, fluorine- and/or chlorine-substituted C₁-C₃-alkylthio, C₁-C₄-alkylsulphinyl, C₁-C₄-alkylsulphonyl and/or C₁-C₄-alkoxy-carbonyl, or represents C₁-C₄-alkylamino which is optionally substituted by fluorine, cyano, C₁-C₄-alkoxy or C₁-C₄-alkoxy-carbonyl, or represents C₃-C₆-cycloalkylamino or di- (C₁-C₄-alkyl)-amino,
R² represents C₁-C₆-alkyl which is optionally substituted by fluorine, chlorine, bromine, cyano, C₃-C₆-cycloalkyl, C₁-C₄-alkoxy or C₁-C₄-alkoxy-carbonyl, or represents C₃-C₆-alkenyl or C₃-C₆-alkinyl, each of which is optionally substituted by fluorine, chlorine and/or bromine, or represents C₃-C₆-cycloalkyl which is optionally substituted by fluorine, chlorine, bromine and/or C₁-C₄-alkyl, or represents cyclohexenyl, or represents phenyl-C₁-C₃-alkyl which is optionally substituted by fluorine, chlorine, bromine, cyano, nitro, C₁-C₄-alkyl, trifluoromethyl, C₁-C₄-alkoxy and/or C₁-C₄-alkoxy-carbonyl, or represents phenyl which is optionally substituted by fluorine, chlorine, bromine, cyano, nitro, C₁-C₄-alkyl, trifluoromethyl, C₁-C₄-alkoxy, fluorine- and/or chlorine-substituted C₁-C₃-alkoxy, C₁-C₄-alkylthio, fluorine- and/or chlorine-substituted C₁-C₃-alkylthio, C₁-C₄-alkyl-sulphinyl, C₁-C₄-alkylsulphonyl and/or C₁-C₄-alkoxy-carbonyl, and
R³ represents the group 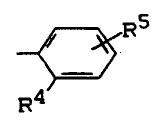 in which
R⁴ and R⁵ are identical or different and represent hydrogen, fluorine, chlorine, bromine, iodine, nitro, C₁-C₆-alkyl (which is optionally substituted by fluorine, chlorine, bromine, cyano, carboxyl, C₁-C₄-alkoxycarbonyl, C₁-C₄-alkylamino-carbonyl, di-(C₁-C₄-alkyl)amino-carbonyl, hydroxyl, C₁-C₄-alkoxy, formyloxy, C₁-C₄-alkyl-carbonyloxy, C₁-C₄-alkoxy-carbonyloxy, C₁-C₄-alkylamino-carbonyloxy, C₁-C₄-alkylthio, C₁-C₄-alkylsulphinyl, C₁-C₄-alkylsulphonyl, di-(C₁-C₄-alkyl)aminosulphonyl, C₃-C₆-cycloalkyl or phenyl), or represent C₂-C₆-alkenyl (which is optionally substituted by fluorine, chlorine, bromine, cyano, C₁-C₄-alkoxy-carbonyl, carboxyl or phenyl), or represent C₂-C₆-alkinyl (which is optionally substituted by fluorine, chlorine, bromine, cyano, C₁-C₄-alkoxy-carbonyl, carboxyl or phenyl), or represent C₁-C₄-alkoxy (which is optionally substituted by fluorine, chlorine, bromine, cyano, carboxyl, C₁-C₄-alkoxycarbonyl, C₁-C₄-alkoxy, C₁-C₄-alkylthio, C₁-C₄-alkylsulphinyl or C₁-C₄-alkylsulphonyl), or represent C₁-C₄-alkylthio (which is optionally substituted by fluorine, chlorine, bromine, cyano, carboxyl, C₁-C₄-alkoxycarbonyl, C₁-C₄-alkylthio, C₁-C₄-alkylsulphinyl or C₁-C₄-alkylsulphonyl), or represent C₃-C₆-alkenyloxy (which is optionally substituted by fluorine, chlorine, bromine, cyano or C₁-C₄-alkoxycarbonyl), or represent C₂-C₆-alkenylthio (which is optionally substituted by fluorine, chlorine, bromine, cyano, nitro, C₁-C₃-alkylthio or C₁-C₄-alkoxycarbonyl), C₃-C₆-alkinyloxy, C₃-C₆-alkinylthio or the radical -S(O)ₚ-R⁶ where
p represents the numbers 1 or 2 and
R⁶ represents C₁-C₄-alkyl (which is optionally substituted by fluorine, chlorine, bromine, cyano or C₁-C₄-alkoxy-carbonyl), C₃-C₆-alkenyl, C₃-C₆-alkinyl, C₁-C₄-alkoxy, C₁-C₄-alkoxy-C₁-C₄-alkylamino, C₁-C₄-alkylamino, di-(C₁-C₄-alkyl)-amino, phenyl or the radical -NHOR⁷ where
R⁷ represents C₁-C₁₂-alkyl (which is optionally substituted by fluorine, chlorine, cyano, C₁-C₄-alkoxy, C₁-C₄-alkylthio, C₁-C₄-alkylsulphinyl, C₁-C₄-alkylsulphonyl, C₁-C₄-alkyl-carbonyl, C₁-C₄-alkoxy-carbonyl, C₁-C₄-alkylamino-carbonyl or di-(C₁-C₄-alkyl)-amino-carbonyl), or represents C₃-C₆-alkenyl (which is optionally substituted by fluorine, chlorine or bromine), C₃-C₆-alkinyl, C₃-C₆-cycloalkyl, C₃-C₆-cycloalkyl-C₁-C₂-alkyl, phenyl-C₁-C₂-alkyl (which is optionally substituted by fluorine, chlorine, nitro, cyano, C₁-C₄-alkyl, C₁-C₄-alkoxy or C₁-C₄-alkoxy-carbonyl), or represents benzhydryl, or represents phenyl (which is optionally substituted by fluorine, chlorine, nitro, cyano, C₁-C₄-alkyl, trifluoromethyl, C₁-C₄-alkoxy, C₁-C₂-fluoroalkoxy, C₁-C₄-alkylthio, trifluoromethylthio or C₁-C₄-alkoxy-carbonyl),
R⁴ and/or R⁵ furthermore represent phenyl or phenoxy, or represent C₁-C₄-alkylcarbonylamino, C₁-C₄-alkoxycarbonylamino, C₁-C₄-alkylamino-carbonyl-amino, di-(C₁-C₄-alkyl)-amino-carbonylamino, or the radical -CO-R⁸ where
R⁸ represents C₁-C₆-alkyl, C₁-C₆-alkoxy, C₃-C₆-cycloalkoxy, C₃-C₆-alkenyloxy, C₁-C₄-alkylthio, C₁-C₄-alkylamino, C₁-C₄-alkoxyamino, C₁-C₄-alkoxy-C₁-C₄-alkyl-amino or di-(C₁-C₄-alkyl)-amino (which are optionally substituted by fluorine and/or chlorine),
R⁴ and/or R⁵ furthermore represent trimethylsilyl, thiazolinyl, C₁-C₄-alkylsulphonyloxy, di-(C₁-C₄-alkyl)-aminosulphonylamino or the radical -CH=N-R⁹ where
R⁹ represents C₁-C₆-alkyl which is optionally substituted by fluorine, chlorine, cyano, carboxyl, C₁-C₄-alkoxy, C₁-C₄-alkylthio, C₁-C₄-alkylsulphinyl or C₁-C₄-alkylsulphonyl, or represents benzyl which is optionally substituted by fluorine or chlorine, or represents C₃-C₆-alkenyl or C₃-C₆-alkinyl, each of which is optionally substituted by fluorine or chlorine, or represents phenyl which is optionally substituted by fluorine, chlorine, bromine, C₁-C₄-alkyl, C₁-C₄-alkoxy, trifluoromethyl, trifluoromethoxy or trifluoromethylthio, or represents optionally fluorine- and/or chlorine-substituted C₁-C₄-alkoxy, C₃-C₆-alkenoxy, C₃-C₆-alkinoxy or benzyloxy, or represents amino, C₁-C₄-alkylamino, di-(C₁-C₄-alkyl)-amino, phenyl-amino, C₁-C₄-alkyl-carbonyl-amino, C₁-C₄-alkoxy-carbonylamino or C₁-C₄-alkyl-sulphonylamino, or represents phenylsulphonylamino which is optionally substituted by fluorine, chlorine, bromine or methyl,
furthermore
R³ represents the radical 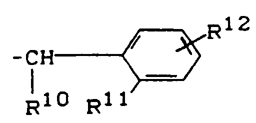 where
R¹⁰ represents hydrogen or C₁-C₄-alkyl,
R¹¹ and R¹² are identical or different and represent hydrogen, fluorine, chlorine, bromine, nitro, cyano, C₁-C₄-alkyl (which is optionally substituted by fluorine and/or chlorine), C₁-C₄-alkoxy (which is optionally substituted by fluorine and/or chlorine), carboxyl, C₁-C₄-alkoxy-carbonyl, dimethylaminocarbonyl, C₁-C₄-alkylsulphonyl or di-(C₁-C₄-alkyl) aminosulphonyl;
furthermore
R³ represents the radical 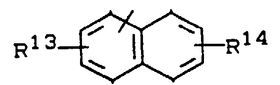 where
R¹³ and R¹⁴ are identical or different and represent hydrogen, fluorine, chlorine, bromine, nitro, cyano, C₁-C₄-alkyl (which is optionally substituted by fluorine and/or chlorine) or C₁-C₄-alkoxy (which is optionally substituted by fluorine and/or chlorine);
R³ represents the radical 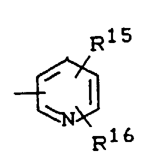 in which
R¹⁵ and R¹⁶ are identical or different and represent hydrogen, fluorine, chlorine, bromine, nitro, cyano, C₁-C₄-alkyl (which is optionally substituted by fluorine and/or chlorine), C₁-C₄-alkoxy (which is optionally substituted by fluorine and/or chlorine), or represent C₁-C₄-alkylthio, C₁-C₄-alkylsulphinyl or C₁-C₄-alkylsulphonyl (which are optionally substituted by fluorine and/or chlorine), or represent aminosulphonyl, mono-(C₁-C₄-alkyl)-aminosulphonyl, di-(C₁-C₄-alkyl)-aminosulphonyl or C₁-C₄-alkoxy-carbonyl or dimethylaminocarbonyl;
furthermore
R³ represents the radical 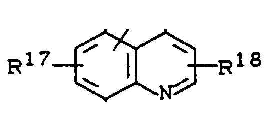 where
R¹⁷ and R¹⁸ are identical or different and represent hydrogen, fluorine, chlorine, bromine, C₁-C₄-alkyl (which is optionally substituted by fluorine and/or bromine), C₁-C₄-alkoxy (which is optionally substituted by fluorine and/or chlorine), or represent C₁-C₄-alkylthio, C₁-C₄-alkylsulphinyl or C₁-C₄-alkylsulphonyl (which are optionally substituted by fluorine and/or chlorine), or represent di-(C₁-C₄-alkyl)-aminosulphonyl;
furthermore
R³ represents the radical 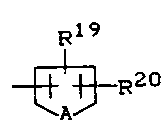 where
R¹⁹ and R²⁰ are identical or different and represent hydrogen, fluorine, chlorine, bromine, cyano, nitro, C₁-C₄-alkyl (which is optionally substituted by fluorine and/or chlorine), C₁-C₄-alkoxy (which is optionally substituted by fluorine and/or chlorine), C₁-C₄-alkylthio, C₁-C₄-alkylsulphinyl or C₁-C₄-alkylsulphonyl (which is optionally substituted by fluorine and/or chlorine), di- (C₁-C₄-alkyl)-amino-sulphonyl, C₁-C₄-alkoxy-carbonyl or dimethylaminocarbonyl, and
A represents oxygen, sulphur or the group N-Z¹, where
Z¹ represents hydrogen, C₁-C₄-alkyl (which is optionally substituted by fluorine, chlorine, bromine or cyano), C₃-C₆-cycloalkyl, benzyl, phenyl (which is optionally substituted by fluorine, chlorine, bromine or nitro), C₁-C₄-alkylcarbonyl, C₁-C₄-alkoxy-carbonyl or di-(C₁-C₄-alkyl)-aminocarbonyl;
furthermore
R³ represents the radical 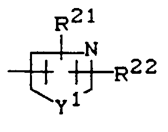 where
R²¹ and R²² are identical or different and represent hydrogen, C₁-C₄-alkyl, halogen, C₁-C₄-alkoxycarbonyl, C₁-C₄-alkoxy or C₁-C₄-halogenoalkoxy,
Y¹ represents sulphur or the group N-R²³, where
R²³ represents hydrogen or C₁-C₄-alkyl;
furthermore
R³ represents the radical 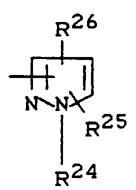 where
R²⁴ represents hydrogen, C₁-C₄-alkyl, benzyl, pyridyl, quinolinyl or phenyl,
R²⁵ represents hydrogen, halogen, cyano, nitro, C₁-C₄-alkyl (which is optionally substituted by fluorine and/or chlorine), C₁-C₄-alkoxy (which is optionally substituted by fluorine and/or chlorine), dioxolanyl or C₁-C₄-alkoxy-carbonyl and
R²⁶ represents hydrogen, halogen or C₁-C₄-alkyl;
furthermore
R³ represents one of the groups listed below 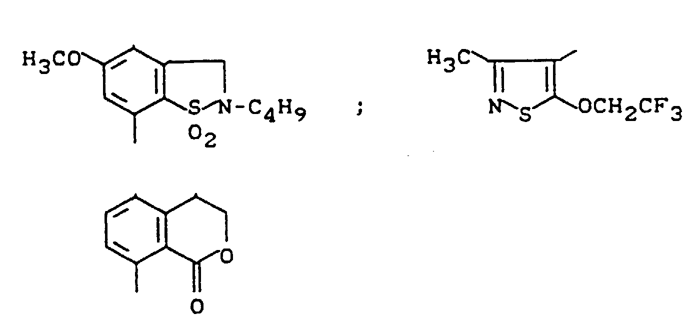
and salts of compounds of the formula (I), with the exception of the compound
2-(2-methoxycarbonyl-phenylsulphonylaminocarbonyl)-4-methyl-5-methoxy-2,4-dihydro-3H-1,2,4-triazol-3-one,
2-(2-trifluoromethoxy-phenylsulphonylaminocarbonyl)-4-cyclopropyl-5-methoxy-2,4-dihydro-3H-1,2,4-triazol-3-one and
2-(difluoromethoxy-phenylsulphonylaminocarbonyl)-4-cyclobutyl-5-ethoxy-2,4-dihydro-3H-1,2,4-triazol-3-one.

2. Sulphonylaminocarbonyltriazolinones of the formula (I) according to Claim 1, characterized in that,
R¹ represents hydrogen, amino, C₁-C₄-alkyl which is optionally substituted by fluorine, cyano, methoxy or ethoxy, or represents allyl, C₃-C₆-cycloalkyl, benzyl, phenyl, C₁-C₃-alkylamino, C₃-C₆-cycloalkylamino or di-(C₁-C₃-alkyl)-amino,
R² represents hydrogen, C₁-C₄-alkyl which is optionally substituted by fluorine and/or chlorine, methoxy or ethoxy, or represents C₃-C₄-alkenyl which is optionally substituted by fluorine and/or chlorine, or represents C₃-C₆-cycloalkyl, or represents benzyl which is optionally substituted by fluorine, chlorine and/or methyl, and
R³ represents the group 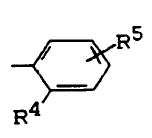 where
R⁴ represents fluorine, chlorine, bromine, methyl, trifluoromethyl, methoxy, difluoromethoxy, trifluoromethoxy, 2-chloroethoxy, 2-methoxy-ethoxy, C₁-C₃-alkylthio, C₁-C₃-alkylsulphinyl, C₁-C₃-alkylsulphonyl, dimethylaminosulphonyl, diethylaminosulphonyl, N-methoxy-N-methylaminosulphonyl, methoxyaminosulphonyl, phenyl,phenoxy or C₁-C₃-alkoxy-carbonyl and
R⁵ represents hydrogen, fluorine, chlorine or bromine;
furthermore
R³ represents the radical 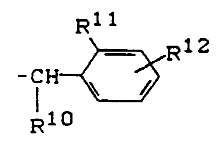 where
R¹⁰ represents hydrogen,
R¹¹ represents fluorine, chlorine, bromine, methyl, methoxy, difluoromethoxy, trifluoromethoxy, ethoxy, methoxycarbonyl, ethoxycarbonyl, methylsulphonyl or dimethylaminosulphonyl and
R¹² represents hydrogen;
furthermore
R³ represents the radical 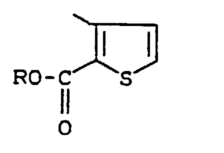 where R represents C₁-C₄-alkyl, or
represents the radical 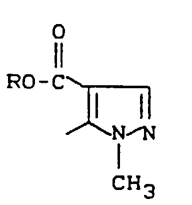 where R represents C₁-C₄-alkyl,
and salts of compounds of the formula (I),
with the exclusion of the same three compounds as in Claim 1.

3. The sodium, potassium, magnesium, calcium, ammonium, C₁-C₄-alkyl-ammonium, di-(C₁-C₄-alkyl) -ammonium, tri-(C₁-C₄-alkyl)-ammonium, C₅- or C₆-cycloalkyl-ammonium and di- (C₁-C₂-alkyl)-benzylammonium salts of compounds of the formula (I) according to Claim 1.

4. Process for the preparation of sulphonylaminotriazolinones of the general formula (I) 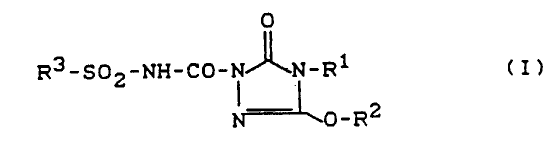 in which
R¹ represents hydrogen, amino, or represents C₁-C₆-alkyl which is optionally substituted by fluorine, chlorine, bromine, cyano, C₁-C₄-alkoxy, C₁-C₄-alkylcarbonyl or C₁-C₄-alkoxycarbonyl, or represents C₃-C₆-alkenyl or C₃-C₆-alkinyl, each of which is optionally substituted by fluorine, chlorine and/or bromine, or represents C₃-C₆-cycloalkyl which is optionally substituted by fluorine, chlorine, bromine and/or C₁-C₄-alkyl, or represents phenyl-C₁-C₃-alkyl which is optionally substituted by fluorine, chlorine, bromine, cyano, nitro, C₁-C₄-alkyl, trifluoromethyl, C₁-C₄-alkoxy and/or C₁-C₄-alkoxy-carbonyl, or represents phenyl which is optionally substituted by fluorine, chlorine, bromine, cyano, nitro, C₁-C₄-alkyl, trifluoromethyl, C₁-C₄-alkoxy, fluorine- and/or chlorine-substituted C₁-C₃-alkoxy, C₁-C₄-alkylthio, fluorine- and/or chlorine-substituted C₁-C₃-alkylthio, C₁-C₄-alkylsulphinyl, C₁-C₄-alkylsulphonyl and/or C₁-C₄-alkoxy-carbonyl, or represents C₁-C₄-alkylamino which is optionally substituted by fluorine, cyano, C₁-C₄-alkoxy or C₁-C₄-alkoxy-carbonyl, or represents C₃-C₆-cycloalkylamino or di-(C₁-C₄-alkyl)-amino,
R² represents C₁-C₆-alkyl which is optionally substituted by fluorine, chlorine, bromine, cyano, C₃-C₆-cycloalkyl, C₁-C₄-alkoxy or C₁-C₄-alkoxycarbonyl, or represents C₃-C₆-alkenyl or C₃-C₆-alkinyl, each of which is optionally substituted by fluorine, chlorine and/or bromine, or represents C₃-C₆-cycloalkyl which is optionally substituted by fluorine, chlorine, bromine and/or C₁-C₄-alkyl, or represents cyclohexenyl, or represents phenyl-C₁-C₃-alkyl which is optionally substituted by fluorine, chlorine, bromine, cyano, nitro, C₁-C₄-alkyl, trifluoromethyl, C₁-C₄-alkoxy and/or C₁-C₄-alkoxy-carbonyl, or represents phenyl which is optionally substituted by fluorine, chlorine, bromine, cyano, nitro, C₁-C₄-alkyl, trifluoromethyl, C₁-C₄-alkoxy, fluorine- and/or chlorine-substituted C₁-C₃-alkoxy, C₁-C₄-alkylthio, fluorine- and/or chlorine-substituted C₁-C₃-alkylthio, C₁-C₄-alkyl-sulphinyl, C₁-C₄-alkylsulphonyl and/or C₁-C₄-alkoxy-carbonyl, and
R³ represents the group 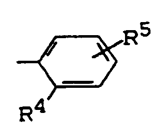 where
R⁴ and R⁵ are identical or different and represent hydrogen, fluorine, chlorine, bromine, iodine, nitro, C₁-C₆-alkyl (which is optionally substituted by fluorine, chlorine, bromine, cyano, carboxyl, C₁-C₄-alkoxycarbonyl, C₁-C₄-alkylamino-carbonyl, di-(C₁-C₄-alkyl)amino-carbonyl, hydroxyl, C₁-C₄-alkoxy, formyloxy, C₁-C₄-alkyl-carbonyloxy, C₁-C₄-alkoxy-carbonyloxy, C₁-C₄-alkylamino-carbonyloxy, C₁-C₄-alkylthio, C₁-C₄-alkylsulphinyl, C₁-C₄-alkylsulphonyl,di-(C₁-C₄-alkyl)-aminosulphonyl, C₃-C₆-cycloalkyl or phenyl), or represent C₂-C₆-alkenyl (which is optionally substituted by fluorine, chlorine, bromine, cyano, C₁-C₄-alkoxy-carbonyl, carboxyl or phenyl), or represent C₂-C₆-alkinyl (which is optionally substituted by fluorine, chlorine, bromine, cyano, C₁-C₄-alkoxy-carbonyl, carboxyl or phenyl), or represent C₁-C₄-alkoxy (which is optionally substituted by fluorine, chlorine, bromine, cyano, carboxyl, C₁-C₄-alkoxy-carbonyl, C₁-C₄-alkoxy, C₁-C₄-alkylthio, C₁-C₄-alkylsulphinyl or C₁-C₄-alkylsulphonyl), or represent C₁-C₄-alkylthio (which is optionally substituted by fluorine, chlorine, bromine, cyano, carboxyl, C₁-C₄-alkoxy-carbonyl, C₁-C₄-alkylthio, C₁-C₄-alkylsulphinyl or C₁-C₄-alkylsulphonyl), or represent C₃-C₆-alkenyloxy (which is optionally substituted by fluorine, chlorine, bromine, cyano or C₁-C₄-alkoxy-carbonyl), or represent C₂-C₆-alkenylthio (which is optionally substituted by fluorine, chlorine, bromine, cyano, nitro, C₁-C₃-alkylthio or C₁-C₄-alkoxycarbonyl), C₃-C₆-alkinyloxy, C₃-C₆-alkinylthio or the radical -S(O)ₚ-R⁶ where
p represents the numbers 1 or 2 and
R⁶ represents C₁-C₄-alkyl (which is optionally substituted by fluorine, chlorine, bromine, cyano or C₁-C₄-alkoxy-carbonyl), C₃-C₆-alkenyl, C₃-C₆-alkinyl, C₁-C₄-alkyl, C₁-C₄-alkoxy-C₁-C₄-alkylamino, C₁-C₄-alkylamino, di-(C₁-C₄-alkyl)-amino, phenyl or the radical -NHOR⁷ where
R⁷ represents C₁-C₁₂-alkyl (which is optionally substituted by fluorine, chlorine, cyano, C₁-C₄-alkoxy, C₁-C₄-alkylthio, C₁-C₄-alkylsulphinyl, C₁-C₄-alkylsulphonyl, C₁-C₄-alkyl-carbonyl, C₁-C₄-alkoxy-carbonyl, C₁-C₄-alkylamino-carbonyl or di-(C₁-C₄-alkyl)-amino-carbonyl), or represents C₃-C₆-alkenyl (which is optionally substituted by fluorine, chlorine or bromine), C₃-C₆-alkinyl, C₃-C₆-cycloalkyl, C₃-C₆-cycloalkyl-C₁-C₂-alkyl, phenyl-C₁-C₂-alkyl (which is optionally substituted by fluorine, chlorine, nitro, cyano, C₁-C₄-alkyl, C₁-C₄-alkoxy or C₁-C₄-alkoxy-carbonyl), or represents benzhydryl, or represents phenyl (which is optionally substituted by fluorine, chlorine, nitro, cyano, C₁-C₄-alkyl, trifluoromethyl, C₁-C₄-alkoxy, C₁-C₂-fluoroalkoxy, C₁-C₄-alkylthio, trifluoromethylthio or C₁-C₄-alkoxy-carbonyl),
R⁴ and/or R⁵ furthermore represent phenyl or phenoxy, or represent C₁-C₄-alkylcarbonylamino, C₁-C₄-alkoxycarbonylamino, C₁-C₄-alkylamino-carbonyl-amino, di-(C₁-C₄-alkyl)-amino-carbonylamino, or the radical -CO-R⁸ where
R⁸ represents C₁-C₆-alkyl, C₁-C₆-alkoxy, C₃-C₆-cycloalkoxy, C₃-C₆-alkenyloxy, C₁-C₄-alkylthio, C₁-C₄-alkylamino, C₁-C₄-alkoxyamino, C₁-C₄-alkoxy-C₁-C₄-alkyl-amino or di-(C₁-C₄-alkyl)-amino (which are optionally substituted by fluorine and/or chlorine),
R⁴ and/or R⁵ furthermore represent trimethylsilyl, thiazolinyl, C₁-C₄-alkylsulphonyloxy, di-(C₁-C₄-alkyl)-aminosulphonylamino or the radical -CH=N-R⁹ where
R⁹ represents C₁-C₆-alkyl which is optionally substituted by fluorine, chlorine, cyano, carboxyl, C₁-C₄-alkoxy, C₁-C₄-alkylthio, C₁-C₄-alkylsulphinyl or C₁-C₄-alkylsulphonyl, or represents benzyl which is optionally substituted by fluorine or chlorine, or represents C₃-C₆-alkenyl or C₃-C₆-alkinyl, each of which is optionally substituted by fluorine or chlorine, or represents phenyl which is optionally substituted by fluorine, chlorine, bromine, C₁-C₄-alkyl, C₁-C₄-alkoxy, trifluoromethyl, trifluoromethoxy or trifluoromethylthio, or represents optionally fluorine- and/or chlorine-substituted C₁-C₆-alkoxy, C₃-C₆-alkenoxy, C₃-C₆-alkinoxy or benzyloxy, or represents amino, C₁-C₄-alkylamino, di-(C₁-C₄-alkyl)-amino, phenyl-amino, C₁-C₄-alkyl-carbonyl-amino, C₁-C₄-alkoxy-carbonylamino or C₁-C₄-alkyl-sulphonylamino, or represents phenylsulphonylamino which is optionally substituted by fluorine, chlorine, bromine or methyl,
furthermore
R³ represents the radical 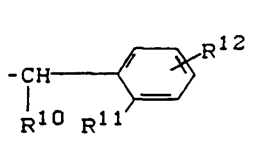 where
R¹⁰ represents hydrogen or C₁-C₄-alkyl,
R¹¹ and R¹² are identical or different and represent hydrogen, fluorine, chlorine, bromine, nitro, cyano, C₁-C₄-alkyl (which is optionally substituted by fluorine and/or chlorine), C₁-C₄-alkoxy (which is optionally substituted by fluorine and/or chlorine), carboxyl, C₁-C₄-alkoxy-carbonyl, dimethylaminocarbonyl, C₁-C₄-alkylsulphonyl or di-(C₁-C₄-alkyl)-aminosulphonyl;
furthermore
R³ represents the radical 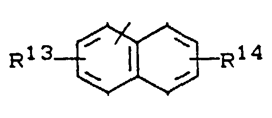 where
R¹³ and R¹⁴ are identical or different and represent hydrogen, fluorine, chlorine, bromine, nitro, cyano, C₁-C₄-alkyl (which is optionally substituted by fluorine and/or chlorine) or C₁-C₄-alkoxy (which is optionally substituted by fluorine and/or chlorine);
furthermore
R³ represents the radical 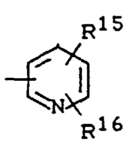 where
R¹⁵ and R¹⁶ are identical or different and represent hydrogen, fluorine, chlorine, bromine, nitro, cyano, C₁-C₄-alkyl (which is optionally substituted by fluorine and/or chlorine), C₁-C₄-alkoxy (which is optionally substituted by fluorine and/or chlorine), or represent C₁-C₄-alkylthio, C₁-C₄-alkylsulphinyl or C₁-C₄-alkylsulphonyl (which are optionally substituted by fluorine and/or chlorine), or represent aminosulphonyl, mono-(C₁-C₄-alkyl)-aminosulphonyl, di-(C₁-C₄-alkyl)-aminosulphonyl or C₁-C₄-alkoxycarbonyl or dimethylaminocarbonyl;
furthermore
R³ represents the radical 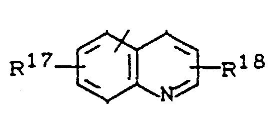 where
R¹⁷ and R¹⁸ are identical or different and represent hydrogen, fluorine, chlorine, bromine, C₁-C₄-alkyl (which is optionally substituted by fluorine and/or bromine), C₁-C₄-alkoxy (which is optionally substituted by fluorine and/or chlorine), or represent C₁-C₄-alkylthio, C₁-C₄-alkylsulphinyl or C₁-C₄-alkylsulphonyl (which are optionally substituted by fluorine and/or chlorine), or represent di-(C₁-C₄-alkyl)-amino-sulphonyl;
furthermore
R³ represents the radical 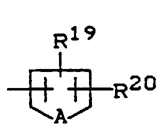 where
R¹⁹ and R²⁰ are identical or different and represent hydrogen, fluorine, chlorine, bromine, cyano, nitro, C₁-C₄-alkyl (which is optionally substituted by fluorine and/or chlorine), C₁-C₄-alkoxy (which is optionally substituted by fluorine and/or chlorine), C₁-C₄-alkylthio, C₁-C₄-alkylsulphinyl or C₁-C₄-alkylsulphonyl (which is optionally substituted by fluorine and/or chlorine), di-(C₁-C₄-alkyl)-amino-sulphonyl, C₁-C₄-alkoxy-carbonyl or dimethylaminocarbonyl, and
A represents oxygen, sulphur or the group N-Z¹, where
Z¹ represents hydrogen, C₁-C₄-alkyl (which is optionally substituted by fluorine, chlorine, bromine or cyano), C₃-C₆-cycloalkyl, benzyl, phenyl (which is optionally substituted by fluorine, chlorine, bromine or nitro), C₁-C₄-alkyl-carbonyl, C₁-C₄-alkoxy-carbonyl or di-(C₁-C₄-alkyl)-aminocarbonyl;
furthermore
R³ represents the radical 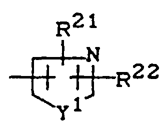 where
R²¹ and R²² are identical or different and represent hydrogen, C₁-C₄-alkyl, halogen, C₁-C₄-alkoxycarbonyl, C₁-C₄-alkoxy or C₁-C₄-halogenoalkoxy,
Y¹ represents sulphur or the group N-R²³, where
R²³ represents hydrogen or C₁-C₄-alkyl;
furthermore
R³ represents the radical 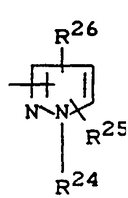 where
R²⁴ represents hydrogen, C₁-C₄-alkyl, benzyl, pyridyl, quinolinyl or phenyl,
R²⁵ represents hydrogen, halogen, cyano, nitro, C₁-C₄-alkyl (which is optionally substituted by fluorine and/or chlorine), C₁-C₄-alkoxy (which is optionally substituted by fluorine and/or chlorine), dioxolanyl or C₁-C₄-alkoxy-carbonyl and
R²⁶ represents hydrogen, halogen or C₁-C₄-alkyl;
furthermore
R³ represents one of the groups listed below 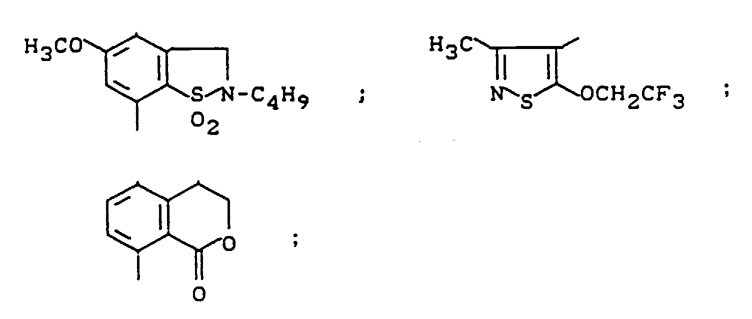
and salts of the compounds of the formula (I), but with the exception of the compounds
2-(2-methoxycarbonyl-phenylsulphonylaminocarbonyl)-4-methyl-5-methoxy-2,4-dihydro-3H-1,2,4-triazol-3-one,
2(2-trifluoromethoxy-phenylsulphonylaminocarbonyl)-4-cyclopropyl-5-methoxy-2,4-dihydro-3H-1,2,4-triazol-3-one and
2-(difluoromethoxy-phenylsulphonylaminocarbonyl)-4-cyclobutyl-5-ethoxy-2,4-dihydro-3H-1,2,4-triazol-3-one,
characterized in that
(a) triazolinones of the general formula (II) 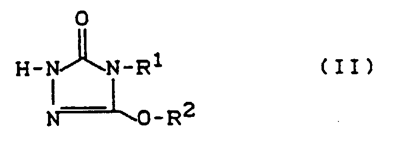 in which
R¹ and R² have the meanings given above for formula (I)
are reacted with sulphonylisocyanates of the general formula (III)
R³-SO₂-N=C=O (III)
in which
R³ has the meaning given above for formula (I),
if appropriate in the presence of a diluent, or in that
(b) triazolinone derivatives of the general formula (IV) 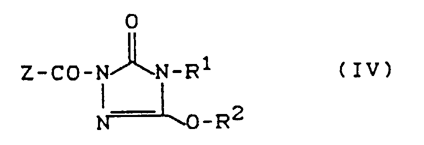 in which
R¹ and R² have the meanings given for formula (I) and
Z represents chlorine, C₁-C₄-alkoxy, benzyloxy or phenoxy,
are reacted with sulphonamides of the general formula (V)
R³-SO₂-NH₂ (V)
in which
R³ has the meaning given for formula (I),
if appropriate in the presence of an acid acceptor and if appropriate in the presence of a diluent, or in that
(c) triazolinones of the general formula (II) 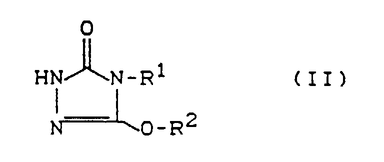 in which
R¹ and R² have the meanings given for formula (I),
are reacted with sulphonamide derivatives of the general formula (VI)
R³-SO₂-NH-CO-Z (VI)
in which
R³ has the meaning given for formula (I) and
Z represents chlorine, C₁-C₄-alkoxy, benzyloxy or phenoxy,
if appropriate in the presence of an acid acceptor and if appropriate in the presence of a diluent,
and, if appropriate, salts are prepared by customary methods from the compounds of the formula (I) prepared as described for process (a), (b) or (c).

5. Herbicidal compositions, characterized in that they comprise at least one sulphonylaminocarbonyltriazolinone of the formula (I) according to Claim 1 or 4.

6. Use of sulphonylaminocarbonyltriazolinones of the general formula (I) according to Claim 1 or 4 for controlling undesired plant growth.

7. Method of controlling weeds, characterized in that sulphonylaminocarbonyltriazolinones of the general formula (I) according to Claim 1 or 4 are allowed to act on weeds or their environment.

8. Process for the preparation of herbicidal compositions, characterised in that sulphonylaminocarbonyltriazolinones of the general formula (I) according to Claim 1 or 4 are mixed with extenders and/or surfactants.

9. Triazolinones of the general formula (II) 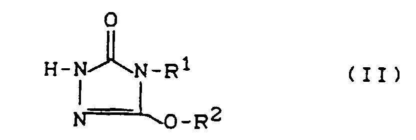 in which
R¹ represents amino, or represents C₁-C₆-alkyl which is optionally substituted by fluorine, chlorine, bromine, cyano, C₁-C₄-alkoxy, C₁-C₄-alkylcarbonyl or C₁-C₄-alkoxy -carbonyl, or represents C₃-C₆-alkenyl or C₃-C₆-alkinyl, each of which is optionally substituted by fluorine, chlorine and/or bromine, or represents C₃-C₆-cycloalkyl which is optionally substituted by fluorine, chlorine, bromine and/or C₁-C₄-alkyl, or represents phenyl-C₁-C₃-alkyl which is optionally substituted by fluorine, chlorine, bromine, cyano, nitro, C₁-C₄-alkyl, trifluoromethyl, C₁-C₄-alkoxy and/or C₁-C₄-alkoxy-carbonyl, or represents phenyl which is optionally substituted by fluorine, chlorine, bromine, cyano, nitro, C₁-C₄-alkyl, trifluoromethyl, C₁-C₄-alkoxy, fluorine- and/or chlorine-substituted C₁-C₃-alkoxy, C₁-C₄-alkylthio, fluorine- and/or chlorine-substituted C₁-C₃-alkylthio, C₁-C₄-alkylsulphinyl, C₁-C₄-alkylsulphonyl and/or C₁-C₄-alkoxy-carbonyl, or represents C₁-C₄-alkylamino which is optionally substituted by fluorine, cyano, C₁-C₄-alkoxy or C₁-C₄-alkoxy-carbonyl, or represents C₃-C₆-cycloalkylamino or di-(C₁-C₄-alkyl)-amino,
R² represents C₃-C₆-cycloalkyl which is optionally substituted by fluorine, chlorine, bromine and/or C₁-C₄-alkyl, or represents cyclohexenyl, or represents phenyl-C₁-C₃-alkyl which is optionally substituted by fluorine, chlorine, bromine, cyano, nitro, C₁-C₄-alkyl, trifluoromethyl, C₁-C₄-alkoxy and/or C₁-C₄-alkoxy-carbonyl, or represents phenyl which is optionally substituted by fluorine, chlorine, bromine, cyano, nitro, C₁-C₄-alkyl, trifluoromethyl, C₁-C₄-alkoxy, fluorine- and/or chlorine-substituted C₁-C₃-alkoxy, C₁-C₄-alkylthio, fluorine- and/or chlorine-substituted C₁-C₃-alkylthio, C₁-C₄-alkyl-sulphinyl, C₁-C₄-alkyl-sulphonyl and/or C₁-C₄-alkoxy-carbonyl.

10. Process for the preparation of triazolinones of the general formula (II) 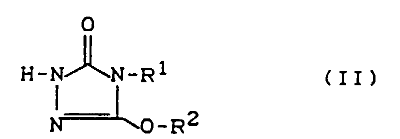 in which
R¹ represents amino, or represents C₁-C₆-alkyl which is optionally substituted by fluorine, chlorine, bromine, cyano, C₁-C₄-alkoxy, C₁-C₄-alkylcarbonyl or C₁-C₄-alkoxy-carbonyl, or represents C₃-C₆-alkenyl or C₃-C₆-alkinyl, each of which is optionally substituted by fluorine, chlorine and/or bromine, or represents C₃-C₆-cycloalkyl which is optionally substituted by fluorine, chlorine, bromine and/or C₁-C₄-alkyl, or represents phenyl-C₁-C₃-alkyl which is optionally substituted by fluorine, chlorine, bromine, cyano, nitro, C₁-C₄-alkyl, trifluoromethyl, C₁-C₄-alkoxy and/or C₁-C₄-alkoxy-carbonyl, or represents phenyl which is optionally substituted by fluorine, chlorine, bromine, cyano, nitro, C₁-C₄-alkyl, trifluoromethyl, C₁-C₄-alkoxy, fluorine- and/or chlorine-substituted C₁-C₃-alkoxy, C₁-C₄-alkylthio, fluorine- and/or chlorine-substituted C₁-C₃-alkylthio, C₁-C₄-alkylsulphinyl, C₁-C₄-alkylsulphonyl and/or C₁-C₄-alkoxy-carbonyl, or represents C₁-C₄-alkylamino which is optionally substituted by fluorine, cyano, C₁-C₄-alkoxy or C₁-C₄-alkoxy-carbonyl, or represents C₃-C₆-cycloalkylamino or di-(C₁-C₄-alkyl)-amino, and
R² represents C₃-C₆-cycloalkyl which is optionally substituted by fluorine, chlorine, bromine and/or C₁-C₄-alkyl, or represents cyclohexenyl, or represents phenyl-C₁-C₃-alkyl which is optionally substituted by fluorine, chlorine, bromine, cyano, nitro, C₁-C₄-alkyl, trifluoromethyl, C₁-C₄-alkoxy and/or C₁-C₄-alkoxy-carbonyl, or represents phenyl which is optionally substituted by fluorine, chlorine, bromine, cyano, nitro, C₁-C₄-alkyl, trifluoromethyl, C₁-C₄-alkoxy, fluorine- and/or chlorine-substituted C₁-C₃-alkoxy, C₁-C₄-alkylthio, fluorine- and/or chlorine-substituted C₁-C₃-alkylthio, C₁-C₄-alkyl-sulphinyl, C₁-C₄-alkyl-sulphonyl and/or C₁-C₄-alkoxy-carbonyl,
characterized in that hydrazinoformic esters of the general formula (VII)
H₂N-NH-CO-O-R²⁷ (VII)
in which
R²⁷ represents methyl, ethyl or phenyl
are reacted with alkyliminocarbonic diesters of the general formula (VIII) 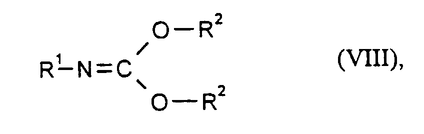 in which
R¹ and R² have the abovementioned meaning,
if appropriate in the presence of a diluent, at temperatures between 0°C and 50°C, if appropriate isolating the resulting compounds of the general formula (IX) 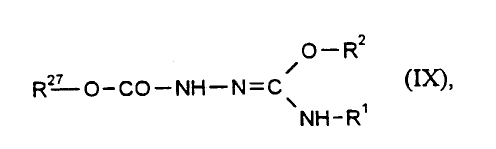 in which
R¹, R² and R²⁷ have the abovementioned meaning
by customary methods and heating them to temperatures between 50°C and 150°C, if appropriate in the presence of a diluent.

11. Compounds of the general formula (IX) 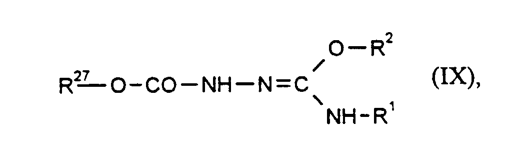 in which
R¹ and R² have the meanings given in Claim 9 and
R²⁷ represents methyl, ethyl or phenyl.

12. Process according to Claim 10, characterized in that the hydrazinoformic ester of the formula (VII) employed is phenyl hydrazinoformate and the alkyliminocarbonic diester of the formula (VIII) employed is trimethyl iminocarbonate, giving 4-methyl-5-methoxy-2,4-dihydro-3H-1,2,4-triazol-3-one (II-2).

## Revendications

1. Sulfonylaminocarbonyltriazolinones portant des substituants liés par de l'oxygène, de formule générale (I) 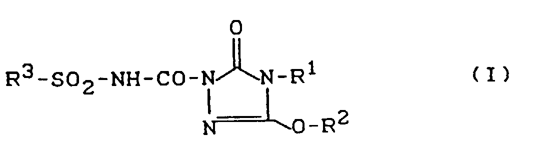 dans laquelle
R¹ représente de l'hydrogène, un groupe amino, un groupe alkyle en C₁ à C₆ éventuellement substitué par du fluor, du chlore, du brome, un groupe cyano, alkoxy en C₁ à C₄, (alkyle en C₁ à C₄)-carbonyle ou (alkoxy en C₁ à C₄)-carbonyle, un groupe alcényle en C₃ à C₆ ou un groupe alcynyle en C₃ à C₆ dont chacun est éventuellement substitué par du fluor, du chlore et/ou du brome, un groupe cycloalkyle en C₃ à C₆ éventuellement substitué par du fluor, du chlore, du brome et/ou un reste alkyle en C₁ à C₄, un groupe phényl-(alkyle en C₁ à C₃) éventuellement substitué par du fluor, du chlore, du brome, un reste cyano, nitro, alkyle en C₁ à C₄, trifluorométhyle, alkoxy en C₁ à C₄ et/ou (alkoxy en C₁ à C₄)-carbonyle, un groupe phényle éventuellement substitué par du fluor, du chlore, du brome, un reste cyano, nitro, alkyle en C₁ à C₄, trifluorométhyle, alkoxy en C₁ à C₄, alkoxy en C₁ à C₃ substitué par du fluor et/ou du chlore, alkylthio en C₁ à C₄, alkylthio en C₁ à C₃ substitué par du fluor et/ou du chlore, alkylsulfinyle en C₁ à C₄, alkylsulfonyle en C₁ à C₄ et/ou (alkoxy en C₁ à C₄)-carbonyle, un groupe alkylamino en C₁ à C₄ éventuellement substitué par du fluor, un reste cyano, alkoxy en C₁ à C₄ ou (alkoxy en C₁ à C₄)-carbonyle, un groupe cycloalkylamino en C₃ à C₆ ou un groupe di(alkyle en C₁ à C₄)-amino,
R² représente un groupe alkyle en C₁ à C₆ éventuellement substitué par du fluor, du chlore, du brome, un reste cyano, cycloalkyle en C₃ à C₆, alkoxy en C₁ à C₄ ou (alkoxy en C₁ à C₄)-carbonyle, un groupe alcényle en C₃ à C₆ ou un groupe alcynyle en C₃ à C₆ dont chacun est éventuellement substitué par du fluor, du chlore et/ou du brome, un groupe cycloalkyle en C₃ à C₆ éventuellement substitué par du fluor, du chlore, du brome et/ou un reste alkyle en C₁ à C₄, un groupe cyclohexényle, un groupe phényl-(alkyle en C₁ à C₃) éventuellement substitué par du fluor, du chlore, du brome, un reste cyano, nitro, alkyle en C₁ à C₄, trifluorométhyle, alkoxy en C₁ à C₄ et/ou (alkoxy en C₁ à C₄)-carbonyle, ou un groupe phényle éventuellement substitué par du fluor, du chlore, du brome, un reste cyano, nitro, alkyle en C₁ à C₄, trifluorométhyle, alkoxy en C₁ à C₄, alkoxy en C₁ à C₃ substitué par du fluor et/ou du chlore, alkylthio en C₁ à C₄, alkylthio en C₁ à C₃ substitué par du fluor et/ou du chlore, alkylsulfinyle en C₁ à C₄, alkylsulfonyle en C₁ à C₄ et/ou (alkoxy en C₁ à C₄)-carbonyle et
R³ représente le groupement 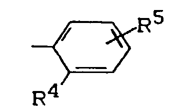 dans lequel
R⁴ et R⁵ sont identiques ou différents et représentent l'hydrogène, du fluor, du chlore, du brome, de l'iode, un groupe nitro, alkyle en C₁ à C₆ (qui est éventuellement substitué par du fluor, du chlore, du brome, un reste cyano, carboxy, (alkoxy en C₁ à C₄)-carbonyle, (alkyle en C₁ à C₄)-aminocarbonyle, di(alkyle en C₁ à C₄)-aminocarbonyle, hydroxy, alkoxy en C₁ à C₄, formyloxy, (alkyle en C₁ à C₄)-carbonyloxy, (alkoxy en C₁ à C₄)-carbonyloxy, (alkyle en C₁ à C₄)-amino-carbonyloxy, alkylthio en C₁ à C₄, alkylsulfinyle en C₁ à C₄, alkylsulfonyle en C₁ à C₄, di(alkyle en C₁ à C₄)-aminosulfonyle, cycloalkyle en C₃ à C₆ ou phényle), un groupe alcényle en C₂ à C₆ (qui est éventuellement substitué par du fluor, du chlore, du brome, un reste cyano, (alkoxy en C₁ à C₄)-carbonyle, carboxy ou phényle), un groupe alcynyle en C₂ à C₆ (qui est éventuellement substitué par du fluor, du chlore, du brome, un reste cyano, (alkoxy en C₁ à C₄)-carbonyle, carboxy ou phényle), un groupe alkoxy en C₁ à C₄ (qui est éventuellement substitué par du fluor, du chlore, du brome, un reste cyano, carboxy, (alkoxy en C₁ à C₄)-carbonyle, alkoxy en C₁ à C₄, alkylthio en C₁ à C₄, alkylsulfinyle en C₁ à C₄ ou alkylsulfonyle en C₁ à C₄), un groupe alkylthio en C₁ à C₄ (qui est éventuellement substitué par du fluor, du chlore, du brome, un reste cyano, carboxy, (alkoxy en C₁ à C₄)-carbonyle, alkylthio en C₁ à C₄, alkylsulfinyle en C₁ à C₄ ou alkylsulfonyle en C₁ à C₄), un groupe alcényloxy en C₃ à C₆ (qui est éventuellement substitué par du fluor, du chlore, du brome, un reste cyano ou (alkoxy en C₁ à C₄)-carbonyle), un groupe alcénylthio en C₂ à C₆ (qui est éventuellement substitué par du fluor, du chlore, du brome, un reste cyano, nitro, alkylthio en C₁ à C₃ ou (alkoxy en C₁ à C₄)-carbonyle), un groupe alcynyloxy en C₃ à C₆, alcynylthio en C₃ à C₆ ou le reste -S(O)ₚ-R⁶ dans lequel
p représente le nombre 1 ou 2 et
R⁶ est un groupe alkyle en C₁ à C₄ (qui est éventuellement substitué par du fluor, du chlore, du brome, un reste cyano ou (alkoxy en C₁ à C₄)-carbonyle), un groupe alcényle en C₃ à C₆, alcynyle en C₃ à C₆, alkoxy en C₁ à C₄, (alkoxy en C₁ à C₄)-(alkylamino en C₁ à C₄), alkylamino en C₁ à C₄, di-(alkyle en C₁ à C₄)-amino, phényle ou le reste -NHOR⁷, dans lequel
R⁷ est un groupe alkyle en C₁ à C₁₂ (qui est éventuellement substitué par du fluor, du chlore, un reste cyano, alkoxy en C₁ à C₄, alkylthio en C₁ à C₄, alkylsulfinyle en C₁ à C₄, alkylsulfonyle en C₁ à C₄, (alkyle en C₁ à C₄)-carbonyle, (alkoxy en C₁ à C₄)-carbonyle, (alkylamino en C₁ à C₄)-carbonyle ou di-(alkyle en C₁ à C₄)-amino-carbonyle), un groupe alcényle en C₃ à C₆ (qui est éventuellement substitué par du fluor, du chlore ou du brome), un groupe alcynyle en C₃ à C₆, cycloalkyle en C₃ à C₆, (cycloalkyle en C₃ à C₆)-(alkyle en C₁ ou C₂), phényl-(alkyle en C₁ ou C₂) (qui est éventuellement substitué par du fluor, du chlore, un reste nitro, cyano, alkyle en C₁ à C₄, alkoxy en C₁ à C₄ ou (alkoxy en C₁ à C₄)-carbonyle), un groupe benzhydryle ou un groupe phényle (qui est éventuellement substitué par du fluor, du chlore, un reste nitro, cyano, alkyle en C₁ à C₄, trifluorométhyle, alkoxy en C₁ à C₄, fluoralkoxy en C₁ ou C₂, alkylthio en C₁ à C₄, trifluorométhylthio ou (alkoxy en C₁ à C₄)-carbonyle),
R⁴ et/ou R⁵ représentent en outre un groupe phényle ou phénoxy, un groupe (alkyle en C₁ à C₄)-carbonylamino, (alkoxy en C₁ à C₄)-carbonylamino, (alkylamino en C₁ à C₄)-carbonyl-amino, di-(alkyle en C₁ à C₄)-amino-carbonylamino ou le reste -CO-R⁸, dans lequel
R⁸ est un groupe alkyle en C₁ à C₆, alkoxy en C₁ à C₆, cycloalkoxy en C₃ à C₆, alcényloxy en C₃ à C₆, alkylthio en C₁ à C₄, alkylamino en C₁ à C₄, alkoxyamino en C₁ à C₄, (alkoxy en C₁ à C₄)-(alkyl-amino en C₁ à C₄) ou di-(alkyle en C₁ à C₄)-amino (qui sont éventuellement substitués par du fluor et/ou du chlore),
R⁴ et/ou R⁵ représentent en outre un groupe triméthylsilyle, thiazolinyle, alkyl-sulfonyloxy en C₁ à C₄, di-(alkyle en C₁ à C₄)-aminosulfonylamino ou le reste -CH=N-R⁹, dans lequel
R⁹ est un groupe alkyle en C₁ à C₆ éventuellement substitué par du fluor, du chlore, un reste cyano, carboxy, alkoxy en C₁ à C₄, alkylthio en C₁ à C₄, alkylsulfinyle en C₁ à C₄ ou alkylsulfonyle en C₁ à C₄, un groupe benzyle éventuellement substitué par du fluor ou du chlore, un groupe alcényle en C₃ à C₆ ou un groupe alcynyle en C₃ à C₆ éventuellement substitué par du fluor ou du chlore, un groupe phényle éventuellement substitué par du fluor, du chlore, du brome, un reste alkyle en C₁ à C₄, alkoxy en C₁ à C₄, trifluorométhyle, trifluorométhoxy ou trifluorométhylthio, un groupe alkoxy en C₁ à C₆ éventuellement substitué par du fluor et/ou du chlore, un groupe alcénoxy en C₃ à C₆, alcynoxy en C₃ à C₆ ou benzyloxy, un groupe amino, alkylamino en C₁ à C₄, di-(alkyle en C₁ à C₄)-amino, phénylamino, (alkyle en C₁ à C₄)-carbonylamino, (alkoxy en C₁ à C₄)-carbonylamino, alkylsulfonylamino en C₁ à C₄ ou un groupe phénylsulfonylamino éventuellement substitué par du fluor, du chlore, du brome ou un reste méthyle,
en outre
R³ représente le reste 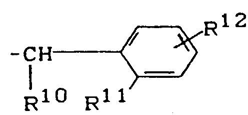 dans lequel
R¹⁰ est de l'hydrogène ou un groupe alkyle en C₁ à C₄,
R¹¹ et R¹² sont identiques ou différents et représentent de l'hydrogène, du fluor, du chlore, du brome, un groupe nitro, cyano, alkyle en C₁ à C₄ (qui est éventuellement substitué par du fluor et/ou du chlore), un groupe alkoxy en C₁ à C₄ (qui est éventuellement substitué par du fluor et/ou du chlore), un groupe carboxy, (alkoxy en C₁ à C₄)-carbonyle, diméthylaminocarbonyle, alkylsulfonyle en C₁ à C₄ ou di-(alkyle en C₁ à C₄)-aminosulfonyle,
en outre
R³ représente le reste 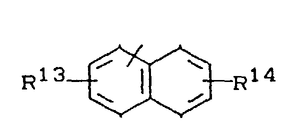 dans lequel
R¹³ et R¹⁴ sont identiques ou différents et représentent de l'hydrogène, du fluor, du chlore, du brome, un groupe nitro, cyano, alkyle en C₁ à C₄ (qui est éventuellement substitué par du fluor et/ou du chlore) ou un groupe alkoxy en C₁ à C₄ (qui est éventuellement substitué par du fluor et/ou du chlore ;
en outre
R³ représente le reste 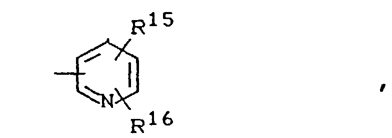 dans lequel
R¹⁵ et R¹⁶ sont identiques ou différents et représentent de l'hydrogène, du fluor, du chlore, du brome, un groupe nitro, cyano, alkyle en C₁ à C₄ (qui est substitué le cas échéant par du fluor et/ou du chlore), un groupe alkoxy en C₁ à C₄ (qui est éventuellement substitué par du fluor et/ou du chlore), un groupe alkylthio en C₁ à C₄, alkylsulfinyle en C₁ à C₄ ou alkylsulfonyle en C₁ à C₄ (qui sont substitués éventuellement par du fluor et/ou du chlore), un groupe aminosulfonyle, mono-(alkyle en C₁ à C₄)-aminosulfonyle, un groupe di-(alkyle en C₁ à C₄)-aminosulfonyle ou (alkoxy en C₁ à C₄)-carbonyle ou diméthylaminocarbonyle ;
ou bien
R³ représente le reste 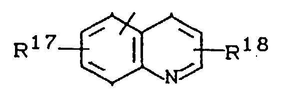 dans lequel
R¹⁷ et R¹⁸ sont identiques ou différents et représentent de l'hydrogène, du fluor, du chlore, du brome, un groupe alkyle en C₁ à C₄ (qui est éventuellement substitué par du fluor et/ou du brome), un groupe alkoxy en C₁ à C₄ (qui est éventuellement substitué par du fluor et/ou du chlore), un groupe alkylthio en C₁ à C₄, alkylsulfinyle en C₁ à C₄ ou alkylsulfonyle en C₁ à C₄ (qui sont substitués le cas échéant par du fluor et/ou du chlore), ou un groupe di-(alkyle en C₁ à C₄)-aminosulfonyle ;
en outre
R³ représente le reste de formule 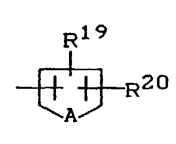 dans laquelle
R¹⁹ et R²⁰ sont identiques ou différents et représentent de l'hydrogène, du fluor, du chlore, du brome, un groupe cyano, nitro, alkyle en C₁ à C₄ (qui est substitué le cas échéant par du fluor et/ou du chlore), un groupe alkoxy en C₁ à C₄ (qui est substitué le cas échéant par du fluor et/ou du chlore), un groupe alkylthio en C₁ à C₄, alkylsulfinyle en C₁ à C₄ ou alkylsulfonyle en C₁ à C₄ (qui est éventuellement substitué par du fluor et/ou du chlore), un groupe di-(alkyle en C₁ à C₄)-aminosulfonyle, (alkoxy en C₁ à C₄)-carbonyle ou diméthylaminocarbonyle, et
A représente de l'oxygène, du soufre ou le groupement N-Z¹, dans lequel
Z¹ représente de l'hydrogène, un groupe alkyle en C₁ à C₄ (qui est éventuellement substitué par du fluor, du chlore, du brome ou un reste cyano), un groupe cycloalkyle en C₃ à C₆, benzyle, phényle, (qui est éventuellement substitué par du fluor, du chlore, du brome ou un reste nitro), un groupe (alkyle en C₁ à C₄)-carbonyle, (alkoxy en C₁ à C₄)-carbonyle ou di-(alkyle en C₁ à C₄)-aminocarbonyle ;
en outre
R³ représente le reste 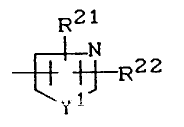 dans lequel
R²¹ et R²² sont identiques ou différents et représentent de l'hydrogène, un groupe alkyle en C₁ à C₄, un halogène, un groupe (alkoxy en C₁ à C₄)-carbonyle, alkoxy en C₁ à C₄ ou halogénalkoxy en C₁ à C₄,
Y¹ représente du soufre ou le groupement N-R²³ dans lequel
R²³ est de l'hydrogène ou un groupe alkyle en C₁ à C₄ ;
en outre
R³ représente le reste 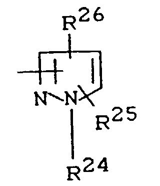 dans lequel
R²⁴ est de l'hydrogène, un groupe alkyle en C₁ à C₄, benzyle, pyridyle, quinolinyle ou phényle,
R²⁵ est de l'hydrogène, un halogène, un groupe cyano, nitro, alkyle en C₁ à C₄ (qui est substitué le cas échéant par du fluor et/ou du chlore), un groupe alkoxy en C₁ à C₄ (qui est substitué le cas échéant par du fluor et/ou du chlore), un groupe dioxolannyle ou (alkoxy en C₁ à C₄)-carbonyle et
R²⁶ représente de l'hydrogène, un halogène ou un groupe alkyle en C₁ à C₄ ;
en outre
R³ représente l'un des groupements indiqués ci-après : 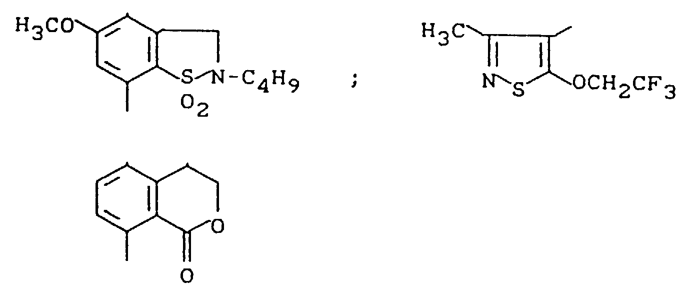
ainsi que des sels de composés de formule (I), à l'exception des composés :
2-(2-méthoxycarbonyl-phénylsulfonylaminocarbonyl)-4-méthyl-5-méthoxy-2,4-dihydro-3H-1,2,4-triazole-3-one,
2-(2-trifluorométhoxy-phénylsulfonylaminocarbonyl)-4-cyclopropyl-5-méthoxy-2,4-dihydro-3H-1,2,4-triazole-3-one et
2-(difluorométhoxy-phénylsulfonylaminocarbonyl)-4-cyclobutyl-5-éthoxy-2,4-dihydro-3H-1,2,4-triazole-3-one.

2. Sulfonylaminocarbonyltriazolinones de formule (I) suivant la revendication 1, caractérisées en ce que dans leur formule :
R¹ représente de l'hydrogène, un groupe amino, un groupe alkyle en C₁ à C₄ éventuellement substitué par du fluor, un reste cyano, méthoxy ou éthoxy, un groupe allyle, un groupe cycloalkyle en C₃ à C₆, un groupe benzyle, un groupe phényle, un groupe alkylamino en C₁ à C₃, cycloalkylamino en C₃ à C₆ ou un groupe di-(alkyle en C₁ à C₃)-amino,
R² est de l'hydrogène, un groupe alkyle en C₁ à C₄ éventuellement substitué par du fluor et/ou du chlore, un reste méthoxy ou éthoxy, un groupe alcényle en C₃ ou C₄ éventuellement substitué par du fluor et/ou du chlore, un groupe cycloalkyle en C₃ à C₆ ou un groupe benzyle éventuellement substitué par du fluor, du chlore et/ou un reste méthyle,
et
R³ représente le groupement 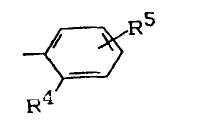 dans lequel
R⁴ est du fluor, du chlore, du brome, un reste méthyle, trifluorométhyle, méthoxy, difluoro-méthoxy, trifluorométhoxy, 2-chloréthoxy, 2-méthoxy-éthoxy, alkylthio en C₁ à C₃, alkylsulfinyle en C₁ à C₃, alkylsulfonyle en C₁ à C₃, diméthylaminosulfonyle, diéthylaminosulfonyle, N-méthoxy-N-méthylaminosulfonyle, méthoxyaminosulfonyle, phényle, phénoxy ou (alkoxy en C₁ à C₃)-carbonyle et
R⁵ représente de l'hydrogène, du fluor, du chlore ou du brome ;
en outre
R³ représente le reste 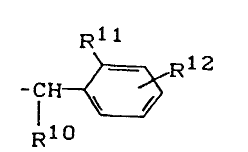 dans lequel
R¹⁰ est de l'hydrogène,
R¹¹ est du fluor, du chlore, du brome, un groupe méthyle, méthoxy, difluorométhoxy, trifluorométhoxy, éthoxy, méthoxycarbonyle, éthoxycarbonyle, méthylsulfonyle ou diméthylaminosulfonyle et
R¹² représente de l'hydrogène ;
en outre
R³ représente le reste 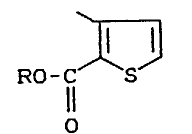 dans lequel R est un groupe alkyle en C₁ à C₄, ou bien
le reste de formule 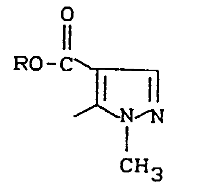
dans laquelle R est un groupe alkyle en C₁ à C₄, ainsi que des sels de composés de formule (I),
avec l'exclusion des trois mêmes composés que dans la revendication 1.

3. Les sels de sodium, de potassium, de magnésium, de calcium, d'ammonium, de (alkyle en C₁ à C₄)-ammonium, di-(alkyle en C₁ à C₄)-ammonium, de tri-(alkyle en C₁ à C₄)-ammonium, de (cycloalkyle en C₅ ou C₆)-ammonium et de di-(alkyle en C₁ ou C₂)-benzylammonium de composés de formule (I) suivant la revendication 1.

4. Procédé de production de sulfonylaminotriazolinones de formule générale (I) 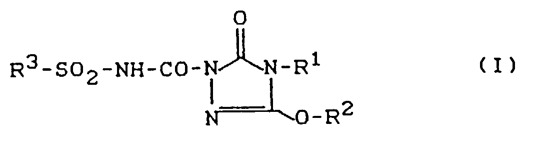 dans laquelle
R¹ représente de l'hydrogène, un groupe amino, un groupe alkyle en C₁ à C₆ éventuellement substitué par du fluor, du chlore, du brome, un groupe cyano, alkoxy en C₁ à C₄, (alkyle en C₁ à C₄)-carbonyle ou (alkoxy en C₁ à C₄)-carbonyle, un groupe alcényle en C₃ à C₆ ou un groupe alcynyle en C₃ à C₆ dont chacun est éventuellement substitué par du fluor, du chlore et/ou du brome, un groupe cycloalkyle en C₃ à C₆ éventuellement substitué par du fluor, du chlore, du brome et/ou un reste alkyle en C₁ à C₄, un groupe phényl-(alkyle en C₁ à C₃) éventuellement substitué par du fluor, du chlore, du brome, un reste cyano, nitro, alkyle en C₁ à C₄, trifluorométhyle, alkoxy en C₁ à C₄ et/ou (alkoxy en C₁ à C₄)-carbonyle, un groupe phényle éventuellement substitué par du fluor, du chlore, du brome, un reste cyano, nitro, alkyle en C₁ à C₄, trifluorométhyle, alkoxy en C₁ à C₄, alkoxy en C₁ à C₃ substitué par du fluor et/ou du chlore, alkylthio en C₁ à C₄, alkylthio en C₁ à C₃ substitué par du fluor et/ou du chlore, alkylsulfinyle en C₁ à C₄, alkylsulfonyle en C₁ à C₄ et/ou (alkoxy en C₁ à C₄)-carbonyle, un groupe alkylamino en C₁ à C₄ éventuellement substitué par du fluor, un reste cyano, alkoxy en C₁ à C₄ ou (alkoxy en C₁ à C₄)-carbonyle, un groupe cycloalkylamino en C₃ à C₆ ou un groupe di(alkyle en C₁ à C₄)-amino,
R² représente un groupe alkyle en C₁ à C₆ éventuellement substitué par du fluor, du chlore, du brome, un reste cyano, cycloalkyle en C₃ à C₆, alkoxy en C₁ à C₄ ou (alkoxy en C₁ à C₄)-carbonyle, un groupe alcényle en C₃ à C₆ ou un groupe alcynyle en C₃ à C₆ dont chacun est éventuellement substitué par du fluor, du chlore et/ou du brome, un groupe cycloalkyle en C₃ à C₆ éventuellement substitué par du fluor, du chlore, du brome et/ou un reste alkyle en C₁ à C₄, un groupe cyclohexényle, un groupe phényl-(alkyle en C₁ à C₃) éventuellement substitué par du fluor, du chlore, du brome, un reste cyano, nitro, alkyle en C₁ à C₄, trifluorométhyle, alkoxy en C₁ à C₄ et/ou (alkoxy en C₁ à C₄)-carbonyle, ou un groupe phényle éventuellement substitué par du fluor, du chlore, du brome, un reste cyano, nitro, alkyle en C₁ à C₄, trifluorométhyle, alkoxy en C₁ à C₄, alkoxy en C₁ à C₃ substitué par du fluor et/ou du chlore, alkylthio en C₁ à C₄, alkylthio en C₁ à C₃ substitué par du fluor et/ou du chlore, alkylsulfinyle en C₁ à C₄, alkylsulfonyle en C₁ à C₄ et/ou (alkoxy en C₁ à C₄)-carbonyle et
R³ représente le groupement 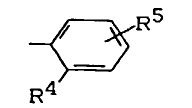 dans lequel
R⁴ et R⁵ sont identiques ou différents et représentent l'hydrogène, du fluor, du chlore, du brome, de l'iode, un groupe nitro, alkyle en C₁ à C₆ (qui est éventuellement substitué par du fluor, du chlore, du brome, un reste cyano, carboxy, (alkoxy en C₁ à C₄)-carbonyle, (alkyle en C₁ à C₄)-aminocarbonyle, di(alkyle en C₁ à C₄)-aminocarbonyle, hydroxy, alkoxy en C₁ à C₄, formyloxy, (alkyle en C₁ à C₄)-carbonyloxy, (alkoxy en C₁ à C₄)-carbonyloxy, (alkyle en C₁ à C₄)-amino-carbonyloxy, alkylthio en C₁ à C₄, alkylsulfinyle en C₁ à C₄, alkylsulfonyle en C₁ à C₄, di(alkyle en C₁ à C₄)-aminosulfonyle, cycloalkyle en C₃ à C₆ ou phényle), un groupe alcényle en C₂ à C₆ (qui est éventuellement substitué par du fluor, du chlore, du brome, un reste cyano, (alkoxy en C₁ à C₄)-carbonyle, carboxy ou phényle), un groupe alcynyle en C₂ à C₆ (qui est éventuellement substitué par du fluor, du chlore, du brome, un reste cyano, (alkoxy en C₁ à C₄)-carbonyle, carboxy ou phényle), un groupe alkoxy en C₁ à C₄ (qui est éventuellement substitué par du fluor, du chlore, du brome, un reste cyano, carboxy, (alkoxy en C₁ à C₄)-carbonyle, alkoxy en C₁ à C₄, alkylthio en C₁ à C₄, alkylsulfinyle en C₁ à C₄ ou alkylsulfonyle en C₁ à C₄), un groupe alkylthio en C₁ à C₄ (qui est éventuellement substitué par du fluor, du chlore, du brome, un reste cyano, carboxy, (alkoxy en C₁ à C₄)-carbonyle, alkylthio en C₁ à C₄, alkylsulfinyle en C₁ à C₄ ou alkylsulfonyle en C₁ à C₄), un groupe alcényloxy en C₃ à C₆ (qui est éventuellement substitué par du fluor, du chlore, du brome, un reste cyano ou (alkoxy en C₁ à C₄)-carbonyle), un groupe alcénylthio en C₂ à C₆ (qui est éventuellement substitué par du fluor, du chlore, du brome, un reste cyano, nitro, alkylthio en C₁ à C₃ ou (alkoxy en C₁ à C₄)-carbonyle), un groupe alcynyloxy en C₃ à C₆, alcynylthio en C₃ à C₆ ou le reste -S(O)ₚ-R⁶ dans lequel
p représente le nombre 1 ou 2 et
R⁶ est un groupe alkyle en C₁ à C₄ (qui est éventuellement substitué par du fluor, du chlore, du brome, un reste cyano ou (alkoxy en C₁ à C₄)-carbonyle), un groupe alcényle en C₃ à C₆, alcynyle en C₃ à C₆, alkoxy en C₁ à C₄, (alkoxy en C₁ à C₄)-(alkylamino en C₁ à C₄), alkylamino en C₁ à C₄, di-(alkyle en C₁ à C₄)-amino, phényle ou le reste -NHOR⁷, dans lequel
R⁷ est un groupe alkyle en C₁ à C₁₂ (qui est éventuellement substitué par du fluor, du chlore, un reste cyano, alkoxy en C₁ à C₄, alkylthio en C₁ à C₄, alkylsulfinyle en C₁ à C₄, alkylsulfonyle en C₁ à C₄, (alkyle en C₁ à C₄)-carbonyle, (alkoxy en C₁ à C₄)-carbonyle, (alkylamino en C₁ à C₄)-carbonyle ou di-(alkyle en C₁ à C₄)-amino-carbonyle), un groupe alcényle en C₃ à C₆ (qui est éventuellement substitué par du fluor, du chlore ou du brome), un groupe alcynyle en C₃ à C₆, cycloalkyle en C₃ à C₆, (cycloalkyle en C₃ à C₆)-(alkyle en C₁ ou C₂), phényl-(alkyle en C₁ ou C₂) (qui est éventuellement substitué par du fluor, du chlore, un reste nitro, cyano, alkyle en C₁ à C₄, alkoxy en C₁ à C₄ ou (alkoxy en C₁ à C₄)-carbonyle), un groupe benzhydryle ou un groupe phényle (qui est éventuellement substitué par du fluor, du chlore, un reste nitro, cyano, alkyle en C₁ à C₄, trifluorométhyle, alkoxy en C₁ à C₄, fluoralkoxy en C₁ ou C₂, alkylthio en C₁ à C₄, trifluorométhylthio ou (alkoxy en C₁ à C₄)-carbonyle),
R⁴ et/ou R⁵ représentent en outre un groupe phényle ou phénoxy, un groupe (alkyle en C₁ à C₄)-carbonylamino, (alkoxy en C₁ à C₄)-carbonylamino, (alkylamino en C₁ à C₄)-carbonyl-amino, di-(alkyle en C₁ à C₄)-amino-carbonylamino ou le reste -CO-R⁸, dans lequel
R⁸ est un groupe alkyle en C₁ à C₆, alkoxy en C₁ à C₆, cycloalkoxy en C₃ à C₆, alcényloxy en C₃ à C₆, alkylthio en C₁ à C₄, alkylamino en C₁ à C₄, alkoxyamino en C₁ à C₄, (alkoxy en C₁ à C₄)-(alkylamino en C₁ à C₄) ou di-(alkyle en C₁ à C₄)-amino (qui sont éventuellement substitués par du fluor et/ou du chlore),
R⁴ et/ou R⁵ représentent en outre un groupe triméthylsilyle, thiazolinyle, alkylsulfonyloxy en C₁ à C₄, di-(alkyle en C₁ à C₄)-aminosulfonylamino ou le reste -CH=N-R⁹, dans lequel
R⁹ est un groupe alkyle en C₁ à C₆ éventuellement substitué par du fluor, du chlore, un reste cyano, carboxy, alkoxy en C₁ à C₄, alkylthio en C₁ à C₄, alkylsulfinyle en C₁ à C₄ ou alkylsulfonyle en C₁ à C₄, un groupe benzyle éventuellement substitué par du fluor ou du chlore, un groupe alcényle en C₃ à C₆ ou un groupe alcynyle en C₃ à C₆ éventuellement substitué par du fluor ou du chlore, un groupe phényle éventuellement substitué par du fluor, du chlore, du brome, un reste alkyle en C₁ à C₄, alkoxy en C₁ à C₄, trifluorométhyle, trifluorométhoxy ou trifluorométhylthio, un groupe alkoxy en C₁ à C₆ éventuellement substitué par du fluor et/ou du chlore, un groupe alcénoxy en C₃ à C₆, alcynoxy en C₃ à C₆ ou benzyloxy, un groupe amino, alkylamino en C₁ à C₄, di-(alkyle en C₁ à C₄)-amino, phénylamino, (alkyle en C₁ à C₄)-carbonylamino, (alkoxy en C₁ à C₄)-carbonylamino, alkylsulfonylamino en C₁ à C₄ ou un groupe phénylsulfonylamino éventuellement substitué par du fluor, du chlore, du brome ou un reste méthyle,
en outre
R³ représente le reste 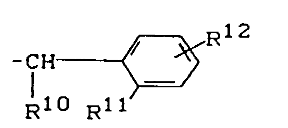 dans lequel
R¹⁰ est de l'hydrogène ou un groupe alkyle en C₁ à C₄,
R¹¹ et R¹² sont identiques ou différents et représentent de l'hydrogène, du fluor, du chlore, du brome, un groupe nitro, cyano, alkyle en C₁ à C₄ (qui est éventuellement substitué par du fluor et/ou du chlore), un groupe alkoxy en C₁ à C₄ (qui est éventuellement substitué par du fluor et/ou du chlore), un groupe carboxy, (alkoxy en C₁ à C₄)-carbonyle, diméthylaminocarbonyle, alkylsulfonyle en C₁ à C₄ ou di-(alkyle en C₁ à C₄)-aminosulfonyle,
en outre
R³ représente le reste 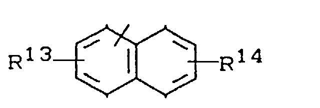 dans lequel
R¹³ et R¹⁴ sont identiques ou différents et représentent de l'hydrogène, du fluor, du chlore, du brome, un groupe nitro, cyano, alkyle en C₁ à C₄ (qui est éventuellement substitué par du fluor et/ou du chlore) ou un groupe alkoxy en C₁ à C₄ (qui est éventuellement substitué par du fluor et/ou du chlore ;
en outre
R³ représente le reste 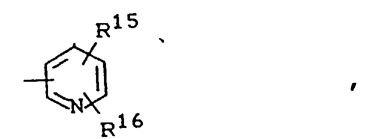 dans lequel
R¹⁵ et R¹⁶ sont identiques ou différents et représentent de l'hydrogène, du fluor, du chlore, du brome, un groupe nitro, cyano, alkyle en C₁ à C₄ (qui est substitué le cas échéant par du fluor et/ou du chlore), un groupe alkoxy en C₁ à C₄ (qui est éventuellement substitué par du fluor et/ou du chlore), un groupe alkylthio en C₁ à C₄, alkylsulfinyle en C₁ à C₄ ou alkylsulfonyle en C₁ à C₄ (qui sont substitués éventuellement par du fluor et/ou du chlore), un groupe aminosulfonyle, mono-(alkyle en C₁ à C₄)-aminosulfonyle, un groupe di-(alkyle en C₁ à C₄)-aminosulfonyle ou (alkoxy en C₁ à C₄)-carbonyle ou diméthylaminocarbonyle ;
ou bien
R³ représente le reste 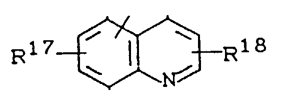 dans lequel
R¹⁷ et R¹⁸ sont identiques ou différents et représentent de l'hydrogène, du fluor, du chlore, du brome, un groupe alkyle en C₁ à C₄ (qui est éventuellement substitué par du fluor et/ou du brome), un groupe alkoxy en C₁ à C₄ (qui est éventuellement substitué par du fluor et/ou du chlore), un groupe alkylthio en C₁ à C₄, alkylsulfinyle en C₁ à C₄ ou alkylsulfonyle en C₁ à C₄ (qui sont substitués le cas échéant par du fluor et/ou du chlore), ou un groupe di-(alkyle en C₁ à C₄)-aminosulfonyle ;
en outre
R³ représente le reste de formule 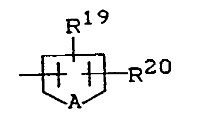 dans laquelle
R¹⁹ et R²⁰ sont identiques ou différents et représentent de l'hydrogène, du fluor, du chlore, du brome, un groupe cyano, nitro, alkyle en C₁ à C₄ (qui est substitué le cas échéant par du fluor et/ou du chlore), un groupe alkoxy en C₁ à C₄ (qui est substitué le cas échéant par du fluor et/ou du chlore), un groupe alkylthio en C₁ à C₄, alkylsulfinyle en C₁ à C₄ ou alkylsulfonyle en C₁ à C₄ (qui est éventuellement substitué par du fluor et/ou du chlore), un groupe di-(alkyle en C₁ à C₄)-aminosulfonyle, (alkoxy en C₁ à C₄)-carbonyle ou diméthylaminocarbonyle, et
A représente de l'oxygène, du soufre ou le groupement N-Z¹, dans lequel
Z¹ représente de l'hydrogène, un groupe alkyle en C₁ à C₄ (qui est éventuellement substitué par du fluor, du chlore, du brome ou un reste cyano), un groupe cycloalkyle en C₃ à C₆, benzyle, phényle, (qui est éventuellement substitué par du fluor, du chlore, du brome ou un reste nitro), un groupe (alkyle en C₁ à C₄)-carbonyle, (alkoxy en C₁ à C₄)-carbonyle ou di-(alkyle en C₁ à C₄)-aminocarbonyle ;
en outre
R³ représente le reste 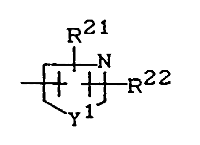 dans lequel
R²¹ et R²² sont identiques ou différents et représentent de l'hydrogène, un groupe alkyle en C₁ à C₄, un halogène, un groupe (alkoxy en C₁ à C₄)-carbonyle, alkoxy en C₁ à C₄ ou halogénalkoxy en C₁ à C₄,
Y¹ représente du soufre ou le groupement N-R²³ dans lequel
R²³ est de l'hydrogène ou un groupe alkyle en C₁ à C₄ ;
en outre
R³ représente le reste 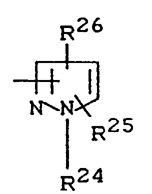 dans lequel
R²⁴ est de l'hydrogène, un groupe alkyle en C₁ à C₄, benzyle, pyridyle, quinolinyle ou phényle,
R²⁵ est de l'hydrogène, un halogène, un groupe cyano, nitro, alkyle en C₁ à C₄ (qui est substitué le cas échéant par du fluor et/ou du chlore), un groupe alkoxy en C₁ à C₄ (qui est substitué le cas échéant par du fluor et/ou du chlore), un groupe dioxolannyle ou (alkoxy en C₁ à C₄)-carbonyle et
R²⁶ représente de l'hydrogène, un halogène ou un groupe alkyle en C₁ à C₄ ;
en outre
R³ représente l'un des groupements indiqués ci-après : 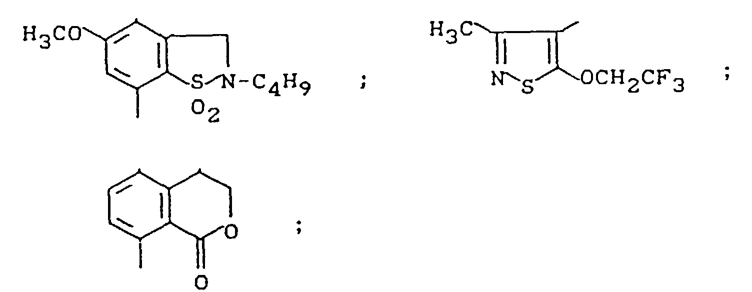
ainsi que des sels de composés de formule (I), à l'exception des composés :
2-(2-méthoxycarbonyl-phénylsulfonylaminocarbonyl)-4-méthyl-5-méthoxy-2,4-dihydro-3H-1,2,4-triazole-3-one,
2-(2 -trifluorométhoxy-phénylsulfonylaminocarbonyl)-4-cyclopropyl-5-méthoxy-2,4-dihydro-3H-1,2,4-triazole-3-one et
2-(difluorométhoxy-phénylsulfonylaminocarbonyl)-4-cyclobutyl-5-éthoxy-2,4-dihydro-3H-1,2,4-triazole-3-one,
caractérisé en ce que
(a) on fait réagir des triazolinones de formule générale (II) 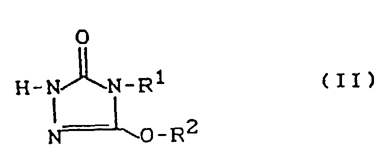 dans laquelle
R¹ et R² ont la définition indiquée pour la formule (I) ci-dessus,
avec des sulfonylisocyanates de formule générale (III)
R³-SO₂-N=C=O (III)
dans laquelle
R³ a la définition indiquée ci-dessus pour la formule (I),
le cas échéant en présence d'un diluant, ou bien
(b) on fait réagir des dérivés de triazolinones de formule générale (IV) 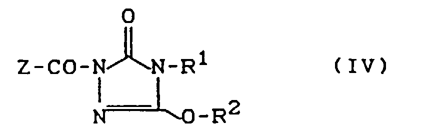 dans laquelle
R¹ et R² ont les définitions indiquées pour la formule (I) et
Z représente du chlore, un groupe alkoxy en C₁ à C₄, benzyloxy ou phénoxy,
avec des amides d'acide sulfonique de formule générale (V)
R³-SO₂-NH₂ (V)
dans laquelle
R³ a la définition indiquée pour la formule (I),
le cas échéant en présence d'un accepteur d'acide et en la présence éventuelle d'un diluant, ou bien
(c) on fait réagir des triazolinones de formule générale (II) 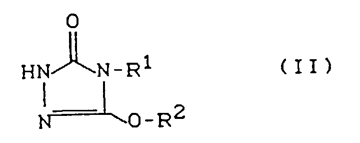 dans laquelle
R¹ et R² ont les définitions indiquées pour la formule (I),
avec des dérivés d'amide d'acide sulfonique de formule générale (VI)
R³-SO₂-NH-CO-Z (VI)
dans laquelle
R³ a la définition indiquée pour la formule (I) et
Z représente du chlore, un groupe alkoxy en C₁ à C₄, benzyloxy ou phénoxy,
le cas échéant en présence d'un accepteur d'acide et en la présence éventuelle d'un diluant, et on produit éventuellement, par des moyens classiques, des sels à partir des composés de formule (I) obtenus d'après les procédés (a), (b) et (c).

5. Compositions herbicides, caractérisées par une teneur en au moins une sulfonylaminocarbonyltriazolinone de formule (I) suivant la revendication 1 ou 4.

6. Utilisation de sulfonylaminocarbonyltriazolinones de formule générale (I) suivant la revendication 1 ou 4 pour combattre une croissance végétale indésirable.

7. Procédé pour combattre des mauvaises herbes, caractérisé en ce qu'on fait agir sur les mauvaises herbes ou sur leur milieu des sulfonylaminocarbonyltriazolinones de formule générale (I) suivant la revendication 1 ou 4.

8. Procédé de préparation de compositions herbicides, caractérisé en ce qu'on mélange des sulfonylaminocarbonyltriazolinones de formule générale (I) suivant la revendication 1 ou 4 avec des diluants et/ou des agents tensio-actifs.

9. Triazolinones de formule générale (II) 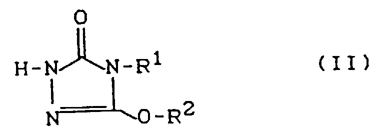 dans laquelle
R¹ représente un groupe amino, un groupe alkyle en C₁ à C₆ éventuellement substitué par du fluor, du chlore, du brome, un reste cyano, alkoxy en C₁ à C₄, (alkyle en C₁ à C₄)-carbonyle ou (alkoxy en C₁ à C₄)-carbonyle, un groupe alcényle en C₃ à C₆ ou un groupe alcynyle en C₃ à C₆ dont chacun est éventuellement substitué par du fluor, du chlore et/ou du brome, un groupe cycloalkyle en C₃ à C₆ éventuellement substitué par du fluor, du chlore, du brome et/ou un reste alkyle en C₁ à C₄, un groupe phényl-(alkyle en C₁ à C₃) éventuellement substitué par du fluor, du chlore, du brome, un reste cyano, nitro, alkyle en C₁ à C₄, trifluorométhyle, alkoxy en C₁ à C₄ et/ou (alkoxy en C₁ à C₄)-carbonyle, un groupe phényle éventuellement substitué par du fluor, du chlore, du brome, un reste cyano, nitro, alkyle en C₁ à C₄, trifluorométhyle, alkoxy en C₁ à C₄, alkoxy en C₁ à C₃ substitué par du fluor et/ou du chlore, alkylthio en C₁ à C₄, alkylthio en C₁ à C₃ éventuellement substitué par du fluor et/ou du chlore, alkylsulfinyle en C₁ à C₄, alkylsulfonyle en C₁ à C₄ et/ou (alkoxy en C₁ à C₄)-carbonyle, un groupe alkylamino en C₁ à C₄ éventuellement substitué par du fluor, un reste cyano, alkoxy en C₁ à C₄ ou (alkoxy en C₁ à C₄)-carbonyle, un groupe cycloalkylamino en C₃ à C₆ ou un groupe di-(alkyle en C₁ à C₄)-amino et
R² représente un groupe cycloalkyle en C₃ à C₆ éventuellement substitué par du fluor, du chlore, du brome et/ou un reste alkyle en C₁ à C₄, un groupe cyclohexényle, un groupe phényl-(alkyle en C₁ à C₃) éventuellement substitué par du fluor, du chlore, du brome, un reste cyano, nitro, alkyle en C₁ à C₄, trifluorométhyle, alkoxy en C₁ à C₄ et/ou (alkoxy en C₁ à C₄)-carbonyle, ou un groupe phényle éventuellement substitué par du fluor, du chlore, du brome, un reste cyano, nitro, alkyle en C₁ à C₄, trifluorométhyle, alkoxy en C₁ à C₄, alkoxy en C₁ à C₃ substitué par du fluor et/ou du chlore, alkylthio en C₁ à C₄, alkylthio en C₁ à C₃ éventuellement substitué par du fluor et/ou du chlore, alkylsulfinyle en C₁ à C₄, alkylsulfonyle en C₁ à C₄ et/ou (alkoxy en C₁ à C₄)-carbonyle.

10. Procédé de production de triazolinones de formule générale (II) 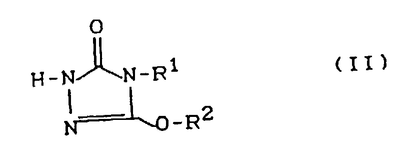 dans laquelle
R¹ représente un groupe amino, un groupe alkyle en C₁ à C₆ éventuellement substitué par du fluor, du chlore, du brome, un reste cyano, alkoxy en C₁ à C₄, (alkyle en C₁ à C₄)-carbonyle ou (alkoxy en C₁ à C₄)-carbonyle, un groupe alcényle en C₃ à C₆ ou un groupe alcynyle en C₃ à C₆ dont chacun est éventuellement substitué par du fluor, du chlore et/ou du brome, un groupe cycloalkyle en C₃ à C₆ éventuellement substitué par du fluor, du chlore, du brome et/ou un reste alkyle en C₁ à C₄, un groupe phényl-(alkyle en C₁ à C₃) éventuellement substitué par du fluor, du chlore, du brome, un reste cyano, nitro, alkyle en C₁ à C₄, trifluorométhyle, alkoxy en C₁ à C₄ et/ou (alkoxy en C₁ à C₄)-carbonyle, un groupe phényle éventuellement substitué par du fluor, du chlore, du brome, un reste cyano, nitro, alkyle en C₁ à C₄, trifluorométhyle, alkoxy en C₁ à C₄, alkoxy en C₁ à C₃ substitué par du fluor et/ou du chlore, alkylthio en C₁ à C₄, alkylthio en C₁ à C₃ éventuellement substitué par du fluor et/ou du chlore, alkylsulfinyle en C₁ à C₄, alkylsulfonyle en C₁ à C₄ et/ou (alkoxy en C₁ à C₄)-carbonyle, un groupe alkylamino en C₁ à C₄ éventuellement substitué par du fluor, un reste cyano, alkoxy en C₁ à C₄ ou (alkoxy en C₁ à C₄)-carbonyle, un groupe cycloalkylamino en C₃ à C₆ ou un groupe di-(alkyle en C₁ à C₄)-amino et
R² représente un groupe cycloalkyle en C₃ à C₆ éventuellement substitué par du fluor, du chlore, du brome et/ou un reste alkyle en C₁ à C₄, un groupe cyclohexényle, un groupe phényl-(alkyle en C₁ à C₃) éventuellement substitué par du fluor, du chlore, du brome, un reste cyano, nitro, alkyle en C₁ à C₄, trifluorométhyle, alkoxy en C₁ à C₄ et/ou (alkoxy en C₁ à C₄)-carbonyle, ou un groupe phényle éventuellement substitué par du fluor, du chlore, du brome, un reste cyano, nitro, alkyle en C₁ à C₄, trifluorométhyle, alkoxy en C₁ à C₄, alkoxy en C₁ à C₃ substitué par du fluor et/ou du chlore, alkylthio en C₁ à C₄, alkylthio en C₁ à C₃ éventuellement substitué par du fluor et/ou du chlore, alkylsulfinyle en C₁ à C₄, alkylsulfonyle en C₁ à C₄ et/ou (alkoxy en C₁ à C₄)-carbonyle,
caractérisé en ce qu'on fait réagir des esters d'acide hydrazinoformiques de formule générale (VII)
H₂N-NH-CO-O-R²⁷ (VII)
dans laquelle
R²⁷ est un groupe méthyle, éthyle ou phényle,
avec des diesters d'acides alkyliminocarboniques de formule générale (VIII) 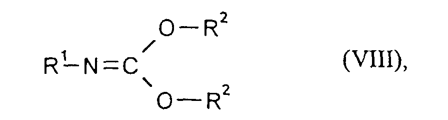 dans laquelle
R¹ et R² ont la définition indiquée ci-dessus,
le cas échéant en présence d'un diluant, à des températures comprises entre 0°C et 50°C, on isole éventuellement par des moyens classiques les composés ainsi formés, de formule générale (IX) 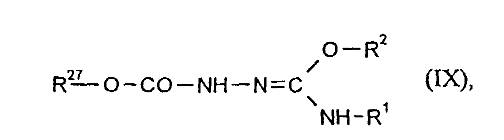 dans laquelle
R¹, R² et R²⁷ ont la définition indiquée ci-dessus,
et on les chauffe à des températures comprises entre 50°C et 150°C, le cas échéant en présence d'un diluant.

11. Composés de formule générale (IX) 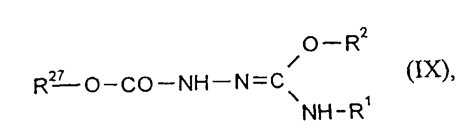 dans laquelle
R¹ et R² ont les définitions indiquées dans la revendication 9 et
R²⁷ est un groupe méthyle, éthyle ou phényle.

12. Procédé suivant la revendicatioin 10, caractérisé en ce qu'on utilise comme esters d'acides hydrazinoformiques de formule (VII), l'ester de phényle de l'acide hydrazinoformique et comme diester d'acide alkyliminocarbonique de formule (VIII), l'iminocarbonate de triméthyle, et on obtient ainsi la 4-méthyl-5-méthoxy-2,4-dihydro-3H-1,2,4-triazole-3-one (II-2).
